# EUROPÄISCHE PATENTANMELDUNG

(11) **EP 2 264 153 A2**
(43) Veröffentlichungstag der Anmeldung: **22.12.2010**
(21) Anmeldenummer: 10174635.2
(22) Anmeldetag: 17.06.2005
(51) Int. Cl.: C12N 9/10, C12N 9/00, C12P 7/16, C12P 7/52

(54) **Neue, Geruchsstoffe bildende Genprodukte von Bacillus licheniformis und darauf aufbauende verbesserte biotechnologische Produktionsverfahren**

(30) Priorität: 29.06.2004 DE 102004031177
(62) Teilanmeldung aus: 05752264.1
(71) Anmelder: Henkel AG & Co. KGaA, 40589 Düsseldorf (DE)
(72) Erfinder: Bessler, Cornelius, 40231 Düsseldorf (DE); Feesche, Jörg, 40699 Erkrath (DE); Evers, Stefan, 40822 Mettmann (DE); Maurer, Karl-Heinz, 40699 Erkrath (DE); Ehrenreich, Armin, 37077 Göttingen (AT); Veith, Birgit, 40211 Düsseldorf (DE); Liesegang, Heiko, 37120 Bovenden (DE); Henne, Anke, 42655 Solingen (DE); Herzberg, Christina, 37434 Bilshausen (DE); Gottschalk, Gerhard, 37176 Nörten-Hardenberg (DE)
(74) Vertreter: Knödel, Matthias

(57) **Zusammenfassung**

Die Bildung unangenehmer Gerüche bei der Fermentation von Mikroorganismen soll verringert werden und es sollen Gene identifiziert werden, die für Proteine codieren, die an der Bildung unangenehm riechender Verbindungen beteiligt sind. Dies gelingt durch eine Nukleinsäure gemäß SEQ ID NO. 31, die für ein an der Synthese von Butanol und/oder Buttersäure beteiligtes Genprodukt (wahrscheinliche Phosphat-Butyryl-Transferase; E.C. 2.3.1.19) codiert sowie alle hinreichend homologen Nukleinsäuren hierzu. Die Identifizierung dieses Gens ermöglicht biotechnologische Produktionsverfahren, die insofern verbessert sind, als mithilfe derartiger Nukleinsäuren die Bildung der über diese Stoffwechselwege synthetisierten Geruchsstoffe durch Inaktivierung des zugehörigen Gens in dem für die biotechnologische Produktion verwendeten Mikroorganismus verringert werden kann.

SEQUENCE LISTING

<110>  Henkel Kommanditgesellschaft auf Aktien

<120>  Neue, Geruchsstoffe bildende Genprodukte von Bacillus
licheniformis und darauf aufbauende verbesserte biotechnologische
Produktionsverfahren

<130>  H 06383 PCT

<150>  DE102004031177.3
<151>  2004-06-29

<160>  50

<170>  PatentIn version 3.1

<210>  1
<211>  918
<212>  DNA
<213>  Bacillus licheniformis DSM 13

<220>
<221>  gene
<222>  (1)..(918)
<223>  Putative branched-chain amino acid aminotransferase (E.C. 2.6.1.4
       2)


<220>
<221>  CDS
<222>  (1)..(915)
<223>


<400>  1
atg ggg gac cag aaa gac cag tgg atc ttc cta aac gac aaa ctc gtt       48
Met Gly Asp Gln Lys Asp Gln Trp Ile Phe Leu Asn Asp Lys Leu Val
1               5                   10                  15

aaa aaa gaa gac gct aaa ata tca gtc tat gat cac gga ttt tta tac       96
Lys Lys Glu Asp Ala Lys Ile Ser Val Tyr Asp His Gly Phe Leu Tyr
            20                  25                  30

ggg gac ggc gtg ttt gaa ggg atc agg gta tac gac ggc aac atc ttc      144
Gly Asp Gly Val Phe Glu Gly Ile Arg Val Tyr Asp Gly Asn Ile Phe
        35                  40                  45

aga atg caa gag cac atg gac cgc ctc tac gat tct gcg aga tcg atc      192
Arg Met Gln Glu His Met Asp Arg Leu Tyr Asp Ser Ala Arg Ser Ile
    50                  55                  60

atg ctg gag att cca tat cca cag gaa gaa ctg aca cag cac gta ctc      240
Met Leu Glu Ile Pro Tyr Pro Gln Glu Glu Leu Thr Gln His Val Leu
65                  70                  75                  80

aaa aca gtc gaa aaa aac ggg ctg aaa gac gct tac atc cgc ttg gtc      288
Lys Thr Val Glu Lys Asn Gly Leu Lys Asp Ala Tyr Ile Arg Leu Val
                85                  90                  95

gtt tca aga gga gca ggt gac ctc gga ctc gat cca aac aat tgt tca      336
Val Ser Arg Gly Ala Gly Asp Leu Gly Leu Asp Pro Asn Asn Cys Ser
            100                 105                 110

aac ccg agt gtc atc ata att gtc gaa cca ttg gca ata ttc ccg aaa      384
Asn Pro Ser Val Ile Ile Ile Val Glu Pro Leu Ala Ile Phe Pro Lys
        115                 120                 125

cat tta tat gaa acg ggg att gac att gtt acg gtt ccg aca aga agg      432
His Leu Tyr Glu Thr Gly Ile Asp Ile Val Thr Val Pro Thr Arg Arg
    130                 135                 140

aac aga ccc gat gtg ctg agc cct aaa gta aaa tcg ctg aac tac tta      480
Asn Arg Pro Asp Val Leu Ser Pro Lys Val Lys Ser Leu Asn Tyr Leu
145                 150                 155                 160

aac aat att ctt gtc cgg atc gag gcg cat atg gcg ggt gtg acg gaa      528
Asn Asn Ile Leu Val Arg Ile Glu Ala His Met Ala Gly Val Thr Glu
                165                 170                 175

gcg ctc atg ctc aat gat caa ggc tat gtc gcc gaa ggg tct gcg gat      576
Ala Leu Met Leu Asn Asp Gln Gly Tyr Val Ala Glu Gly Ser Ala Asp
            180                 185                 190

aac gta ttt att tat aaa aac gga aag ctc ttg acg cct ccg ggc tat      624
Asn Val Phe Ile Tyr Lys Asn Gly Lys Leu Leu Thr Pro Pro Gly Tyr
        195                 200                 205

atc gga gcg ctt gaa gga atc acc cgg aat gcc atc atc gaa ata gcg      672
Ile Gly Ala Leu Glu Gly Ile Thr Arg Asn Ala Ile Ile Glu Ile Ala
    210                 215                 220

cga gag ctc ggc tat gaa gtg aaa gaa gag ccg ttt acc cgc cat gac      720
Arg Glu Leu Gly Tyr Glu Val Lys Glu Glu Pro Phe Thr Arg His Asp
225                 230                 235                 240

gta tac aca gcc gag gaa gtg ttt tta acc gga acg gct gca gaa gtc      768
Val Tyr Thr Ala Glu Glu Val Phe Leu Thr Gly Thr Ala Ala Glu Val
                245                 250                 255

atc gcg gtc gta aaa gtt gac ggc cgc aag atc ggg gac ggc aaa ccg      816
Ile Ala Val Val Lys Val Asp Gly Arg Lys Ile Gly Asp Gly Lys Pro
            260                 265                 270

gga gtc cac aca aac cgg atg ctt gaa aag ttc cgc gag cgc gtc gtc      864
Gly Val His Thr Asn Arg Met Leu Glu Lys Phe Arg Glu Arg Val Val
        275                 280                 285

cgt gaa ggg tta aaa gtc agc ctc aaa gat caa agc tta agt gtc agc      912
Arg Glu Gly Leu Lys Val Ser Leu Lys Asp Gln Ser Leu Ser Val Ser
    290                 295                 300

tga ata                                                              918

<210>  2
<211>  304
<212>  PRT

<213>  Bacillus licheniformis DSM 13

<400>  2

Met Gly Asp Gln Lys Asp Gln Trp Ile Phe Leu Asn Asp Lys Leu Val
1               5                   10                  15

Lys Lys Glu Asp Ala Lys Ile Ser Val Tyr Asp His Gly Phe Leu Tyr
            20                  25                  30

Gly Asp Gly Val Phe Glu Gly Ile Arg Val Tyr Asp Gly Asn Ile Phe
        35                  40                  45

Arg Met Gln Glu His Met Asp Arg Leu Tyr Asp Ser Ala Arg Ser Ile
    50                  55                  60

Met Leu Glu Ile Pro Tyr Pro Gln Glu Glu Leu Thr Gln His Val Leu
65                  70                  75                  80

Lys Thr Val Glu Lys Asn Gly Leu Lys Asp Ala Tyr Ile Arg Leu Val
                85                  90                  95

Val Ser Arg Gly Ala Gly Asp Leu Gly Leu Asp Pro Asn Asn Cys Ser
            100                 105                 110

Asn Pro Ser Val Ile Ile Ile Val Glu Pro Leu Ala Ile Phe Pro Lys
        115                 120                 125

His Leu Tyr Glu Thr Gly Ile Asp Ile Val Thr Val Pro Thr Arg Arg
    130                 135                 140

Asn Arg Pro Asp Val Leu Ser Pro Lys Val Lys Ser Leu Asn Tyr Leu
145                 150                 155                 160

Asn Asn Ile Leu Val Arg Ile Glu Ala His Met Ala Gly Val Thr Glu
                165                 170                 175

Ala Leu Met Leu Asn Asp Gln Gly Tyr Val Ala Glu Gly Ser Ala Asp
            180                 185                 190

Asn Val Phe Ile Tyr Lys Asn Gly Lys Leu Leu Thr Pro Pro Gly Tyr
        195                 200                 205

Ile Gly Ala Leu Glu Gly Ile Thr Arg Asn Ala Ile Ile Glu Ile Ala
    210                 215                 220

Arg Glu Leu Gly Tyr Glu Val Lys Glu Glu Pro Phe Thr Arg His Asp
225                 230                 235                 240

Val Tyr Thr Ala Glu Glu Val Phe Leu Thr Gly Thr Ala Ala Glu Val
                245                 250                 255

Ile Ala Val Val Lys Val Asp Gly Arg Lys Ile Gly Asp Gly Lys Pro
            260                 265                 270

Gly Val His Thr Asn Arg Met Leu Glu Lys Phe Arg Glu Arg Val Val
        275                 280                 285

Arg Glu Gly Leu Lys Val Ser Leu Lys Asp Gln Ser Leu Ser Val Ser
    290                 295                 300

<210>  3
<211>  1095
<212>  DNA
<213>  Bacillus licheniformis DSM 13

<220>
<221>  gene
<222>  (1)..(1095)
<223>  Putative branched-chain amino acid aminotransferase (E.C. 2.6.1.4
       2)

<220>
<221>  CDS
<222>  (1)..(1092)
<223>

<400>  3
atg aca aaa caa acg atc agc gta cag ctc agt aca gca aaa aag caa       48
Met Thr Lys Gln Thr Ile Ser Val Gln Leu Ser Thr Ala Lys Lys Gln
1               5                   10                  15

aag cca gag gct gac aag ctc gaa ttc ggc cgg acc ttt acc gac cac       96
Lys Pro Glu Ala Asp Lys Leu Glu Phe Gly Arg Thr Phe Thr Asp His
            20                  25                  30

atg ttt atc atg gac tat acg gct gaa aac ggc tgg cac gat ccg aga      144
Met Phe Ile Met Asp Tyr Thr Ala Glu Asn Gly Trp His Asp Pro Arg
        35                  40                  45

atc gtt cct tac cag ccg att gaa atg gac ccg gct gca atg gtt tac      192
Ile Val Pro Tyr Gln Pro Ile Glu Met Asp Pro Ala Ala Met Val Tyr
    50                  55                  60

cac tac gga caa tcc gtt ttt gaa gga tta aaa gct tat tta tca agc      240
His Tyr Gly Gln Ser Val Phe Glu Gly Leu Lys Ala Tyr Leu Ser Ser
65                  70                  75                  80

gaa ggc aga gtt ctt ctg ttc aga cct gaa aaa aac ttc gag aga ctc 288
Glu Gly Arg Val Leu Leu Phe Arg Pro Glu Lys Asn Phe Glu Arg Leu
                85                  90                  95

aat aaa tcc aac gac cgc ctc tgc att ccc cgg gtt gat cct gaa atc 336
Asn Lys Ser Asn Asp Arg Leu Cys Ile Pro Arg Val Asp Pro Glu Ile
            100                 105                 110

gtt ctg gaa ggg ctg aag cag ctg gtt cag atc gac aag gaa tgg att 384
Val Leu Glu Gly Leu Lys Gln Leu Val Gln Ile Asp Lys Glu Trp Ile
        115                 120                 125

cct caa gct gag ggg aca tcc ctt tat atc cgt ccg ttc att att tca 432
Pro Gln Ala Glu Gly Thr Ser Leu Tyr Ile Arg Pro Phe Ile Ile Ser
    130                 135                 140

aca gaa ccg tac ctt ggc gtc gcc cca tcc aat atg tat aaa atg ctg 480
Thr Glu Pro Tyr Leu Gly Val Ala Pro Ser Asn Met Tyr Lys Met Leu
145                 150                 155                 160

atc att tta tcg ccg gtc gga tct tat tac aaa gaa ggc atc cgc cct 528
Ile Ile Leu Ser Pro Val Gly Ser Tyr Tyr Lys Glu Gly Ile Arg Pro
                165                 170                 175

gtg aaa att gct gtt gaa agc gaa ttt gtc cgt gct gtg gca ggc ggt 576
Val Lys Ile Ala Val Glu Ser Glu Phe Val Arg Ala Val Ala Gly Gly
            180                 185                 190

aca ggc aat gca aaa acg gcc gga aac tac gct gcg agc ctg aag gct 624
Thr Gly Asn Ala Lys Thr Ala Gly Asn Tyr Ala Ala Ser Leu Lys Ala
        195                 200                 205

cag gaa gtt gcg gaa agc aaa ggc ttc tca caa gtg ctg tgg ctt gac 672
Gln Glu Val Ala Glu Ser Lys Gly Phe Ser Gln Val Leu Trp Leu Asp
    210                 215                 220

gga gtt gaa aag aaa tac att gaa gaa gta ggc agc atg aac atc ttc 720
Gly Val Glu Lys Lys Tyr Ile Glu Glu Val Gly Ser Met Asn Ile Phe
225                 230                 235                 240

ttc aaa atc agc ggt gaa att gtc act ccg gct cta aac gga agc att 768
Phe Lys Ile Ser Gly Glu Ile Val Thr Pro Ala Leu Asn Gly Ser Ile
                245                 250                 255

ttg gaa ggc att acg aga aac tcc gtc atc cat ctc tta aaa caa tgg 816
Leu Glu Gly Ile Thr Arg Asn Ser Val Ile His Leu Leu Lys Gln Trp
            260                 265                 270

gga ctc tcc gta acc gaa aga aaa att tca gtt gat gaa ctg gtt cag 864
Gly Leu Ser Val Thr Glu Arg Lys Ile Ser Val Asp Glu Leu Val Gln
        275                 280                 285

gct cac aaa gac ggc ctg ctt gag gaa gca ttc ggc acc gga acc gcc 912
Ala His Lys Asp Gly Leu Leu Glu Glu Ala Phe Gly Thr Gly Thr Ala
    290                 295                 300

gct gtc att tcc ccc gtc ggc gag ctg atc tgg aaa gac gaa agc ctt 960
Ala Val Ile Ser Pro Val Gly Glu Leu Ile Trp Lys Asp Glu Ser Leu

305                 310                 315                 320

gtg atc aac aac ggt caa act gga gaa atc gcc aaa agg ctc tac caa      1008
Val Ile Asn Asn Gly Gln Thr Gly Glu Ile Ala Lys Arg Leu Tyr Gln
                325                 330                 335

acg atc acc ggt att caa aaa ggc gct ttg cct gac aca ttc ggc tgg      1056
Thr Ile Thr Gly Ile Gln Lys Gly Ala Leu Pro Asp Thr Phe Gly Trp
            340                 345                 350

aca gtt gaa gtt gat aaa gta agc cag tcc tgc taa gcg                  1095
Thr Val Glu Val Asp Lys Val Ser Gln Ser Cys
        355                 360

<210>  4
<211>  363
<212>  PRT
<213>  Bacillus licheniformis DSM 13

<400>  4

Met Thr Lys Gln Thr Ile Ser Val Gln Leu Ser Thr Ala Lys Lys Gln
1               5                   10                  15

Lys Pro Glu Ala Asp Lys Leu Glu Phe Gly Arg Thr Phe Thr Asp His
            20                  25                  30

Met Phe Ile Met Asp Tyr Thr Ala Glu Asn Gly Trp His Asp Pro Arg
        35                  40                  45

Ile Val Pro Tyr Gln Pro Ile Glu Met Asp Pro Ala Ala Met Val Tyr
    50                  55                  60

His Tyr Gly Gln Ser Val Phe Glu Gly Leu Lys Ala Tyr Leu Ser Ser
65                  70                  75                  80

Glu Gly Arg Val Leu Leu Phe Arg Pro Glu Lys Asn Phe Glu Arg Leu
                85                  90                  95

Asn Lys Ser Asn Asp Arg Leu Cys Ile Pro Arg Val Asp Pro Glu Ile
            100                 105                 110

Val Leu Glu Gly Leu Lys Gln Leu Val Gln Ile Asp Lys Glu Trp Ile
        115                 120                 125

Pro Gln Ala Glu Gly Thr Ser Leu Tyr Ile Arg Pro Phe Ile Ile Ser
    130                 135                 140

Thr Glu Pro Tyr Leu Gly Val Ala Pro Ser Asn Met Tyr Lys Met Leu

145                 150                 155                 160

Ile Ile Leu Ser Pro Val Gly Ser Tyr Tyr Lys Glu Gly Ile Arg Pro
                165                 170                 175

Val Lys Ile Ala Val Glu Ser Glu Phe Val Arg Ala Val Ala Gly Gly
            180                 185                 190

Thr Gly Asn Ala Lys Thr Ala Gly Asn Tyr Ala Ala Ser Leu Lys Ala
        195                 200                 205

Gln Glu Val Ala Glu Ser Lys Gly Phe Ser Gln Val Leu Trp Leu Asp
    210                 215                 220

Gly Val Glu Lys Lys Tyr Ile Glu Glu Val Gly Ser Met Asn Ile Phe
225                 230                 235                 240

Phe Lys Ile Ser Gly Glu Ile Val Thr Pro Ala Leu Asn Gly Ser Ile
                245                 250                 255

Leu Glu Gly Ile Thr Arg Asn Ser Val Ile His Leu Leu Lys Gln Trp
            260                 265                 270

Gly Leu Ser Val Thr Glu Arg Lys Ile Ser Val Asp Glu Leu Val Gln
        275                 280                 285

Ala His Lys Asp Gly Leu Leu Glu Glu Ala Phe Gly Thr Gly Thr Ala
    290                 295                 300

Ala Val Ile Ser Pro Val Gly Glu Leu Ile Trp Lys Asp Glu Ser Leu
305                 310                 315                 320

Val Ile Asn Asn Gly Gln Thr Gly Glu Ile Ala Lys Arg Leu Tyr Gln
                325                 330                 335

Thr Ile Thr Gly Ile Gln Lys Gly Ala Leu Pro Asp Thr Phe Gly Trp
            340                 345                 350

Thr Val Glu Val Asp Lys Val Ser Gln Ser Cys
        355                 360

<210>  5
<211>  1494
<212>  DNA
<213>  Bacillus licheniformis DSM 13

<220>
<221>  gene
<222>  (1)..(1494)
<223>  Lysine and/or Arginine decarboxylase (E.C. 4.1.1.18 or E.C. 4.1.1
       .19, respectively), speA


<220>
<221>  CDS
<222>  (1)..(1491)
<223>


<400>  5
atg aag gtg gga aaa caa ttg ttg cag cac gaa aca ccc cta tat acc       48
Met Lys Val Gly Lys Gln Leu Leu Gln His Glu Thr Pro Leu Tyr Thr
1               5                   10                  15

gga tta aaa aaa cac gcc ggc aaa aac ccg atc cag ttc cat ata ccg       96
Gly Leu Lys Lys His Ala Gly Lys Asn Pro Ile Gln Phe His Ile Pro
            20                  25                  30

ggg cac aaa aaa ggc tct ggg atg gac cct gaa ttc agg gag ttt atc      144
Gly His Lys Lys Gly Ser Gly Met Asp Pro Glu Phe Arg Glu Phe Ile
        35                  40                  45

gga gaa aat gca tta agc ata gat tta atc aac atc gag cct ctc gac      192
Gly Glu Asn Ala Leu Ser Ile Asp Leu Ile Asn Ile Glu Pro Leu Asp
    50                  55                  60

gat ctg cac gcg ccg aaa gga ata atc aaa cag gcg cag gat ctg gcg      240
Asp Leu His Ala Pro Lys Gly Ile Ile Lys Gln Ala Gln Asp Leu Ala
65                  70                  75                  80

gct gaa gca ttc gga gcc gac tac acg ttc ttt tcc gtc cag gga acg      288
Ala Glu Ala Phe Gly Ala Asp Tyr Thr Phe Phe Ser Val Gln Gly Thr
                85                  90                  95

agc ggc gcc atc atg acg atg gtc atg gcc gta tgc gga ccc gga gac      336
Ser Gly Ala Ile Met Thr Met Val Met Ala Val Cys Gly Pro Gly Asp
            100                 105                 110

aaa atc atc gtc ccg aga aat gtt cat aaa tcg gtg atg tca gcg atc      384
Lys Ile Ile Val Pro Arg Asn Val His Lys Ser Val Met Ser Ala Ile
        115                 120                 125

gtc ttc tca ggc gcc gta ccg atc ttc atc cat ccg gaa atc gac gat      432
Val Phe Ser Gly Ala Val Pro Ile Phe Ile His Pro Glu Ile Asp Asp
    130                 135                 140

gag ctg ggg att tca cac ggg atc acc cct gaa tca gcg aaa aaa gcg      480
Glu Leu Gly Ile Ser His Gly Ile Thr Pro Glu Ser Ala Lys Lys Ala
145                 150                 155                 160

ctg ctt gag cat cct gac gca aaa gga ctg ctc gtc atc aac ccg act      528
Leu Leu Glu His Pro Asp Ala Lys Gly Leu Leu Val Ile Asn Pro Thr
                165                 170                 175

tat ttc ggc ata gct gca gac tta aaa agc atc gtc gag ctg gct cat      576
Tyr Phe Gly Ile Ala Ala Asp Leu Lys Ser Ile Val Glu Leu Ala His
            180                 185                 190

tct ttt cat gtc ccg gtg cta gtt gac gag gcg cac ggc gtt cat atc      624
Ser Phe His Val Pro Val Leu Val Asp Glu Ala His Gly Val His Ile
        195                 200                 205

cac ttc cat gaa gat ctg cct ctt tcg gca atg cag gcc gga gcg gat      672
His Phe His Glu Asp Leu Pro Leu Ser Ala Met Gln Ala Gly Ala Asp
    210                 215                 220

atg gcg gcg acg agc gtt cat aag ctc gga gga tcc ctt aca caa agt      720
Met Ala Ala Thr Ser Val His Lys Leu Gly Gly Ser Leu Thr Gln Ser
225                 230                 235                 240

tcg att ctc aat atg aaa gaa ggt ctg gtt tca aag gaa agg gtg caa      768
Ser Ile Leu Asn Met Lys Glu Gly Leu Val Ser Lys Glu Arg Val Gln
                245                 250                 255

tcg att tta agc atg ctg acg acg aca tcg acc tcc tat cta ttg ctc      816
Ser Ile Leu Ser Met Leu Thr Thr Thr Ser Thr Ser Tyr Leu Leu Leu
            260                 265                 270

gct tcg ctt gat gtc gcc aga aaa cgc tta gcc aca gag gga cac gaa      864
Ala Ser Leu Asp Val Ala Arg Lys Arg Leu Ala Thr Glu Gly His Glu
        275                 280                 285

ctg atc gaa caa acg att aag ctc gcc aac gaa aca agg gaa cgc atc      912
Leu Ile Glu Gln Thr Ile Lys Leu Ala Asn Glu Thr Arg Glu Arg Ile
    290                 295                 300

aat aat atc aac ggg att tca tgc gtc ggg agg gaa atc ctc ggc tcc      960
Asn Asn Ile Asn Gly Ile Ser Cys Val Gly Arg Glu Ile Leu Gly Ser
305                 310                 315                 320

aaa gcg gct ttt gac tat gat ccg acc aaa ttg atc ata tct gtg aaa     1008
Lys Ala Ala Phe Asp Tyr Asp Pro Thr Lys Leu Ile Ile Ser Val Lys
                325                 330                 335

gac ctc ggc ctg acc ggt cat gat gtg gaa aaa tgg ctg cgc gag tca     1056
Asp Leu Gly Leu Thr Gly His Asp Val Glu Lys Trp Leu Arg Glu Ser
            340                 345                 350

tgc caa atc gaa gtg gag ctt tct gac tta tac aac atc ctg tgc att     1104
Cys Gln Ile Glu Val Glu Leu Ser Asp Leu Tyr Asn Ile Leu Cys Ile
        355                 360                 365

ttt aca ccg gga gac cgg aaa gag gat gca gat gcg tta att aaa gga     1152
Phe Thr Pro Gly Asp Arg Lys Glu Asp Ala Asp Ala Leu Ile Lys Gly
    370                 375                 380

tta acc gag att gct caa cag gcc gct tca tct gcg gaa aac aga cgc     1200
Leu Thr Glu Ile Ala Gln Gln Ala Ala Ser Ser Ala Glu Asn Arg Arg
385                 390                 395                 400

aag cct gaa gtg ctt ctg cca aac att ccg gcg ctt gcg atg aca ccg     1248
Lys Pro Glu Val Leu Leu Pro Asn Ile Pro Ala Leu Ala Met Thr Pro
                405                 410                 415

cgt gac gct ttt tac gca aac acg gag atc att ccg ttc aaa aaa gca     1296
Arg Asp Ala Phe Tyr Ala Asn Thr Glu Ile Ile Pro Phe Lys Lys Ala
            420                 425                 430

gct ggc aga atg att gcc gag ttt gtt atg gtt tat ccg cca ggg ata     1344
Ala Gly Arg Met Ile Ala Glu Phe Val Met Val Tyr Pro Pro Gly Ile
        435                 440                 445

ccg atc ttc att ccg ggc gag atc att acc gag gat aat atc aac tac     1392
Pro Ile Phe Ile Pro Gly Glu Ile Ile Thr Glu Asp Asn Ile Asn Tyr
    450                 455                 460

atc gaa aag aac ctg gaa gct ggg ctg cct gtt cag gga ccg gaa gat     1440
Ile Glu Lys Asn Leu Glu Ala Gly Leu Pro Val Gln Gly Pro Glu Asp
465                 470                 475                 480

gac acc ctc cat atg atc cgc gtt att aaa gaa cag cag gca atc ctg     1488
Asp Thr Leu His Met Ile Arg Val Ile Lys Glu Gln Gln Ala Ile Leu
                485                 490                 495

taa aaa                                                             1494


<210>  6
<211>  496
<212>  PRT
<213>  Bacillus licheniformis DSM 13

<400>  6

Met Lys Val Gly Lys Gln Leu Leu Gln His Glu Thr Pro Leu Tyr Thr
1               5                   10                  15


Gly Leu Lys Lys His Ala Gly Lys Asn Pro Ile Gln Phe His Ile Pro
            20                  25                  30


Gly His Lys Lys Gly Ser Gly Met Asp Pro Glu Phe Arg Glu Phe Ile
        35                  40                  45


Gly Glu Asn Ala Leu Ser Ile Asp Leu Ile Asn Ile Glu Pro Leu Asp
    50                  55                  60


Asp Leu His Ala Pro Lys Gly Ile Ile Lys Gln Ala Gln Asp Leu Ala
65                  70                  75                  80


Ala Glu Ala Phe Gly Ala Asp Tyr Thr Phe Phe Ser Val Gln Gly Thr
                85                  90                  95


Ser Gly Ala Ile Met Thr Met Val Met Ala Val Cys Gly Pro Gly Asp
            100                 105                 110

Lys Ile Ile Val Pro Arg Asn Val His Lys Ser Val Met Ser Ala Ile
        115                 120                 125

Val Phe Ser Gly Ala Val Pro Ile Phe Ile His Pro Glu Ile Asp Asp
    130                 135                 140

Glu Leu Gly Ile Ser His Gly Ile Thr Pro Glu Ser Ala Lys Lys Ala
145                 150                 155                 160

Leu Leu Glu His Pro Asp Ala Lys Gly Leu Leu Val Ile Asn Pro Thr
                165                 170                 175

Tyr Phe Gly Ile Ala Ala Asp Leu Lys Ser Ile Val Glu Leu Ala His
            180                 185                 190

Ser Phe His Val Pro Val Leu Val Asp Glu Ala His Gly Val His Ile
        195                 200                 205

His Phe His Glu Asp Leu Pro Leu Ser Ala Met Gln Ala Gly Ala Asp
    210                 215                 220

Met Ala Ala Thr Ser Val His Lys Leu Gly Gly Ser Leu Thr Gln Ser
225                 230                 235                 240

Ser Ile Leu Asn Met Lys Glu Gly Leu Val Ser Lys Glu Arg Val Gln
                245                 250                 255

Ser Ile Leu Ser Met Leu Thr Thr Thr Ser Thr Ser Tyr Leu Leu Leu
            260                 265                 270

Ala Ser Leu Asp Val Ala Arg Lys Arg Leu Ala Thr Glu Gly His Glu
        275                 280                 285

Leu Ile Glu Gln Thr Ile Lys Leu Ala Asn Glu Thr Arg Glu Arg Ile
    290                 295                 300

Asn Asn Ile Asn Gly Ile Ser Cys Val Gly Arg Glu Ile Leu Gly Ser
305                 310                 315                 320

Lys Ala Ala Phe Asp Tyr Asp Pro Thr Lys Leu Ile Ile Ser Val Lys
                325                 330                 335

Asp Leu Gly Leu Thr Gly His Asp Val Glu Lys Trp Leu Arg Glu Ser
            340                 345                 350

Cys Gln Ile Glu Val Glu Leu Ser Asp Leu Tyr Asn Ile Leu Cys Ile
        355                 360                 365

Phe Thr Pro Gly Asp Arg Lys Glu Asp Ala Asp Ala Leu Ile Lys Gly
    370                 375                 380

Leu Thr Glu Ile Ala Gln Gln Ala Ala Ser Ser Ala Glu Asn Arg Arg
385                 390                 395                 400

Lys Pro Glu Val Leu Leu Pro Asn Ile Pro Ala Leu Ala Met Thr Pro
                405                 410                 415

Arg Asp Ala Phe Tyr Ala Asn Thr Glu Ile Ile Pro Phe Lys Lys Ala
            420                 425                 430

Ala Gly Arg Met Ile Ala Glu Phe Val Met Val Tyr Pro Pro Gly Ile
        435                 440                 445

Pro Ile Phe Ile Pro Gly Glu Ile Ile Thr Glu Asp Asn Ile Asn Tyr
    450                 455                 460

Ile Glu Lys Asn Leu Glu Ala Gly Leu Pro Val Gln Gly Pro Glu Asp
465                 470                 475                 480

Asp Thr Leu His Met Ile Arg Val Ile Lys Glu Gln Gln Ala Ile Leu
                485                 490                 495

<210>  7
<211>  1167
<212>  DNA
<213>  Bacillus licheniformis DSM 13

<220>
<221>  gene
<222>  (1)..(1167)
<223>  NADH-dependent butanol dehydrogenase A (E.C. 1.1.1.-),  yugJ

<220>
<221>  CDS
<222>  (1)..(1164)
<223>

<400>  7
atg gat aac ttt aca tat tgg aat ccc aca aag ctg ata ttc ggc cgg       48
Met Asp Asn Phe Thr Tyr Trp Asn Pro Thr Lys Leu Ile Phe Gly Arg
1               5                   10                  15

gga gaa gtt gaa aag ctg gca gaa gag gtg aaa caa tac ggc cgc aat          96
Gly Glu Val Glu Lys Leu Ala Glu Glu Val Lys Gln Tyr Gly Arg Asn
            20                  25                  30

gtc ctg ctc gta tac ggc gga ggc agc att aag cga aac ggt tta tac         144
Val Leu Leu Val Tyr Gly Gly Gly Ser Ile Lys Arg Asn Gly Leu Tyr
        35                  40                  45

gat caa gtc att tca atc ctt gaa aag gcg ggc gcg acc gtc cat gaa         192
Asp Gln Val Ile Ser Ile Leu Glu Lys Ala Gly Ala Thr Val His Glu
    50                  55                  60

ctg ccc ggc gtc gaa ccg aat ccg cgt gtt gcc act gtg aat aaa gga         240
Leu Pro Gly Val Glu Pro Asn Pro Arg Val Ala Thr Val Asn Lys Gly
65                  70                  75                  80

gtt gcg atc tgc aaa gag aac gat att gac ttt ctt ttg gca gtc ggc         288
Val Ala Ile Cys Lys Glu Asn Asp Ile Asp Phe Leu Leu Ala Val Gly
                85                  90                  95

ggc gga agc gtc att gat tgt acg aaa gca att gct gcc gga gcg aaa         336
Gly Gly Ser Val Ile Asp Cys Thr Lys Ala Ile Ala Ala Gly Ala Lys
            100                 105                 110

tac gac ggc gat gcg tgg gat att gtg acg aaa aaa cat att ccg gct         384
Tyr Asp Gly Asp Ala Trp Asp Ile Val Thr Lys Lys His Ile Pro Ala
        115                 120                 125

gat gcg ctg ccg ttt gga aca gtt tta acg tta gca gca aca ggc tct         432
Asp Ala Leu Pro Phe Gly Thr Val Leu Thr Leu Ala Ala Thr Gly Ser
    130                 135                 140

gaa atg aac tcg gga tct gtg atc aca aat tgg gaa acc aat gaa aaa         480
Glu Met Asn Ser Gly Ser Val Ile Thr Asn Trp Glu Thr Asn Glu Lys
145                 150                 155                 160

tac ggc tgg gga agc ccg ctc gta ttc cct aaa ttt tca att ctt gat         528
Tyr Gly Trp Gly Ser Pro Leu Val Phe Pro Lys Phe Ser Ile Leu Asp
                165                 170                 175

ccg gtc aac acg ttt acc gtc ccg aaa gac cac acg att tac ggc att         576
Pro Val Asn Thr Phe Thr Val Pro Lys Asp His Thr Ile Tyr Gly Ile
            180                 185                 190

gtc gac atg atg tcc cac gtg ttt gag caa tat ttt cac cat acc gaa         624
Val Asp Met Met Ser His Val Phe Glu Gln Tyr Phe His His Thr Glu
        195                 200                 205

aat acc cct tat cag gac cgg atg tgc gaa tcc ctg ctt aaa acg gta         672
Asn Thr Pro Tyr Gln Asp Arg Met Cys Glu Ser Leu Leu Lys Thr Val
    210                 215                 220

att gaa aca gct cct aag ctc att gaa gac cta gaa aac tat gag ctg         720
Ile Glu Thr Ala Pro Lys Leu Ile Glu Asp Leu Glu Asn Tyr Glu Leu
225                 230                 235                 240

cgt gaa acg att ctg tat aca ggc acc att gcg ctg aac ggc atg cta         768
Arg Glu Thr Ile Leu Tyr Thr Gly Thr Ile Ala Leu Asn Gly Met Leu
                245                 250                 255

tca atg ggc gca cgc gga gac tgg gca acg cac aat atc gag cac gct      816
Ser Met Gly Ala Arg Gly Asp Trp Ala Thr His Asn Ile Glu His Ala
            260                 265                 270

gtt tca gcc gta tac gat att ccg cat gcg gga ggg ctt gcg att ctg      864
Val Ser Ala Val Tyr Asp Ile Pro His Ala Gly Gly Leu Ala Ile Leu
        275                 280                 285

ttc ccg aat tgg atg aag cac acg ctt tcc gag aac gtc ggc cgc ttt      912
Phe Pro Asn Trp Met Lys His Thr Leu Ser Glu Asn Val Gly Arg Phe
    290                 295                 300

aaa cag ctt gcc gtc cgt gtt ttt gac gta gat gaa aca gga aaa acc      960
Lys Gln Leu Ala Val Arg Val Phe Asp Val Asp Glu Thr Gly Lys Thr
305                 310                 315                 320

gat cgc gaa gtg gcg ctt gtt gga atc gag aaa ctg tct gaa ttc tgg     1008
Asp Arg Glu Val Ala Leu Val Gly Ile Glu Lys Leu Ser Glu Phe Trp
                325                 330                 335

acc agc ctt ggc gcg cca aat cgt ctt gcc gat tat gac att aca gat     1056
Thr Ser Leu Gly Ala Pro Asn Arg Leu Ala Asp Tyr Asp Ile Thr Asp
            340                 345                 350

gag aag ctt gat ctc att gcc gac aaa gcg atg gca aac ggc gaa ttc     1104
Glu Lys Leu Asp Leu Ile Ala Asp Lys Ala Met Ala Asn Gly Glu Phe
        355                 360                 365

ggc cgc ttt aaa acg ctg aat aaa gac gat gtt ctg tct att ttg aag     1152
Gly Arg Phe Lys Thr Leu Asn Lys Asp Asp Val Leu Ser Ile Leu Lys
    370                 375                 380

gct tct tta taa gat                                                 1167
Ala Ser Leu
385


<210>  8
<211>  387
<212>  PRT
<213>  Bacillus licheniformis DSM 13

<400>  8

Met Asp Asn Phe Thr Tyr Trp Asn Pro Thr Lys Leu Ile Phe Gly Arg
1               5                   10                  15


Gly Glu Val Glu Lys Leu Ala Glu Glu Val Lys Gln Tyr Gly Arg Asn
            20                  25                  30


Val Leu Leu Val Tyr Gly Gly Gly Ser Ile Lys Arg Asn Gly Leu Tyr
        35                  40                  45


Asp Gln Val Ile Ser Ile Leu Glu Lys Ala Gly Ala Thr Val His Glu
    50                  55                  60

Leu Pro Gly Val Glu Pro Asn Pro Arg Val Ala Thr Val Asn Lys Gly
65                  70                  75                  80

Val Ala Ile Cys Lys Glu Asn Asp Ile Asp Phe Leu Leu Ala Val Gly
                85                  90                  95

Gly Gly Ser Val Ile Asp Cys Thr Lys Ala Ile Ala Ala Gly Ala Lys
            100                 105                 110

Tyr Asp Gly Asp Ala Trp Asp Ile Val Thr Lys Lys His Ile Pro Ala
        115                 120                 125

Asp Ala Leu Pro Phe Gly Thr Val Leu Thr Leu Ala Ala Thr Gly Ser
    130                 135                 140

Glu Met Asn Ser Gly Ser Val Ile Thr Asn Trp Glu Thr Asn Glu Lys
145                 150                 155                 160

Tyr Gly Trp Gly Ser Pro Leu Val Phe Pro Lys Phe Ser Ile Leu Asp
                165                 170                 175

Pro Val Asn Thr Phe Thr Val Pro Lys Asp His Thr Ile Tyr Gly Ile
            180                 185                 190

Val Asp Met Met Ser His Val Phe Glu Gln Tyr Phe His His Thr Glu
        195                 200                 205

Asn Thr Pro Tyr Gln Asp Arg Met Cys Glu Ser Leu Leu Lys Thr Val
    210                 215                 220

Ile Glu Thr Ala Pro Lys Leu Ile Glu Asp Leu Glu Asn Tyr Glu Leu
225                 230                 235                 240

Arg Glu Thr Ile Leu Tyr Thr Gly Thr Ile Ala Leu Asn Gly Met Leu
                245                 250                 255

Ser Met Gly Ala Arg Gly Asp Trp Ala Thr His Asn Ile Glu His Ala
            260                 265                 270

Val Ser Ala Val Tyr Asp Ile Pro His Ala Gly Gly Leu Ala Ile Leu
        275                 280                 285

Phe Pro Asn Trp Met Lys His Thr Leu Ser Glu Asn Val Gly Arg Phe

290                 295                 300

Lys Gln Leu Ala Val Arg Val Phe Asp Val Asp Glu Thr Gly Lys Thr
305                 310                 315                 320

Asp Arg Glu Val Ala Leu Val Gly Ile Glu Lys Leu Ser Glu Phe Trp
                325                 330                 335

Thr Ser Leu Gly Ala Pro Asn Arg Leu Ala Asp Tyr Asp Ile Thr Asp
            340                 345                 350

Glu Lys Leu Asp Leu Ile Ala Asp Lys Ala Met Ala Asn Gly Glu Phe
        355                 360                 365

Gly Arg Phe Lys Thr Leu Asn Lys Asp Asp Val Leu Ser Ile Leu Lys
    370                 375                 380

Ala Ser Leu
385

<210>  9
<211>  1143
<212>  DNA
<213>  Bacillus licheniformis DSM 13

<220>
<221>  gene
<222>  (1)..(1143)
<223>  Acyl-CoA dehydrogenase (E.C. 1.3.99.-)

<220>
<221>  CDS
<222>  (1)..(1140)
<223>

<400>  9
atg atc ttt aag ctg agc gaa gag cac caa atg ata cag aag atg gtg       48
Met Ile Phe Lys Leu Ser Glu Glu His Gln Met Ile Gln Lys Met Val
1               5                   10                  15

cgc gac ttt gcc cat cac gag gtg gag ccg act gcg aaa gag cgg gat       96
Arg Asp Phe Ala His His Glu Val Glu Pro Thr Ala Lys Glu Arg Asp
            20                  25                  30

gag gaa gag cgg ttt gac atg gaa ctg ttt gca aaa atg gcg gaa ttg      144
Glu Glu Glu Arg Phe Asp Met Glu Leu Phe Ala Lys Met Ala Glu Leu
        35                  40                  45

ggg ctc acg ggt ata ccg tgg cca gag gaa tac ggg gga atc ggc agc      192
Gly Leu Thr Gly Ile Pro Trp Pro Glu Glu Tyr Gly Gly Ile Gly Ser

50                  55                  60

gac tat ctc gcc tac gtc atc gcg gtt gaa gag ctt tcg aaa gtc tgc      240
Asp Tyr Leu Ala Tyr Val Ile Ala Val Glu Glu Leu Ser Lys Val Cys
65                  70                  75                  80

gcc tca acg ggt gtc acc ttg tct gcc cat aca tcg ctt gcg ggc tgg      288
Ala Ser Thr Gly Val Thr Leu Ser Ala His Thr Ser Leu Ala Gly Trp
                85                  90                  95

ccg att tat gca ttt gga aca gaa gaa cag aaa cag gaa tat tta aag      336
Pro Ile Tyr Ala Phe Gly Thr Glu Glu Gln Lys Gln Glu Tyr Leu Lys
            100                 105                 110

ccg atg gcc cgc ggc gaa aag ata gga gcc tac gga ttg acg gag ccc      384
Pro Met Ala Arg Gly Glu Lys Ile Gly Ala Tyr Gly Leu Thr Glu Pro
        115                 120                 125

ggt tca ggc tcc gat gcc ggc ggc atg aaa acg acc gca gaa aaa aaa      432
Gly Ser Gly Ser Asp Ala Gly Gly Met Lys Thr Thr Ala Glu Lys Lys
    130                 135                 140

ggc gat gaa tat att ttg aac ggg acg aag atc ttt atc aca aac ggc      480
Gly Asp Glu Tyr Ile Leu Asn Gly Thr Lys Ile Phe Ile Thr Asn Gly
145                 150                 155                 160

gga atc gcg gac ttc tac atc gtg ttt gcc aac ctt gcc ccg gaa cag      528
Gly Ile Ala Asp Phe Tyr Ile Val Phe Ala Asn Leu Ala Pro Glu Gln
                165                 170                 175

aaa cac aaa ggc aca acc gcc ttt atc gtt gaa aag gac ttc ccc ggc      576
Lys His Lys Gly Thr Thr Ala Phe Ile Val Glu Lys Asp Phe Pro Gly
            180                 185                 190

ttt tct gtc gga aaa aaa gag agg aaa ctg ggc atc cgt tca tca ccg      624
Phe Ser Val Gly Lys Lys Glu Arg Lys Leu Gly Ile Arg Ser Ser Pro
        195                 200                 205

aca acc gaa atc atc ttt cag gac tgc cgc gtg cct tta aaa aac cgc      672
Thr Thr Glu Ile Ile Phe Gln Asp Cys Arg Val Pro Leu Lys Asn Arg
    210                 215                 220

ctt ggc ggg gaa ggt gaa ggc ttt aaa atc gcg atg aag acg ctc gat      720
Leu Gly Gly Glu Gly Glu Gly Phe Lys Ile Ala Met Lys Thr Leu Asp
225                 230                 235                 240

gga ggc aga aac gga att gcc gct caa gcc gtc ggg att gcc cag ggc      768
Gly Gly Arg Asn Gly Ile Ala Ala Gln Ala Val Gly Ile Ala Gln Gly
                245                 250                 255

gca ttt gag gcg gcg aag gcg tat gcg aaa gag cgg aag caa ttc ggc      816
Ala Phe Glu Ala Ala Lys Ala Tyr Ala Lys Glu Arg Lys Gln Phe Gly
            260                 265                 270

agg ccg atc gcc gag cag cag ggc atc gct ttt aaa ctg gct gat atg      864
Arg Pro Ile Ala Glu Gln Gln Gly Ile Ala Phe Lys Leu Ala Asp Met
        275                 280                 285

gcg acg gag att gaa gct tca agg ctt tta acc tac cag gcg gca tgg      912

Ala Thr Glu Ile Glu Ala Ser Arg Leu Leu Thr Tyr Gln Ala Ala Trp
    290                 295                 300

ctg gaa tca gaa gga ctg cct tat gga aag gct tct gcc atg tca aag      960
Leu Glu Ser Glu Gly Leu Pro Tyr Gly Lys Ala Ser Ala Met Ser Lys
305                 310                 315                 320

ctt tac gca ggg gat acg gcc atg aaa gtg acg acg gag gcc gtg caa     1008
Leu Tyr Ala Gly Asp Thr Ala Met Lys Val Thr Thr Glu Ala Val Gln
                325                 330                 335

ata ttc ggc ggg tac ggc tac aca aag gat tat ccg gtc gag cgc ttt     1056
Ile Phe Gly Gly Tyr Gly Tyr Thr Lys Asp Tyr Pro Val Glu Arg Phe
            340                 345                 350

atg cgc gat gca aaa atc aca cag atc tat gaa ggt acg cag gaa att     1104
Met Arg Asp Ala Lys Ile Thr Gln Ile Tyr Glu Gly Thr Gln Glu Ile
        355                 360                 365

cag aag ctc gtc att tcg aga atg ctg atg aaa taa ggg                 1143
Gln Lys Leu Val Ile Ser Arg Met Leu Met Lys
    370                 375


<210>  10
<211>  379
<212>  PRT
<213>  Bacillus licheniformis DSM 13

<400>  10

Met Ile Phe Lys Leu Ser Glu Glu His Gln Met Ile Gln Lys Met Val
1               5                   10                  15


Arg Asp Phe Ala His His Glu Val Glu Pro Thr Ala Lys Glu Arg Asp
            20                  25                  30


Glu Glu Glu Arg Phe Asp Met Glu Leu Phe Ala Lys Met Ala Glu Leu
        35                  40                  45


Gly Leu Thr Gly Ile Pro Trp Pro Glu Glu Tyr Gly Gly Ile Gly Ser
    50                  55                  60


Asp Tyr Leu Ala Tyr Val Ile Ala Val Glu Glu Leu Ser Lys Val Cys
65                  70                  75                  80


Ala Ser Thr Gly Val Thr Leu Ser Ala His Thr Ser Leu Ala Gly Trp
                85                  90                  95


Pro Ile Tyr Ala Phe Gly Thr Glu Glu Gln Lys Gln Glu Tyr Leu Lys
            100                 105                 110

Pro Met Ala Arg Gly Glu Lys Ile Gly Ala Tyr Gly Leu Thr Glu Pro
        115                 120                 125

Gly Ser Gly Ser Asp Ala Gly Gly Met Lys Thr Thr Ala Glu Lys Lys
    130                 135                 140

Gly Asp Glu Tyr Ile Leu Asn Gly Thr Lys Ile Phe Ile Thr Asn Gly
145                 150                 155                 160

Gly Ile Ala Asp Phe Tyr Ile Val Phe Ala Asn Leu Ala Pro Glu Gln
                165                 170                 175

Lys His Lys Gly Thr Thr Ala Phe Ile Val Glu Lys Asp Phe Pro Gly
            180                 185                 190

Phe Ser Val Gly Lys Lys Glu Arg Lys Leu Gly Ile Arg Ser Ser Pro
        195                 200                 205

Thr Thr Glu Ile Ile Phe Gln Asp Cys Arg Val Pro Leu Lys Asn Arg
    210                 215                 220

Leu Gly Gly Glu Gly Glu Gly Phe Lys Ile Ala Met Lys Thr Leu Asp
225                 230                 235                 240

Gly Gly Arg Asn Gly Ile Ala Ala Gln Ala Val Gly Ile Ala Gln Gly
                245                 250                 255

Ala Phe Glu Ala Ala Lys Ala Tyr Ala Lys Glu Arg Lys Gln Phe Gly
            260                 265                 270

Arg Pro Ile Ala Glu Gln Gln Gly Ile Ala Phe Lys Leu Ala Asp Met
        275                 280                 285

Ala Thr Glu Ile Glu Ala Ser Arg Leu Leu Thr Tyr Gln Ala Ala Trp
    290                 295                 300

Leu Glu Ser Glu Gly Leu Pro Tyr Gly Lys Ala Ser Ala Met Ser Lys
305                 310                 315                 320

Leu Tyr Ala Gly Asp Thr Ala Met Lys Val Thr Thr Glu Ala Val Gln
                325                 330                 335

Ile Phe Gly Gly Tyr Gly Tyr Thr Lys Asp Tyr Pro Val Glu Arg Phe
            340                 345                 350

Met Arg Asp Ala Lys Ile Thr Gln Ile Tyr Glu Gly Thr Gln Glu Ile
        355                 360                 365

Gln Lys Leu Val Ile Ser Arg Met Leu Met Lys
    370                 375

<210>  11
<211>  1134
<212>  DNA
<213>  Bacillus licheniformis DSM 13

<220>
<221>  gene
<222>  (1)..(1134)
<223>  Acyl-CoA dehydrogenase (E.C. 1.3.99.-)

<220>
<221>  misc_feature
<222>  (1)..(3)
<223>  First codon translated as Met.

<220>
<221>  CDS
<222>  (1)..(1131)
<223>

<400>  11
ttg aat ttt cag ttt aca gaa gag cag acc cgc atg cag aag gtt gta       48
Leu Asn Phe Gln Phe Thr Glu Glu Gln Thr Arg Met Gln Lys Val Val
1               5                   10                  15

agg gat ttt gtc aaa cag gag gtc gct cct ttt gta cct gaa atg gaa       96
Arg Asp Phe Val Lys Gln Glu Val Ala Pro Phe Val Pro Glu Met Glu
            20                  25                  30

aaa ggc cgt ttt cca acc tct ctt ttg aag aaa atg gcg gag cgc ggc      144
Lys Gly Arg Phe Pro Thr Ser Leu Leu Lys Lys Met Ala Glu Arg Gly
        35                  40                  45

tgg atg ggg ctt ccg ata ccg gct aag tac aac ggg gcc ggt cat gac      192
Trp Met Gly Leu Pro Ile Pro Ala Lys Tyr Asn Gly Ala Gly His Asp
    50                  55                  60

ttt atc acc tat atg atg acc ata cat gag ctg tca aag aaa agc gcc      240
Phe Ile Thr Tyr Met Met Thr Ile His Glu Leu Ser Lys Lys Ser Ala
65                  70                  75                  80

gtt ctt ggt gcg gtt cta tct gta cac acg tca att gtc acc att ccc      288
Val Leu Gly Ala Val Leu Ser Val His Thr Ser Ile Val Thr Ile Pro
                85                  90                  95

att ctt tta aac ggt aat gag cgg caa aaa gag cac tac gtt aaa aag      336
Ile Leu Leu Asn Gly Asn Glu Arg Gln Lys Glu His Tyr Val Lys Lys

            100                 105                 110

ctg gca gca ggg cag tac ttg gga gct ttt tgt tta acc gaa ccg agt 384
Leu Ala Ala Gly Gln Tyr Leu Gly Ala Phe Cys Leu Thr Glu Pro Ser
        115                 120                 125

gcc ggt tcc gat gcg ggc agt cta aaa acc agg gcc gaa aag cgc ggc 432
Ala Gly Ser Asp Ala Gly Ser Leu Lys Thr Arg Ala Glu Lys Arg Gly
    130                 135                 140

gat acc tat gtg ctg aac gga acg aaa gtt ttt att aca aac ggc gga 480
Asp Thr Tyr Val Leu Asn Gly Thr Lys Val Phe Ile Thr Asn Gly Gly
145                 150                 155                 160

gcc gcg gac att tat ctt gtt ttc gct tca acc gat ccg gag gcg gga 528
Ala Ala Asp Ile Tyr Leu Val Phe Ala Ser Thr Asp Pro Glu Ala Gly
                165                 170                 175

acg gga ggc att tcc gct ttt atc gtg gag aag ggc acg ccg ggc ttt 576
Thr Gly Gly Ile Ser Ala Phe Ile Val Glu Lys Gly Thr Pro Gly Phe
            180                 185                 190

ttc atc gga aaa aat gaa gag aaa atg ggg ctc cac ggt tcg cta acg 624
Phe Ile Gly Lys Asn Glu Glu Lys Met Gly Leu His Gly Ser Leu Thr
        195                 200                 205

gtc act tta aat ttt gat aac gct gtc att ccc gcc cgg cag ctg ctg 672
Val Thr Leu Asn Phe Asp Asn Ala Val Ile Pro Ala Arg Gln Leu Leu
    210                 215                 220

ggg gag gaa gga atg gga ttc aaa atg gcc ctg tcc aac ctg gat acc 720
Gly Glu Glu Gly Met Gly Phe Lys Met Ala Leu Ser Asn Leu Asp Thr
225                 230                 235                 240

ggc cgg atc gga att gcg gcg cag gct ctg ggt atc gcc gag gga gcg 768
Gly Arg Ile Gly Ile Ala Ala Gln Ala Leu Gly Ile Ala Glu Gly Ala
                245                 250                 255

ctt tct gaa gcc gtt caa ttt cta aaa aaa cgg tat ccg gac gga gag 816
Leu Ser Glu Ala Val Gln Phe Leu Lys Lys Arg Tyr Pro Asp Gly Glu
            260                 265                 270

ctg tat aag aac ggc caa gcg ctt gcc ttt aag ctg gcc gac atg gcg 864
Leu Tyr Lys Asn Gly Gln Ala Leu Ala Phe Lys Leu Ala Asp Met Ala
        275                 280                 285

gcg agg aca gag gcg gcc agg ctt ctc gtc tac cag gcc gcc tcg ttg 912
Ala Arg Thr Glu Ala Ala Arg Leu Leu Val Tyr Gln Ala Ala Ser Leu
    290                 295                 300

aaa cag caa ggc atg cag acg ggg aaa gct gct tca atg gcc aaa ttg 960
Lys Gln Gln Gly Met Gln Thr Gly Lys Ala Ala Ser Met Ala Lys Leu
305                 310                 315                 320

ttc gct tcg gaa acg gcg atg tat gtc gcc gga gag gcc gtg cag ctg 1008
Phe Ala Ser Glu Thr Ala Met Tyr Val Ala Gly Glu Ala Val Gln Leu
                325                 330                 335

ctc gga gat ttc gga tac aca aag gat ttc agc gct gaa cga tat ttc 1056

Leu Gly Asp Phe Gly Tyr Thr Lys Asp Phe Ser Ala Glu Arg Tyr Phe
            340                 345                 350

agg gat gcg aaa gtg tgt gaa att tat gag ggc acg agc gag atc cag      1104
Arg Asp Ala Lys Val Cys Glu Ile Tyr Glu Gly Thr Ser Glu Ile Gln
        355                 360                 365

cgg atc gtc atc ggt aaa cat tta taa ggt                              1134
Arg Ile Val Ile Gly Lys His Leu
    370                 375


<210>  12
<211>  376
<212>  PRT
<213>  Bacillus licheniformis DSM 13

<220>
<221>  misc_feature
<222>  (1)..(3)
<223>  First codon translated as Met.

<400>  12

Leu Asn Phe Gln Phe Thr Glu Glu Gln Thr Arg Met Gln Lys Val Val
1               5                   10                  15


Arg Asp Phe Val Lys Gln Glu Val Ala Pro Phe Val Pro Glu Met Glu
            20                  25                  30


Lys Gly Arg Phe Pro Thr Ser Leu Leu Lys Lys Met Ala Glu Arg Gly
        35                  40                  45


Trp Met Gly Leu Pro Ile Pro Ala Lys Tyr Asn Gly Ala Gly His Asp
    50                  55                  60


Phe Ile Thr Tyr Met Met Thr Ile His Glu Leu Ser Lys Lys Ser Ala
65                  70                  75                  80


Val Leu Gly Ala Val Leu Ser Val His Thr Ser Ile Val Thr Ile Pro
                85                  90                  95


Ile Leu Leu Asn Gly Asn Glu Arg Gln Lys Glu His Tyr Val Lys Lys
            100                 105                 110


Leu Ala Ala Gly Gln Tyr Leu Gly Ala Phe Cys Leu Thr Glu Pro Ser
        115                 120                 125


Ala Gly Ser Asp Ala Gly Ser Leu Lys Thr Arg Ala Glu Lys Arg Gly
    130                 135                 140

Asp Thr Tyr Val Leu Asn Gly Thr Lys Val Phe Ile Thr Asn Gly Gly
145                 150                 155                 160

Ala Ala Asp Ile Tyr Leu Val Phe Ala Ser Thr Asp Pro Glu Ala Gly
                165                 170                 175

Thr Gly Gly Ile Ser Ala Phe Ile Val Glu Lys Gly Thr Pro Gly Phe
            180                 185                 190

Phe Ile Gly Lys Asn Glu Glu Lys Met Gly Leu His Gly Ser Leu Thr
        195                 200                 205

Val Thr Leu Asn Phe Asp Asn Ala Val Ile Pro Ala Arg Gln Leu Leu
    210                 215                 220

Gly Glu Glu Gly Met Gly Phe Lys Met Ala Leu Ser Asn Leu Asp Thr
225                 230                 235                 240

Gly Arg Ile Gly Ile Ala Ala Gln Ala Leu Gly Ile Ala Glu Gly Ala
                245                 250                 255

Leu Ser Glu Ala Val Gln Phe Leu Lys Lys Arg Tyr Pro Asp Gly Glu
            260                 265                 270

Leu Tyr Lys Asn Gly Gln Ala Leu Ala Phe Lys Leu Ala Asp Met Ala
        275                 280                 285

Ala Arg Thr Glu Ala Ala Arg Leu Leu Val Tyr Gln Ala Ala Ser Leu
    290                 295                 300

Lys Gln Gln Gly Met Gln Thr Gly Lys Ala Ala Ser Met Ala Lys Leu
305                 310                 315                 320

Phe Ala Ser Glu Thr Ala Met Tyr Val Ala Gly Glu Ala Val Gln Leu
                325                 330                 335

Leu Gly Asp Phe Gly Tyr Thr Lys Asp Phe Ser Ala Glu Arg Tyr Phe
            340                 345                 350

Arg Asp Ala Lys Val Cys Glu Ile Tyr Glu Gly Thr Ser Glu Ile Gln
        355                 360                 365

Arg Ile Val Ile Gly Lys His Leu
    370                 375

<210>  13
<211>  867
<212>  DNA
<213>  Bacillus licheniformis DSM 13

<220>
<221>  gene
<222>  (1)..(867)
<223>  3-hydroxybutyryl-CoA dehydrogenase (E.C. 1.1.1.157)


<220>
<221>  CDS
<222>  (1)..(864)
<223>


<400>  13
atg aaa aaa gac acg atc atg gtc atc gga gcc ggc cag atg ggg tcc       48
Met Lys Lys Asp Thr Ile Met Val Ile Gly Ala Gly Gln Met Gly Ser
1               5                   10                  15

ggg atc gct cag gtc tct gcc cag gcc gga tac aat gtt tac atg tat       96
Gly Ile Ala Gln Val Ser Ala Gln Ala Gly Tyr Asn Val Tyr Met Tyr
            20                  25                  30

gac gta tca cct gaa caa att gaa aaa gga atg aag cgc att tcc ggc      144
Asp Val Ser Pro Glu Gln Ile Glu Lys Gly Met Lys Arg Ile Ser Gly
        35                  40                  45

cag ctt ttc aga cag gcg gaa aaa ggc aag ctg ccg cat gaa gat gtg      192
Gln Leu Phe Arg Gln Ala Glu Lys Gly Lys Leu Pro His Glu Asp Val
    50                  55                  60

aag caa atc tac cag cgc ctt tct ccg tcg gct gcg ctc gac gaa gcg      240
Lys Gln Ile Tyr Gln Arg Leu Ser Pro Ser Ala Ala Leu Asp Glu Ala
65                  70                  75                  80

cgg gaa gct ttt ctc atc att gaa gcg gct gtt gaa caa atg gac gta      288
Arg Glu Ala Phe Leu Ile Ile Glu Ala Ala Val Glu Gln Met Asp Val
                85                  90                  95

aaa aaa gac att ttc acg cgg ctt gat gaa gtg acc gaa gac tca gcg      336
Lys Lys Asp Ile Phe Thr Arg Leu Asp Glu Val Thr Glu Asp Ser Ala
            100                 105                 110

ata ttg gca tca aat aca tcg tcc ctg tcg att acg gaa ctt gct gcc      384
Ile Leu Ala Ser Asn Thr Ser Ser Leu Ser Ile Thr Glu Leu Ala Ala
        115                 120                 125

gtc aca aaa aaa cct caa aac gtc atc ggc atg cat ttt atg aac ccg      432
Val Thr Lys Lys Pro Gln Asn Val Ile Gly Met His Phe Met Asn Pro
    130                 135                 140

gtt ccc gtc atg cag ctt gtc gaa gtc atc agg ggg ctt gag acg agc      480
Val Pro Val Met Gln Leu Val Glu Val Ile Arg Gly Leu Glu Thr Ser
145                 150                 155                 160

ggc gaa aca tat gaa acc gtc gtg gct gcg gcg gag cgg atg aac aag      528
Gly Glu Thr Tyr Glu Thr Val Val Ala Ala Ala Glu Arg Met Asn Lys
                165                 170                 175

gtt ccg ata gag gtg cgg gat ttc ccg gga ttt atc tcc aac cgc atc      576
Val Pro Ile Glu Val Arg Asp Phe Pro Gly Phe Ile Ser Asn Arg Ile
            180                 185                 190

tta atg ccg atg att aat gag gcg gtt ttt gca ctt tat gaa ggc atc      624
Leu Met Pro Met Ile Asn Glu Ala Val Phe Ala Leu Tyr Glu Gly Ile
        195                 200                 205

gcc gag aaa gaa agc ata gac gga atc atg aag ctt ggc atg aac cat      672
Ala Glu Lys Glu Ser Ile Asp Gly Ile Met Lys Leu Gly Met Asn His
    210                 215                 220

ccg atg ggc ccg ttg gct ctc gcc gat ctg atc ggt ctg gat acg tgt      720
Pro Met Gly Pro Leu Ala Leu Ala Asp Leu Ile Gly Leu Asp Thr Cys
225                 230                 235                 240

ctg tat att atg gag acg ctg cat gaa ggc ttt ggc gac gat aag tac      768
Leu Tyr Ile Met Glu Thr Leu His Glu Gly Phe Gly Asp Asp Lys Tyr
                245                 250                 255

agg cct tgt ccg ctc ctt aaa caa tat gtc agc gca gga cga ctc gga      816
Arg Pro Cys Pro Leu Leu Lys Gln Tyr Val Ser Ala Gly Arg Leu Gly
            260                 265                 270

aag aaa acg ggc aga ggg ttt tat acg tat gaa aag cag ccg aca taa      864
Lys Lys Thr Gly Arg Gly Phe Tyr Thr Tyr Glu Lys Gln Pro Thr
        275                 280                 285

gga                                                                  867


<210>  14
<211>  287
<212>  PRT
<213>  Bacillus licheniformis DSM 13

<400>  14

Met Lys Lys Asp Thr Ile Met Val Ile Gly Ala Gly Gln Met Gly Ser
1               5                   10                  15


Gly Ile Ala Gln Val Ser Ala Gln Ala Gly Tyr Asn Val Tyr Met Tyr
            20                  25                  30


Asp Val Ser Pro Glu Gln Ile Glu Lys Gly Met Lys Arg Ile Ser Gly
        35                  40                  45


Gln Leu Phe Arg Gln Ala Glu Lys Gly Lys Leu Pro His Glu Asp Val
    50                  55                  60

Lys Gln Ile Tyr Gln Arg Leu Ser Pro Ser Ala Ala Leu Asp Glu Ala
65                  70                  75                  80

Arg Glu Ala Phe Leu Ile Ile Glu Ala Ala Val Glu Gln Met Asp Val
                85                  90                  95

Lys Lys Asp Ile Phe Thr Arg Leu Asp Glu Val Thr Glu Asp Ser Ala
            100                 105                 110

Ile Leu Ala Ser Asn Thr Ser Ser Leu Ser Ile Thr Glu Leu Ala Ala
        115                 120                 125

Val Thr Lys Lys Pro Gln Asn Val Ile Gly Met His Phe Met Asn Pro
    130                 135                 140

Val Pro Val Met Gln Leu Val Glu Val Ile Arg Gly Leu Glu Thr Ser
145                 150                 155                 160

Gly Glu Thr Tyr Glu Thr Val Val Ala Ala Ala Glu Arg Met Asn Lys
                165                 170                 175

Val Pro Ile Glu Val Arg Asp Phe Pro Gly Phe Ile Ser Asn Arg Ile
            180                 185                 190

Leu Met Pro Met Ile Asn Glu Ala Val Phe Ala Leu Tyr Glu Gly Ile
        195                 200                 205

Ala Glu Lys Glu Ser Ile Asp Gly Ile Met Lys Leu Gly Met Asn His
    210                 215                 220

Pro Met Gly Pro Leu Ala Leu Ala Asp Leu Ile Gly Leu Asp Thr Cys
225                 230                 235                 240

Leu Tyr Ile Met Glu Thr Leu His Glu Gly Phe Gly Asp Asp Lys Tyr
                245                 250                 255

Arg Pro Cys Pro Leu Leu Lys Gln Tyr Val Ser Ala Gly Arg Leu Gly
            260                 265                 270

Lys Lys Thr Gly Arg Gly Phe Tyr Thr Tyr Glu Lys Gln Pro Thr
        275                 280                 285

<210>  15
<211>  780
<212>  DNA

<213>  Bacillus licheniformis DSM 13

<220>
<221>  gene
<222>  (1)..(780)
<223>  Putative enoyl-CoA hydratase protein (E.C. 4.2.1.17)


<220>
<221>  CDS
<222>  (1)..(777)
<223>


<400>  15
atg gaa tat atc aaa tgg aaa gac gaa gac ggg att ttt gaa att gta       48
Met Glu Tyr Ile Lys Trp Lys Asp Glu Asp Gly Ile Phe Glu Ile Val
1               5                   10                  15

ctg aat cgc tct gag gcg tac aat gcc ttg aat gaa aaa atg ctt gaa       96
Leu Asn Arg Ser Glu Ala Tyr Asn Ala Leu Asn Glu Lys Met Leu Glu
            20                  25                  30

gaa ctg aat gaa gct ttg cgt gtt gca gag gaa agc gaa tcg ctg ctt      144
Glu Leu Asn Glu Ala Leu Arg Val Ala Glu Glu Ser Glu Ser Leu Leu
        35                  40                  45

ctc ctt gtg aga ggc agc ggc aaa gga ttt tcg gca ggc gga gac att      192
Leu Leu Val Arg Gly Ser Gly Lys Gly Phe Ser Ala Gly Gly Asp Ile
    50                  55                  60

aag atg atg ctg tct tcc gga gat caa gac agc tcc gcc cgc gtc att      240
Lys Met Met Leu Ser Ser Gly Asp Gln Asp Ser Ser Ala Arg Val Ile
65                  70                  75                  80

gac aca att tct gaa att gcg gtg aag ctt tac agc atg ccg aag atg      288
Asp Thr Ile Ser Glu Ile Ala Val Lys Leu Tyr Ser Met Pro Lys Met
                85                  90                  95

acg aca gcg gct gtc cac ggt gcg gct gcg ggt ctg gga ttg agc ctc      336
Thr Thr Ala Ala Val His Gly Ala Ala Ala Gly Leu Gly Leu Ser Leu
            100                 105                 110

gcg ctc agc tgc gac cat gta ctg gtc gaa aaa gag gcg aag ctg gcg      384
Ala Leu Ser Cys Asp His Val Leu Val Glu Lys Glu Ala Lys Leu Ala
        115                 120                 125

atg aat ttt atc ggc atc ggg ctt gtt ccc gac gga ggc gga cac ttc      432
Met Asn Phe Ile Gly Ile Gly Leu Val Pro Asp Gly Gly Gly His Phe
    130                 135                 140

ttt tta gag cgg aga atc ggt gaa act gcg gcc aaa gaa ttg att tgg      480
Phe Leu Glu Arg Arg Ile Gly Glu Thr Ala Ala Lys Glu Leu Ile Trp
145                 150                 155                 160

agc ggg aaa aaa ttg acg ggg gcc gaa gcg cac gag ctt cgg atc gca      528
Ser Gly Lys Lys Leu Thr Gly Ala Glu Ala His Glu Leu Arg Ile Ala
                165                 170                 175

gac gcc gta ttc agc ggg gac tcc ggc cgt ttt gcg cgc atc tat ctt      576
Asp Ala Val Phe Ser Gly Asp Ser Gly Arg Phe Ala Arg Ile Tyr Leu
            180                 185                 190

gaa aag ctt ctg cac gct ccg ctg gca gcg atg att gag aca aaa aag      624
Glu Lys Leu Leu His Ala Pro Leu Ala Ala Met Ile Glu Thr Lys Lys
        195                 200                 205

atc tat cag gcg ttg aat gga ggc agg ctg cag aaa acg ctt gaa ctc      672
Ile Tyr Gln Ala Leu Asn Gly Gly Arg Leu Gln Lys Thr Leu Glu Leu
    210                 215                 220

gag aaa acg gcc cag atg aaa atg agg ctg aca agc gac cat cag gaa      720
Glu Lys Thr Ala Gln Met Lys Met Arg Leu Thr Ser Asp His Gln Glu
225                 230                 235                 240

ggg atc cgc gca ttt tta gaa aag cgc cag ccg caa ttt aac cgt cag      768
Gly Ile Arg Ala Phe Leu Glu Lys Arg Gln Pro Gln Phe Asn Arg Gln
                245                 250                 255

caa gta taa caa                                                      780
Gln Val

<210>  16
<211>  258
<212>  PRT
<213>  Bacillus licheniformis DSM 13

<400>  16

Met Glu Tyr Ile Lys Trp Lys Asp Glu Asp Gly Ile Phe Glu Ile Val
1               5                   10                  15

Leu Asn Arg Ser Glu Ala Tyr Asn Ala Leu Asn Glu Lys Met Leu Glu
            20                  25                  30

Glu Leu Asn Glu Ala Leu Arg Val Ala Glu Glu Ser Glu Ser Leu Leu
        35                  40                  45

Leu Leu Val Arg Gly Ser Gly Lys Gly Phe Ser Ala Gly Gly Asp Ile
    50                  55                  60

Lys Met Met Leu Ser Ser Gly Asp Gln Asp Ser Ser Ala Arg Val Ile
65                  70                  75                  80

Asp Thr Ile Ser Glu Ile Ala Val Lys Leu Tyr Ser Met Pro Lys Met
                85                  90                  95

Thr Thr Ala Ala Val His Gly Ala Ala Ala Gly Leu Gly Leu Ser Leu
            100                 105                 110

Ala Leu Ser Cys Asp His Val Leu Val Glu Lys Glu Ala Lys Leu Ala
        115                 120                 125

Met Asn Phe Ile Gly Ile Gly Leu Val Pro Asp Gly Gly Gly His Phe
    130                 135                 140

Phe Leu Glu Arg Arg Ile Gly Glu Thr Ala Ala Lys Glu Leu Ile Trp
145                 150                 155                 160

Ser Gly Lys Lys Leu Thr Gly Ala Glu Ala His Glu Leu Arg Ile Ala
                165                 170                 175

Asp Ala Val Phe Ser Gly Asp Ser Gly Arg Phe Ala Arg Ile Tyr Leu
            180                 185                 190

Glu Lys Leu Leu His Ala Pro Leu Ala Ala Met Ile Glu Thr Lys Lys
        195                 200                 205

Ile Tyr Gln Ala Leu Asn Gly Gly Arg Leu Gln Lys Thr Leu Glu Leu
    210                 215                 220

Glu Lys Thr Ala Gln Met Lys Met Arg Leu Thr Ser Asp His Gln Glu
225                 230                 235                 240

Gly Ile Arg Ala Phe Leu Glu Lys Arg Gln Pro Gln Phe Asn Arg Gln
                245                 250                 255

Gln Val

<210>  17
<211>  1065
<212>  DNA
<213>  Bacillus licheniformis DSM 13

<220>
<221>  gene
<222>  (1)..(1065)
<223>  Probable Enoyl(3-hydroxyisobutyryl)-coenzyme A hydrolase protein

<220>
<221>  CDS
<222>  (1)..(1062)
<223>

<400>  17

atg tcc gat gac gtg ctg ttt tcc gtc aat caa aac ggc gcc gca gcg          48
Met Ser Asp Asp Val Leu Phe Ser Val Asn Gln Asn Gly Ala Ala Ala
1               5                   10                  15

att gtt ctg aat cgc ccg aaa gcg ctc aac tca ctc aca tac gac atg          96
Ile Val Leu Asn Arg Pro Lys Ala Leu Asn Ser Leu Thr Tyr Asp Met
            20                  25                  30

gtc cgt ctg att ggt gaa aag tta aac gag tgg gag aca gat caa aac         144
Val Arg Leu Ile Gly Glu Lys Leu Asn Glu Trp Glu Thr Asp Gln Asn
        35                  40                  45

gtt tct atc gtg gtc atc aaa ggt gca gga cca aaa gga cta tgt gcc         192
Val Ser Ile Val Val Ile Lys Gly Ala Gly Pro Lys Gly Leu Cys Ala
    50                  55                  60

gga ggg gat att aag gca ctc tat gaa gct cgt tcg tca aaa cag gcc         240
Gly Gly Asp Ile Lys Ala Leu Tyr Glu Ala Arg Ser Ser Lys Gln Ala
65                  70                  75                  80

ctg caa gat gcc gag cgc ttt ttt gaa aca gag tac gaa gtc gat atg         288
Leu Gln Asp Ala Glu Arg Phe Phe Glu Thr Glu Tyr Glu Val Asp Met
                85                  90                  95

gca gtc cat cga ttt tcg aaa ccg atc atc gcc tgc ttg gac ggg atc         336
Ala Val His Arg Phe Ser Lys Pro Ile Ile Ala Cys Leu Asp Gly Ile
            100                 105                 110

gtc atg ggg gga ggc gtc ggc ctg acg tac ggg gcc agc cac cgg atc         384
Val Met Gly Gly Gly Val Gly Leu Thr Tyr Gly Ala Ser His Arg Ile
        115                 120                 125

gtc acg gag agg aca aaa tgg gcg atg ccc gaa atg aat atc ggc ttc         432
Val Thr Glu Arg Thr Lys Trp Ala Met Pro Glu Met Asn Ile Gly Phe
    130                 135                 140

ttt ccg gat gtc ggg gca gcc tat ttt tta aac aaa gcc ccg ggc cgc         480
Phe Pro Asp Val Gly Ala Ala Tyr Phe Leu Asn Lys Ala Pro Gly Arg
145                 150                 155                 160

tta ggg cgg tat ctt gga tta acg gcg tct gtc atc cat gca gcc gac         528
Leu Gly Arg Tyr Leu Gly Leu Thr Ala Ser Val Ile His Ala Ala Asp
                165                 170                 175

gtg ctg tat atc aat ggg gca gac gcc tac atg gag agc ggc gct tta         576
Val Leu Tyr Ile Asn Gly Ala Asp Ala Tyr Met Glu Ser Gly Ala Leu
            180                 185                 190

gaa cga ttg ctt caa gca gtg gaa caa acc gat tgg cgc ctt gca agc         624
Glu Arg Leu Leu Gln Ala Val Glu Gln Thr Asp Trp Arg Leu Ala Ser
        195                 200                 205

gtt gaa gaa aag ctc gat cag ctg atc cgc gaa tcg aaa acg gag ccc         672
Val Glu Glu Lys Leu Asp Gln Leu Ile Arg Glu Ser Lys Thr Glu Pro
    210                 215                 220

tcc cag gag agc acg ctc gcc cgt gat cag caa gcg att gac cgt cat         720
Ser Gln Glu Ser Thr Leu Ala Arg Asp Gln Gln Ala Ile Asp Arg His
225                 230                 235                 240

ttt aag tat gat aag ctg gaa gag atc ctt caa tcg ctc gaa agc gag      768
Phe Lys Tyr Asp Lys Leu Glu Glu Ile Leu Gln Ser Leu Glu Ser Glu
                245                 250                 255

gga agc acc ttt agc tcg aat gtg aaa aaa aca atg ctt tcc aaa tcg      816
Gly Ser Thr Phe Ser Ser Asn Val Lys Lys Thr Met Leu Ser Lys Ser
            260                 265                 270

cca ttt tca tta aaa atc aca ttg aaa cag ctg gcg gac gga cgt caa      864
Pro Phe Ser Leu Lys Ile Thr Leu Lys Gln Leu Ala Asp Gly Arg Gln
        275                 280                 285

aaa aca ctg gaa gaa tgc ttt gcc acg gat ctg gtg ctg gca aag aac      912
Lys Thr Leu Glu Glu Cys Phe Ala Thr Asp Leu Val Leu Ala Lys Asn
    290                 295                 300

ttt ttg aag cac aat gat ttc ttc gaa ggc gtc agg tcc gtc ctg atc      960
Phe Leu Lys His Asn Asp Phe Phe Glu Gly Val Arg Ser Val Leu Ile
305                 310                 315                 320

gac cgt gat caa tca ccg aac tac aag tac cgg aac gtt tca gat gta     1008
Asp Arg Asp Gln Ser Pro Asn Tyr Lys Tyr Arg Asn Val Ser Asp Val
                325                 330                 335

acc gat gaa gcg gtg gac cgg ttt ttc caa ccc tct gaa tct gtc cgg     1056
Thr Asp Glu Ala Val Asp Arg Phe Phe Gln Pro Ser Glu Ser Val Arg
            340                 345                 350

ttt taa aag                                                         1065
Phe

<210>  18
<211>  353
<212>  PRT
<213>  Bacillus licheniformis DSM 13

<400>  18

Met Ser Asp Asp Val Leu Phe Ser Val Asn Gln Asn Gly Ala Ala Ala
1               5                   10                  15

Ile Val Leu Asn Arg Pro Lys Ala Leu Asn Ser Leu Thr Tyr Asp Met
            20                  25                  30

Val Arg Leu Ile Gly Glu Lys Leu Asn Glu Trp Glu Thr Asp Gln Asn
        35                  40                  45

Val Ser Ile Val Val Ile Lys Gly Ala Gly Pro Lys Gly Leu Cys Ala
    50                  55                  60

Gly Gly Asp Ile Lys Ala Leu Tyr Glu Ala Arg Ser Ser Lys Gln Ala
65                  70                  75                  80

Leu Gln Asp Ala Glu Arg Phe Phe Glu Thr Glu Tyr Glu Val Asp Met
                85                  90                  95

Ala Val His Arg Phe Ser Lys Pro Ile Ile Ala Cys Leu Asp Gly Ile
            100                 105                 110

Val Met Gly Gly Gly Val Gly Leu Thr Tyr Gly Ala Ser His Arg Ile
        115                 120                 125

Val Thr Glu Arg Thr Lys Trp Ala Met Pro Glu Met Asn Ile Gly Phe
    130                 135                 140

Phe Pro Asp Val Gly Ala Ala Tyr Phe Leu Asn Lys Ala Pro Gly Arg
145                 150                 155                 160

Leu Gly Arg Tyr Leu Gly Leu Thr Ala Ser Val Ile His Ala Ala Asp
                165                 170                 175

Val Leu Tyr Ile Asn Gly Ala Asp Ala Tyr Met Glu Ser Gly Ala Leu
            180                 185                 190

Glu Arg Leu Leu Gln Ala Val Glu Gln Thr Asp Trp Arg Leu Ala Ser
        195                 200                 205

Val Glu Glu Lys Leu Asp Gln Leu Ile Arg Glu Ser Lys Thr Glu Pro
    210                 215                 220

Ser Gln Glu Ser Thr Leu Ala Arg Asp Gln Gln Ala Ile Asp Arg His
225                 230                 235                 240

Phe Lys Tyr Asp Lys Leu Glu Glu Ile Leu Gln Ser Leu Glu Ser Glu
                245                 250                 255

Gly Ser Thr Phe Ser Ser Asn Val Lys Lys Thr Met Leu Ser Lys Ser
            260                 265                 270

Pro Phe Ser Leu Lys Ile Thr Leu Lys Gln Leu Ala Asp Gly Arg Gln
        275                 280                 285

Lys Thr Leu Glu Glu Cys Phe Ala Thr Asp Leu Val Leu Ala Lys Asn
    290                 295                 300

Phe Leu Lys His Asn Asp Phe Phe Glu Gly Val Arg Ser Val Leu Ile

305                 310                 315                 320

Asp Arg Asp Gln Ser Pro Asn Tyr Lys Tyr Arg Asn Val Ser Asp Val
                325                 330                 335

Thr Asp Glu Ala Val Asp Arg Phe Phe Gln Pro Ser Glu Ser Val Arg
            340                 345                 350

Phe

<210>  19
<211>  789
<212>  DNA
<213>  Bacillus licheniformis DSM 13

<220>
<221>  gene
<222>  (1)..(789)
<223>  Probable enoyl-CoA hydratase (E.C. 4.2.1.17), echA8

<220>
<221>  misc_feature
<222>  (1)..(3)
<223>  First codon translated as Met.

<220>
<221>  CDS
<222>  (1)..(786)
<223>

<400>  19
ttg aac ttg aaa tat gcc aca ctt aaa aca gaa cat ggc atc aca acc       48
Leu Asn Leu Lys Tyr Ala Thr Leu Lys Thr Glu His Gly Ile Thr Thr
1               5                   10                  15

gtg acg ttg aac aat ccg ccg gca aat aca ctt tct tct tcc tgt atc       96
Val Thr Leu Asn Asn Pro Pro Ala Asn Thr Leu Ser Ser Ser Cys Ile
            20                  25                  30

gcc gaa ttg cgc tct ctt ttt cgg gaa ttg gcc cgc gac gag gag aca      144
Ala Glu Leu Arg Ser Leu Phe Arg Glu Leu Ala Arg Asp Glu Glu Thr
        35                  40                  45

aaa gca atc atc att act gga gaa ggc cgc ttt ttt gtg gcg gga gcg      192
Lys Ala Ile Ile Ile Thr Gly Glu Gly Arg Phe Phe Val Ala Gly Ala
    50                  55                  60

gat ata aaa gaa ttc gtt tca aaa ctt ggg gac caa aaa caa gga ttg      240
Asp Ile Lys Glu Phe Val Ser Lys Leu Gly Asp Gln Lys Gln Gly Leu
65                  70                  75                  80

gcg ctc gca caa ggg ggc cag gcg ctc tgc gat gaa atc gaa gct tcc        288
Ala Leu Ala Gln Gly Gly Gln Ala Leu Cys Asp Glu Ile Glu Ala Ser
                85                  90                  95

aaa aaa ccc gtc att gcg gcg ata aac gga ccg gct ctt ggc gga ggc        336
Lys Lys Pro Val Ile Ala Ala Ile Asn Gly Pro Ala Leu Gly Gly Gly
            100                 105                 110

ctg gaa ctc gcg atg agc tgc cac ttc aga atc gta tca gac gat gca        384
Leu Glu Leu Ala Met Ser Cys His Phe Arg Ile Val Ser Asp Asp Ala
        115                 120                 125

aca gtc ggt ctt ccc gaa tta aag ctc ggc ttg att cct gca ttt ggt        432
Thr Val Gly Leu Pro Glu Leu Lys Leu Gly Leu Ile Pro Ala Phe Gly
    130                 135                 140

ggt aca cag cgg ctt cgc aac ata acg gac aca gcg aca gca ctc gac        480
Gly Thr Gln Arg Leu Arg Asn Ile Thr Asp Thr Ala Thr Ala Leu Asp
145                 150                 155                 160

ctt atc ctg acg ggc cga tcg ctt tca gct caa gag gcg gta gag ctg        528
Leu Ile Leu Thr Gly Arg Ser Leu Ser Ala Gln Glu Ala Val Glu Leu
                165                 170                 175

aaa att gca cag atg gct gta aag gga gag gaa ctg atg aag acg gca        576
Lys Ile Ala Gln Met Ala Val Lys Gly Glu Glu Leu Met Lys Thr Ala
            180                 185                 190

gct gct gtc gcg tcg tct ttt atc gaa gga aaa agc atg acc agc gtg        624
Ala Ala Val Ala Ser Ser Phe Ile Glu Gly Lys Ser Met Thr Ser Val
        195                 200                 205

agg cgc gcc gtc gaa tgt gtc gta cag ggc gcc agc gaa agc atg gaa        672
Arg Arg Ala Val Glu Cys Val Val Gln Gly Ala Ser Glu Ser Met Glu
    210                 215                 220

caa gca ctg gag agg gag cga aac aga ttc gcc gag ctg ttt gtc act        720
Gln Ala Leu Glu Arg Glu Arg Asn Arg Phe Ala Glu Leu Phe Val Thr
225                 230                 235                 240

tca gat gcc aaa gaa gga att cac gca ttt gtc gaa aag cgc aaa cca        768
Ser Asp Ala Lys Glu Gly Ile His Ala Phe Val Glu Lys Arg Lys Pro
                245                 250                 255

aac ttt cat cat tca taa aag                                            789
Asn Phe His His Ser
            260


<210>  20
<211>  261
<212>  PRT
<213>  Bacillus licheniformis DSM 13

<220>
<221>  misc_feature
<222>  (1)..(3)
<223>  First codon translated as Met.

<400> 20

Leu Asn Leu Lys Tyr Ala Thr Leu Lys Thr Glu His Gly Ile Thr Thr
1               5                   10                  15

Val Thr Leu Asn Asn Pro Pro Ala Asn Thr Leu Ser Ser Ser Cys Ile
            20                  25                  30

Ala Glu Leu Arg Ser Leu Phe Arg Glu Leu Ala Arg Asp Glu Glu Thr
        35                  40                  45

Lys Ala Ile Ile Ile Thr Gly Glu Gly Arg Phe Phe Val Ala Gly Ala
    50                  55                  60

Asp Ile Lys Glu Phe Val Ser Lys Leu Gly Asp Gln Lys Gln Gly Leu
65                  70                  75                  80

Ala Leu Ala Gln Gly Gly Gln Ala Leu Cys Asp Glu Ile Glu Ala Ser
                85                  90                  95

Lys Lys Pro Val Ile Ala Ala Ile Asn Gly Pro Ala Leu Gly Gly Gly
            100                 105                 110

Leu Glu Leu Ala Met Ser Cys His Phe Arg Ile Val Ser Asp Asp Ala
        115                 120                 125

Thr Val Gly Leu Pro Glu Leu Lys Leu Gly Leu Ile Pro Ala Phe Gly
    130                 135                 140

Gly Thr Gln Arg Leu Arg Asn Ile Thr Asp Thr Ala Thr Ala Leu Asp
145                 150                 155                 160

Leu Ile Leu Thr Gly Arg Ser Leu Ser Ala Gln Glu Ala Val Glu Leu
                165                 170                 175

Lys Ile Ala Gln Met Ala Val Lys Gly Glu Glu Leu Met Lys Thr Ala
            180                 185                 190

Ala Ala Val Ala Ser Ser Phe Ile Glu Gly Lys Ser Met Thr Ser Val
        195                 200                 205

Arg Arg Ala Val Glu Cys Val Val Gln Gly Ala Ser Glu Ser Met Glu
    210                 215                 220

Gln Ala Leu Glu Arg Glu Arg Asn Arg Phe Ala Glu Leu Phe Val Thr

225                 230                 235                 240

Ser Asp Ala Lys Glu Gly Ile His Ala Phe Val Glu Lys Arg Lys Pro
                245                 250                 255

Asn Phe His His Ser
            260

<210>  21
<211>  1719
<212>  DNA
<213>  Bacillus licheniformis DSM 13

<220>
<221>  gene
<222>  (1)..(1719)
<223>  Acyl-CoA dehydrogenase (E.C. 1.3.99.-)

<220>
<221>  CDS
<222>  (1)..(1716)
<223>

<400>  21
atg ggg aaa gca aaa ctt cga tgg aat gaa ccc ttg att tct caa cac       48
Met Gly Lys Ala Lys Leu Arg Trp Asn Glu Pro Leu Ile Ser Gln His
1               5                   10                  15

gaa tcg gct gct gaa gga ttt aca ccg gaa gat ttc aca gag gaa gat       96
Glu Ser Ala Ala Glu Gly Phe Thr Pro Glu Asp Phe Thr Glu Glu Asp
            20                  25                  30

cga ctg att tca aaa acg aca gaa tca ttt gtc aaa aac gaa gtc atg      144
Arg Leu Ile Ser Lys Thr Thr Glu Ser Phe Val Lys Asn Glu Val Met
        35                  40                  45

ccc ctt ctt gaa tcg att gat cag cag gat cac gaa agc gtg aaa aaa      192
Pro Leu Leu Glu Ser Ile Asp Gln Gln Asp His Glu Ser Val Lys Lys
    50                  55                  60

ttg ttt caa aaa gca gga gag ctc ggt ttg ctc agt atc gaa gtt ccg      240
Leu Phe Gln Lys Ala Gly Glu Leu Gly Leu Leu Ser Ile Glu Val Pro
65                  70                  75                  80

gag gat tgc ggc ggc ctt tca ctc agc aag aag ctt tcc ggg ttg gtg      288
Glu Asp Cys Gly Gly Leu Ser Leu Ser Lys Lys Leu Ser Gly Leu Val
                85                  90                  95

gca gag aaa atg gga gcc ggc gga tcg ttc agc gtc tcc ttt aat att      336
Ala Glu Lys Met Gly Ala Gly Gly Ser Phe Ser Val Ser Phe Asn Ile
            100                 105                 110

cat gcg gga gtc ggg aca ctg ccg tat att tat tat gga aca gag gaa      384
His Ala Gly Val Gly Thr Leu Pro Tyr Ile Tyr Tyr Gly Thr Glu Glu

115                 120                 125

caa aaa caa aaa tac ctt cca aaa ctg gca tcg ggc gaa tgg atc gga      432
Gln Lys Gln Lys Tyr Leu Pro Lys Leu Ala Ser Gly Glu Trp Ile Gly
    130                 135                 140

gca tat gct ctg aca gag ccg ggc gca gga tcg gat gct tta aac gca      480
Ala Tyr Ala Leu Thr Glu Pro Gly Ala Gly Ser Asp Ala Leu Asn Ala
145                 150                 155                 160

aaa acg aca gcc gtc ttg aat agg gaa ggg aca gcc tgg att tta aat      528
Lys Thr Thr Ala Val Leu Asn Arg Glu Gly Thr Ala Trp Ile Leu Asn
                165                 170                 175

ggg gaa aag cag tgg att acg aac gcg caa gta gct gat gta tat gtt      576
Gly Glu Lys Gln Trp Ile Thr Asn Ala Gln Val Ala Asp Val Tyr Val
            180                 185                 190

gtc ttt gca aaa acg gcg gaa ggc atg aca gca ttt atc gtc gaa cgc      624
Val Phe Ala Lys Thr Ala Glu Gly Met Thr Ala Phe Ile Val Glu Arg
        195                 200                 205

tcg ttt aaa ggt gtt tcc atc gga cct gaa gag aag aag atg gga atc      672
Ser Phe Lys Gly Val Ser Ile Gly Pro Glu Glu Lys Lys Met Gly Ile
    210                 215                 220

aaa ggg tct tcg aca gca acc tta atc ttg gag gaa gtc gag gtg cca      720
Lys Gly Ser Ser Thr Ala Thr Leu Ile Leu Glu Glu Val Glu Val Pro
225                 230                 235                 240

agc gac aat gtt cta ggt cat atc ggg aaa ggt cat cac gtc gct ttg      768
Ser Asp Asn Val Leu Gly His Ile Gly Lys Gly His His Val Ala Leu
                245                 250                 255

aac att tta aac atg gcc cgc tta aag ctc gcg ttt tcg aac att gga      816
Asn Ile Leu Asn Met Ala Arg Leu Lys Leu Ala Phe Ser Asn Ile Gly
            260                 265                 270

acg gca aaa caa gca ttg aac ctt gct gtt agc tac gcc aaa cag cga      864
Thr Ala Lys Gln Ala Leu Asn Leu Ala Val Ser Tyr Ala Lys Gln Arg
        275                 280                 285

aag caa ttt aac aag ccg atc atc ggt ttt tca atg att caa gaa aag      912
Lys Gln Phe Asn Lys Pro Ile Ile Gly Phe Ser Met Ile Gln Glu Lys
    290                 295                 300

att gcc gac atg gcg gtc tcg att ttc ggc gcg gaa agc gct gct tac      960
Ile Ala Asp Met Ala Val Ser Ile Phe Gly Ala Glu Ser Ala Ala Tyr
305                 310                 315                 320

aga acg gca gat tgc ttg gac aat gtt tta gat tcg gct ctc cca tta     1008
Arg Thr Ala Asp Cys Leu Asp Asn Val Leu Asp Ser Ala Leu Pro Leu
                325                 330                 335

gac gat aga ctg aga aaa ctc aca aac tat gca tcc gaa tgt gcg atc     1056
Asp Asp Arg Leu Arg Lys Leu Thr Asn Tyr Ala Ser Glu Cys Ala Ile
            340                 345                 350

aat aaa gtt tac tgc tcc gaa atc ctc ggc cgg atc gca gac gaa gcg     1104

Asn Lys Val Tyr Cys Ser Glu Ile Leu Gly Arg Ile Ala Asp Glu Ala
        355                 360                 365

gtg cag att cat ggc ggc tac gga tac atg cag gag tac gaa gtc gaa      1152
Val Gln Ile His Gly Gly Tyr Gly Tyr Met Gln Glu Tyr Glu Val Glu
    370                 375                 380

cga ttg tac cgg gac gcg agg atc agc cgg att ttc gag ggg aca aat      1200
Arg Leu Tyr Arg Asp Ala Arg Ile Ser Arg Ile Phe Glu Gly Thr Asn
385                 390                 395                 400

gaa ata aac cgc tta acg atc gcc aaa ctg ctc atg aaa gaa gtg cag      1248
Glu Ile Asn Arg Leu Thr Ile Ala Lys Leu Leu Met Lys Glu Val Gln
                405                 410                 415

caa aac ggc atc tca gag ccg gaa gct caa cta ggc agc gag gga aat      1296
Gln Asn Gly Ile Ser Glu Pro Glu Ala Gln Leu Gly Ser Glu Gly Asn
            420                 425                 430

cga aac cgg cga ttt att cag ctg tcc aac agg ctt ttc ggc aag aca      1344
Arg Asn Arg Arg Phe Ile Gln Leu Ser Asn Arg Leu Phe Gly Lys Thr
        435                 440                 445

ctg aaa gcg ctt atc cgc tct cgc gtg aac act caa gaa gat cag gaa      1392
Leu Lys Ala Leu Ile Arg Ser Arg Val Asn Thr Gln Glu Asp Gln Glu
    450                 455                 460

tac gca cgg ctt ctc gcc gac atg aaa aaa gaa atc tat gtg atg gaa      1440
Tyr Ala Arg Leu Leu Ala Asp Met Lys Lys Glu Ile Tyr Val Met Glu
465                 470                 475                 480

tcc gcc gcc cgc cga acc gaa aaa gct agg caa ata tac ggt ggt gaa      1488
Ser Ala Ala Arg Arg Thr Glu Lys Ala Arg Gln Ile Tyr Gly Gly Glu
                485                 490                 495

aaa gca cgg ttg aaa gaa atg atg acg aat gtc att tgt gaa gaa ggc      1536
Lys Ala Arg Leu Lys Glu Met Met Thr Asn Val Ile Cys Glu Glu Gly
            500                 505                 510

tac cgc aga atc gaa gag atg gcg gtg acc gcc tta tca agc ata gca      1584
Tyr Arg Arg Ile Glu Glu Met Ala Val Thr Ala Leu Ser Ser Ile Ala
        515                 520                 525

tct gat gaa gcc gaa aga aaa ctc gca ttt gaa gag gct cgc agc gtt      1632
Ser Asp Glu Ala Glu Arg Lys Leu Ala Phe Glu Glu Ala Arg Ser Val
    530                 535                 540

tct ttg cct ctt ttc agc aat ctg ttt act caa aaa cgc gaa atc gca      1680
Ser Leu Pro Leu Phe Ser Asn Leu Phe Thr Gln Lys Arg Glu Ile Ala
545                 550                 555                 560

gaa aaa ata gcc gct cat gaa aaa tat acg gtg tga tcg                  1719
Glu Lys Ile Ala Ala His Glu Lys Tyr Thr Val
                565                 570

<210>  22
<211>  571
<212>  PRT

<213>  Bacillus licheniformis DSM 13

<400>  22

Met Gly Lys Ala Lys Leu Arg Trp Asn Glu Pro Leu Ile Ser Gln His
1               5                   10                  15

Glu Ser Ala Ala Glu Gly Phe Thr Pro Glu Asp Phe Thr Glu Glu Asp
            20                  25                  30

Arg Leu Ile Ser Lys Thr Thr Glu Ser Phe Val Lys Asn Glu Val Met
        35                  40                  45

Pro Leu Leu Glu Ser Ile Asp Gln Gln Asp His Glu Ser Val Lys Lys
    50                  55                  60

Leu Phe Gln Lys Ala Gly Glu Leu Gly Leu Leu Ser Ile Glu Val Pro
65                  70                  75                  80

Glu Asp Cys Gly Gly Leu Ser Leu Ser Lys Lys Leu Ser Gly Leu Val
                85                  90                  95

Ala Glu Lys Met Gly Ala Gly Gly Ser Phe Ser Val Ser Phe Asn Ile
            100                 105                 110

His Ala Gly Val Gly Thr Leu Pro Tyr Ile Tyr Tyr Gly Thr Glu Glu
        115                 120                 125

Gln Lys Gln Lys Tyr Leu Pro Lys Leu Ala Ser Gly Glu Trp Ile Gly
    130                 135                 140

Ala Tyr Ala Leu Thr Glu Pro Gly Ala Gly Ser Asp Ala Leu Asn Ala
145                 150                 155                 160

Lys Thr Thr Ala Val Leu Asn Arg Glu Gly Thr Ala Trp Ile Leu Asn
                165                 170                 175

Gly Glu Lys Gln Trp Ile Thr Asn Ala Gln Val Ala Asp Val Tyr Val
            180                 185                 190

Val Phe Ala Lys Thr Ala Glu Gly Met Thr Ala Phe Ile Val Glu Arg
        195                 200                 205

Ser Phe Lys Gly Val Ser Ile Gly Pro Glu Glu Lys Lys Met Gly Ile
    210                 215                 220

Lys Gly Ser Ser Thr Ala Thr Leu Ile Leu Glu Glu Val Glu Val Pro
225                 230                 235                 240

Ser Asp Asn Val Leu Gly His Ile Gly Lys Gly His His Val Ala Leu
                245                 250                 255

Asn Ile Leu Asn Met Ala Arg Leu Lys Leu Ala Phe Ser Asn Ile Gly
            260                 265                 270

Thr Ala Lys Gln Ala Leu Asn Leu Ala Val Ser Tyr Ala Lys Gln Arg
        275                 280                 285

Lys Gln Phe Asn Lys Pro Ile Ile Gly Phe Ser Met Ile Gln Glu Lys
    290                 295                 300

Ile Ala Asp Met Ala Val Ser Ile Phe Gly Ala Glu Ser Ala Ala Tyr
305                 310                 315                 320

Arg Thr Ala Asp Cys Leu Asp Asn Val Leu Asp Ser Ala Leu Pro Leu
                325                 330                 335

Asp Asp Arg Leu Arg Lys Leu Thr Asn Tyr Ala Ser Glu Cys Ala Ile
            340                 345                 350

Asn Lys Val Tyr Cys Ser Glu Ile Leu Gly Arg Ile Ala Asp Glu Ala
        355                 360                 365

Val Gln Ile His Gly Gly Tyr Gly Tyr Met Gln Glu Tyr Glu Val Glu
    370                 375                 380

Arg Leu Tyr Arg Asp Ala Arg Ile Ser Arg Ile Phe Glu Gly Thr Asn
385                 390                 395                 400

Glu Ile Asn Arg Leu Thr Ile Ala Lys Leu Leu Met Lys Glu Val Gln
                405                 410                 415

Gln Asn Gly Ile Ser Glu Pro Glu Ala Gln Leu Gly Ser Glu Gly Asn
            420                 425                 430

Arg Asn Arg Arg Phe Ile Gln Leu Ser Asn Arg Leu Phe Gly Lys Thr
        435                 440                 445

Leu Lys Ala Leu Ile Arg Ser Arg Val Asn Thr Gln Glu Asp Gln Glu
    450                 455                 460

Tyr Ala Arg Leu Leu Ala Asp Met Lys Lys Glu Ile Tyr Val Met Glu
465                 470                 475                 480

Ser Ala Ala Arg Arg Thr Glu Lys Ala Arg Gln Ile Tyr Gly Gly Glu
                485                 490                 495

Lys Ala Arg Leu Lys Glu Met Met Thr Asn Val Ile Cys Glu Glu Gly
            500                 505                 510

Tyr Arg Arg Ile Glu Glu Met Ala Val Thr Ala Leu Ser Ser Ile Ala
        515                 520                 525

Ser Asp Glu Ala Glu Arg Lys Leu Ala Phe Glu Glu Ala Arg Ser Val
    530                 535                 540

Ser Leu Pro Leu Phe Ser Asn Leu Phe Thr Gln Lys Arg Glu Ile Ala
545                 550                 555                 560

Glu Lys Ile Ala Ala His Glu Lys Tyr Thr Val
                565                 570

<210>  23
<211>  1956
<212>  DNA
<213>  Bacillus licheniformis DSM 13

<220>
<221>  gene
<222>  (1)..(1956)
<223>  Acetyl-coenzyme A synthetase (E.C. 6.2.1.1), acsA

<220>
<221>  CDS
<222>  (1)..(1953)
<223>

<400>  23
atg ggc gaa aaa gcg gtt tgg cag cct gat ccg gaa ttt gta aaa aca       48
Met Gly Glu Lys Ala Val Trp Gln Pro Asp Pro Glu Phe Val Lys Thr
1               5                   10                  15

acc cgg ctg ttt caa tgg atg aca gcc ctc ggt ttt tcc gac tat gat       96
Thr Arg Leu Phe Gln Trp Met Thr Ala Leu Gly Phe Ser Asp Tyr Asp
            20                  25                  30

gat ttt ttg aaa gca agt aca aac gat atc gcc tgg ttc tgg gaa gag      144
Asp Phe Leu Lys Ala Ser Thr Asn Asp Ile Ala Trp Phe Trp Glu Glu
        35                  40                  45

gcc gaa aaa gcg ctc ggg atc agc tgg tac aag cgg tac agc caa aca     192
Ala Glu Lys Ala Leu Gly Ile Ser Trp Tyr Lys Arg Tyr Ser Gln Thr
    50                  55                  60

ttg aat ctc gac aaa ggc ata aaa tgg ccg caa tgg ttt acc ggc ggc     240
Leu Asn Leu Asp Lys Gly Ile Lys Trp Pro Gln Trp Phe Thr Gly Gly
65                  70                  75                  80

cgc tta aat gcc gtt tac aac gcc gtg gaa aaa tgg gcc cgc cgg cct     288
Arg Leu Asn Ala Val Tyr Asn Ala Val Glu Lys Trp Ala Arg Arg Pro
                85                  90                  95

gat acg gcc ggc agg acg gca ctc atc tgg gaa agt gaa gac gga aaa     336
Asp Thr Ala Gly Arg Thr Ala Leu Ile Trp Glu Ser Glu Asp Gly Lys
            100                 105                 110

aca gaa cag atc acc tat tct tct tta cac caa caa gtc gcc cgt gcg     384
Thr Glu Gln Ile Thr Tyr Ser Ser Leu His Gln Gln Val Ala Arg Ala
        115                 120                 125

gcg gca ggc ttt aaa aag caa ggc atc tca aaa ggg gat gtc att gcg     432
Ala Ala Gly Phe Lys Lys Gln Gly Ile Ser Lys Gly Asp Val Ile Ala
    130                 135                 140

att tac atg ccg atg atc ccc gaa acg gtc atc gcc atg ctg gcc gcc     480
Ile Tyr Met Pro Met Ile Pro Glu Thr Val Ile Ala Met Leu Ala Ala
145                 150                 155                 160

gct aaa atc gga gcg gta ttc tca ccg gtt ttt tca ggc tac ggc gcc     528
Ala Lys Ile Gly Ala Val Phe Ser Pro Val Phe Ser Gly Tyr Gly Ala
                165                 170                 175

cat gca gca gcg gcg aga ctt acc gct gcc gga gcg aaa atc ctt gtc     576
His Ala Ala Ala Ala Arg Leu Thr Ala Ala Gly Ala Lys Ile Leu Val
            180                 185                 190

aca gca gat gcc ttt ttg cga agg gga aag aag gtc tgc atg aag aaa     624
Thr Ala Asp Ala Phe Leu Arg Arg Gly Lys Lys Val Cys Met Lys Lys
        195                 200                 205

gaa gct gac aaa gcc gcg gac cgt tcc ccc act gtt caa aaa gtc gtc     672
Glu Ala Asp Lys Ala Ala Asp Arg Ser Pro Thr Val Gln Lys Val Val
    210                 215                 220

gtc tgc aag ctt cac ggc ggc gat caa gat tgg aat tat aag aga gat     720
Val Cys Lys Leu His Gly Gly Asp Gln Asp Trp Asn Tyr Lys Arg Asp
225                 230                 235                 240

atc gac tgg aat gaa ttg atg aaa aac gag ccc atg caa aac acc gaa     768
Ile Asp Trp Asn Glu Leu Met Lys Asn Glu Pro Met Gln Asn Thr Glu
                245                 250                 255

gaa atg gac agt tca gat ccg ctc atg ctg cta tac aca tca ggg acg     816
Glu Met Asp Ser Ser Asp Pro Leu Met Leu Leu Tyr Thr Ser Gly Thr
            260                 265                 270

aca gga cag tcg aag gga gcg gtt cat acc cat gcc ggt ttt ccg ctg     864
Thr Gly Gln Ser Lys Gly Ala Val His Thr His Ala Gly Phe Pro Leu

275                 280                 285

aaa gct gca ttt gat gcg gga ttc ggg atg gat gtc aaa caa ggg gac      912
Lys Ala Ala Phe Asp Ala Gly Phe Gly Met Asp Val Lys Gln Gly Asp
    290                 295                 300

aca ttt ttc tgg ttt aca gac atg ggc tgg atg atg ggg ccg ttt tta      960
Thr Phe Phe Trp Phe Thr Asp Met Gly Trp Met Met Gly Pro Phe Leu
305                 310                 315                 320

ata ttc ggg ggc ctc ata aac gga gcg gct gtt ttg ctg ttt gac gga     1008
Ile Phe Gly Gly Leu Ile Asn Gly Ala Ala Val Leu Leu Phe Asp Gly
                325                 330                 335

gca ccg gac tac ccg gcc ccg gat cgg ctg tgg gag ctt gtc agc aga     1056
Ala Pro Asp Tyr Pro Ala Pro Asp Arg Leu Trp Glu Leu Val Ser Arg
            340                 345                 350

cac cgg gtg acg cat ctc ggc gtc tct ccg acg ctc att cgc tcg ctg     1104
His Arg Val Thr His Leu Gly Val Ser Pro Thr Leu Ile Arg Ser Leu
        355                 360                 365

atg cag cac ggc gaa gat ttt ctc tat caa tac aat ctg aac agt ctg     1152
Met Gln His Gly Glu Asp Phe Leu Tyr Gln Tyr Asn Leu Asn Ser Leu
    370                 375                 380

aag gca atc ggc tca acg ggc gaa cca tgg aat tat gag ccg tgg atg     1200
Lys Ala Ile Gly Ser Thr Gly Glu Pro Trp Asn Tyr Glu Pro Trp Met
385                 390                 395                 400

tgg ctg ttc cgc cat gtt gga aaa gaa cgg att cct ata ttt aat tat     1248
Trp Leu Phe Arg His Val Gly Lys Glu Arg Ile Pro Ile Phe Asn Tyr
                405                 410                 415

tca gga gga aca gag atc tca ggc gga att tta ggc aat gtg ctc ctg     1296
Ser Gly Gly Thr Glu Ile Ser Gly Gly Ile Leu Gly Asn Val Leu Leu
            420                 425                 430

cgg ccg atc acg ccg atg acg ttt aat tcg cct ctt ccc ggc atg gcg     1344
Arg Pro Ile Thr Pro Met Thr Phe Asn Ser Pro Leu Pro Gly Met Ala
        435                 440                 445

gcc aat gtc ttc aat gaa aaa gga gag gaa gtc gtc aat gaa gtc gga     1392
Ala Asn Val Phe Asn Glu Lys Gly Glu Glu Val Val Asn Glu Val Gly
    450                 455                 460

gag ctt gtc ctg aca aag ccc tgg gtc ggc atg acg aac ggt ttt tgg     1440
Glu Leu Val Leu Thr Lys Pro Trp Val Gly Met Thr Asn Gly Phe Trp
465                 470                 475                 480

aag gag ccg tca aga tac gaa gaa gca tat tgg agc cgc tgg acc gac     1488
Lys Glu Pro Ser Arg Tyr Glu Glu Ala Tyr Trp Ser Arg Trp Thr Asp
                485                 490                 495

gtc tgg gtg cac ggc gat tgg gca aaa cgg gat gaa aac ggc tac tgg     1536
Val Trp Val His Gly Asp Trp Ala Lys Arg Asp Glu Asn Gly Tyr Trp
            500                 505                 510

acg atc agc gga cgc tct gat gat gtg atc aat gct gcc ggg aaa agg     1584

Thr Ile Ser Gly Arg Ser Asp Asp Val Ile Asn Ala Ala Gly Lys Arg
        515                 520                 525

atc ggc ccc gcc gaa ata gag tcc gtt ctg gtc ggc cat cca gcg gtg      1632
Ile Gly Pro Ala Glu Ile Glu Ser Val Leu Val Gly His Pro Ala Val
    530                 535                 540

gcg gaa gca ggc gtc atc ggc gtt ccg gat aag ctc aaa ggc cag gct      1680
Ala Glu Ala Gly Val Ile Gly Val Pro Asp Lys Leu Lys Gly Gln Ala
545                 550                 555                 560

gcc gtc tgc ttc gtc gtc ctc aga cag tcg gaa aag ccg tcg gaa gaa      1728
Ala Val Cys Phe Val Val Leu Arg Gln Ser Glu Lys Pro Ser Glu Glu
                565                 570                 575

tta aaa gat gat ttg ctg aac ctt gca tct gat gcg atc ggt aaa gcg      1776
Leu Lys Asp Asp Leu Leu Asn Leu Ala Ser Asp Ala Ile Gly Lys Ala
            580                 585                 590

gtc aag ccc aaa gcg gtt tat ttt gtc agc ggt ttg ccg aag acg aga      1824
Val Lys Pro Lys Ala Val Tyr Phe Val Ser Gly Leu Pro Lys Thr Arg
        595                 600                 605

aat gca aaa gtg atg aga cgg ctg atc aga gct gcc tat atg aac gag      1872
Asn Ala Lys Val Met Arg Arg Leu Ile Arg Ala Ala Tyr Met Asn Glu
    610                 615                 620

ccc gca ggc gat ttg tca act ttg gaa aac cgc gaa aca tat gat gaa      1920
Pro Ala Gly Asp Leu Ser Thr Leu Glu Asn Arg Glu Thr Tyr Asp Glu
625                 630                 635                 640

att gcc ggt ctt tca gtg cga aaa aat ctg tag tat                      1956
Ile Ala Gly Leu Ser Val Arg Lys Asn Leu
                645                 650


<210>  24
<211>  650
<212>  PRT
<213>  Bacillus licheniformis DSM 13

<400>  24

Met Gly Glu Lys Ala Val Trp Gln Pro Asp Pro Glu Phe Val Lys Thr
1               5                   10                  15


Thr Arg Leu Phe Gln Trp Met Thr Ala Leu Gly Phe Ser Asp Tyr Asp
            20                  25                  30


Asp Phe Leu Lys Ala Ser Thr Asn Asp Ile Ala Trp Phe Trp Glu Glu
        35                  40                  45


Ala Glu Lys Ala Leu Gly Ile Ser Trp Tyr Lys Arg Tyr Ser Gln Thr
    50                  55                  60

Leu Asn Leu Asp Lys Gly Ile Lys Trp Pro Gln Trp Phe Thr Gly Gly
65                  70                  75                  80

Arg Leu Asn Ala Val Tyr Asn Ala Val Glu Lys Trp Ala Arg Arg Pro
                85                  90                  95

Asp Thr Ala Gly Arg Thr Ala Leu Ile Trp Glu Ser Glu Asp Gly Lys
            100                 105                 110

Thr Glu Gln Ile Thr Tyr Ser Ser Leu His Gln Gln Val Ala Arg Ala
        115                 120                 125

Ala Ala Gly Phe Lys Lys Gln Gly Ile Ser Lys Gly Asp Val Ile Ala
    130                 135                 140

Ile Tyr Met Pro Met Ile Pro Glu Thr Val Ile Ala Met Leu Ala Ala
145                 150                 155                 160

Ala Lys Ile Gly Ala Val Phe Ser Pro Val Phe Ser Gly Tyr Gly Ala
                165                 170                 175

His Ala Ala Ala Ala Arg Leu Thr Ala Ala Gly Ala Lys Ile Leu Val
            180                 185                 190

Thr Ala Asp Ala Phe Leu Arg Arg Gly Lys Lys Val Cys Met Lys Lys
        195                 200                 205

Glu Ala Asp Lys Ala Ala Asp Arg Ser Pro Thr Val Gln Lys Val Val
    210                 215                 220

Val Cys Lys Leu His Gly Gly Asp Gln Asp Trp Asn Tyr Lys Arg Asp
225                 230                 235                 240

Ile Asp Trp Asn Glu Leu Met Lys Asn Glu Pro Met Gln Asn Thr Glu
                245                 250                 255

Glu Met Asp Ser Ser Asp Pro Leu Met Leu Leu Tyr Thr Ser Gly Thr
            260                 265                 270

Thr Gly Gln Ser Lys Gly Ala Val His Thr His Ala Gly Phe Pro Leu
        275                 280                 285

Lys Ala Ala Phe Asp Ala Gly Phe Gly Met Asp Val Lys Gln Gly Asp
    290                 295                 300

Thr Phe Phe Trp Phe Thr Asp Met Gly Trp Met Met Gly Pro Phe Leu
305                 310                 315                 320

Ile Phe Gly Gly Leu Ile Asn Gly Ala Ala Val Leu Leu Phe Asp Gly
                325                 330                 335

Ala Pro Asp Tyr Pro Ala Pro Asp Arg Leu Trp Glu Leu Val Ser Arg
            340                 345                 350

His Arg Val Thr His Leu Gly Val Ser Pro Thr Leu Ile Arg Ser Leu
        355                 360                 365

Met Gln His Gly Glu Asp Phe Leu Tyr Gln Tyr Asn Leu Asn Ser Leu
    370                 375                 380

Lys Ala Ile Gly Ser Thr Gly Glu Pro Trp Asn Tyr Glu Pro Trp Met
385                 390                 395                 400

Trp Leu Phe Arg His Val Gly Lys Glu Arg Ile Pro Ile Phe Asn Tyr
                405                 410                 415

Ser Gly Gly Thr Glu Ile Ser Gly Gly Ile Leu Gly Asn Val Leu Leu
            420                 425                 430

Arg Pro Ile Thr Pro Met Thr Phe Asn Ser Pro Leu Pro Gly Met Ala
        435                 440                 445

Ala Asn Val Phe Asn Glu Lys Gly Glu Glu Val Val Asn Glu Val Gly
    450                 455                 460

Glu Leu Val Leu Thr Lys Pro Trp Val Gly Met Thr Asn Gly Phe Trp
465                 470                 475                 480

Lys Glu Pro Ser Arg Tyr Glu Glu Ala Tyr Trp Ser Arg Trp Thr Asp
                485                 490                 495

Val Trp Val His Gly Asp Trp Ala Lys Arg Asp Glu Asn Gly Tyr Trp
            500                 505                 510

Thr Ile Ser Gly Arg Ser Asp Asp Val Ile Asn Ala Ala Gly Lys Arg
        515                 520                 525

Ile Gly Pro Ala Glu Ile Glu Ser Val Leu Val Gly His Pro Ala Val
    530                 535                 540

Ala Glu Ala Gly Val Ile Gly Val Pro Asp Lys Leu Lys Gly Gln Ala
545                 550                 555                 560

Ala Val Cys Phe Val Val Leu Arg Gln Ser Glu Lys Pro Ser Glu Glu
                565                 570                 575

Leu Lys Asp Asp Leu Leu Asn Leu Ala Ser Asp Ala Ile Gly Lys Ala
            580                 585                 590

Val Lys Pro Lys Ala Val Tyr Phe Val Ser Gly Leu Pro Lys Thr Arg
        595                 600                 605

Asn Ala Lys Val Met Arg Arg Leu Ile Arg Ala Ala Tyr Met Asn Glu
    610                 615                 620

Pro Ala Gly Asp Leu Ser Thr Leu Glu Asn Arg Glu Thr Tyr Asp Glu
625                 630                 635                 640

Ile Ala Gly Leu Ser Val Arg Lys Asn Leu
                645                 650

<210>  25
<211>  786
<212>  DNA
<213>  Bacillus licheniformis DSM 13

<220>
<221>  gene
<222>  (1)..(786)
<223>  3-hydroxybutyryl-CoA dehydratase (E.C. 4.2.1.55), yngF

<220>
<221>  CDS
<222>  (1)..(783)
<223>

<400>  25
atg gag ccc aat gtt ctt tat tcc ata aac gag cag tcg gtc gcc gtg       48
Met Glu Pro Asn Val Leu Tyr Ser Ile Asn Glu Gln Ser Val Ala Val
1               5                   10                  15

ttg acg ctg aac agg ccg cag gct gca aat gcc ctc tcg ctt ggg ctt       96
Leu Thr Leu Asn Arg Pro Gln Ala Ala Asn Ala Leu Ser Leu Gly Leu
            20                  25                  30

ctc gac gac ttt cag cgc atc ctt cga gat att cgt tca aac ccg gcc      144
Leu Asp Asp Phe Gln Arg Ile Leu Arg Asp Ile Arg Ser Asn Pro Ala
        35                  40                  45

gtc cgc tgt gtc atc ata acg gga aaa ggg gac agg acg ttt tgt gca     192
Val Arg Cys Val Ile Ile Thr Gly Lys Gly Asp Arg Thr Phe Cys Ala
    50                  55                  60

ggc gcc gat tta aag gaa aga gcc cgc atg agc caa aca gaa gcg aag     240
Gly Ala Asp Leu Lys Glu Arg Ala Arg Met Ser Gln Thr Glu Ala Lys
65                  70                  75                  80

cag gct gtt tcc ctg att caa cgc gtg gtc agc gaa acg gaa aaa ctg     288
Gln Ala Val Ser Leu Ile Gln Arg Val Val Ser Glu Thr Glu Lys Leu
                85                  90                  95

ccg cag ccc gtc atc gct tca tta aac gga agc gct tta gga ggg ggg     336
Pro Gln Pro Val Ile Ala Ser Leu Asn Gly Ser Ala Leu Gly Gly Gly
            100                 105                 110

ctg gag ctt gca ttg gcg tgc gac atc agg atc gca gcc gaa cat att     384
Leu Glu Leu Ala Leu Ala Cys Asp Ile Arg Ile Ala Ala Glu His Ile
        115                 120                 125

gaa ctc ggc ctc ccc gaa aca acg ctc gca atc att cca ggg gca gga     432
Glu Leu Gly Leu Pro Glu Thr Thr Leu Ala Ile Ile Pro Gly Ala Gly
    130                 135                 140

ggg aca cag cgg ctg ccc cgc ttg atc ggc agg gga aag gcg aaa gaa     480
Gly Thr Gln Arg Leu Pro Arg Leu Ile Gly Arg Gly Lys Ala Lys Glu
145                 150                 155                 160

atg atc ttt acc ggc tgc cgc atc agc gcc gaa gaa gcg caa aag atc     528
Met Ile Phe Thr Gly Cys Arg Ile Ser Ala Glu Glu Ala Gln Lys Ile
                165                 170                 175

agc ctg gtt gaa cat gtc gtt ccg ctt tcg aag tta aag gaa gcg agt     576
Ser Leu Val Glu His Val Val Pro Leu Ser Lys Leu Lys Glu Ala Ser
            180                 185                 190

gaa agc atc gcg gcg aac atc gcg gcg aac gga ccg gta gcc gtc aga     624
Glu Ser Ile Ala Ala Asn Ile Ala Ala Asn Gly Pro Val Ala Val Arg
        195                 200                 205

caa gcg aag ttt gcc atc aat caa ggc ctt gag aca gct atc gaa aca     672
Gln Ala Lys Phe Ala Ile Asn Gln Gly Leu Glu Thr Ala Ile Glu Thr
    210                 215                 220

ggg ctt gcc att gaa caa aaa gcc tat gaa ctg acg att ccg acg aaa     720
Gly Leu Ala Ile Glu Gln Lys Ala Tyr Glu Leu Thr Ile Pro Thr Lys
225                 230                 235                 240

gac agg aca gaa ggg ctg aaa gct ttt gca gaa aag cgg aag ccg gat     768
Asp Arg Thr Glu Gly Leu Lys Ala Phe Ala Glu Lys Arg Lys Pro Asp
                245                 250                 255

tat acg gga gaa taa aac                                             786
Tyr Thr Gly Glu
            260

<210>  26

<211>  260
<212>  PRT
<213>  Bacillus licheniformis DSM 13

<400>  26

Met Glu Pro Asn Val Leu Tyr Ser Ile Asn Glu Gln Ser Val Ala Val
1               5                   10                  15

Leu Thr Leu Asn Arg Pro Gln Ala Ala Asn Ala Leu Ser Leu Gly Leu
            20                  25                  30

Leu Asp Asp Phe Gln Arg Ile Leu Arg Asp Ile Arg Ser Asn Pro Ala
        35                  40                  45

Val Arg Cys Val Ile Ile Thr Gly Lys Gly Asp Arg Thr Phe Cys Ala
    50                  55                  60

Gly Ala Asp Leu Lys Glu Arg Ala Arg Met Ser Gln Thr Glu Ala Lys
65                  70                  75                  80

Gln Ala Val Ser Leu Ile Gln Arg Val Val Ser Glu Thr Glu Lys Leu
                85                  90                  95

Pro Gln Pro Val Ile Ala Ser Leu Asn Gly Ser Ala Leu Gly Gly Gly
            100                 105                 110

Leu Glu Leu Ala Leu Ala Cys Asp Ile Arg Ile Ala Ala Glu His Ile
        115                 120                 125

Glu Leu Gly Leu Pro Glu Thr Thr Leu Ala Ile Ile Pro Gly Ala Gly
    130                 135                 140

Gly Thr Gln Arg Leu Pro Arg Leu Ile Gly Arg Gly Lys Ala Lys Glu
145                 150                 155                 160

Met Ile Phe Thr Gly Cys Arg Ile Ser Ala Glu Glu Ala Gln Lys Ile
                165                 170                 175

Ser Leu Val Glu His Val Val Pro Leu Ser Lys Leu Lys Glu Ala Ser
            180                 185                 190

Glu Ser Ile Ala Ala Asn Ile Ala Ala Asn Gly Pro Val Ala Val Arg
        195                 200                 205

Gln Ala Lys Phe Ala Ile Asn Gln Gly Leu Glu Thr Ala Ile Glu Thr

210                 215                 220

Gly Leu Ala Ile Glu Gln Lys Ala Tyr Glu Leu Thr Ile Pro Thr Lys
225                 230                 235                 240

Asp Arg Thr Glu Gly Leu Lys Ala Phe Ala Glu Lys Arg Lys Pro Asp
                245                 250                 255

Tyr Thr Gly Glu
            260

<210>  27
<211>  1164
<212>  DNA
<213>  Bacillus licheniformis DSM 13

<220>
<221>  gene
<222>  (1)..(1164)
<223>  Acyl-CoA dehydrogenase (E.C. 1.3.99.-), yusJ

<220>
<221>  CDS
<222>  (1)..(1161)
<223>

<400>  27
atg aat ttt gaa cta acc aga gaa cag caa atg att cgt gaa ctc gca       48
Met Asn Phe Glu Leu Thr Arg Glu Gln Gln Met Ile Arg Glu Leu Ala
1               5                   10                  15

aga gat ttt gcg aaa cag gaa att gca ccg cac gcc gaa cat gtt gac       96
Arg Asp Phe Ala Lys Gln Glu Ile Ala Pro His Ala Glu His Val Asp
            20                  25                  30

agg acg gga gaa ttt ccg att gag aca ttt aaa aaa atg ggg gag ctc      144
Arg Thr Gly Glu Phe Pro Ile Glu Thr Phe Lys Lys Met Gly Glu Leu
        35                  40                  45

ggc ctc ttg ggg att ccg ttt cct gaa agc tac ggc ggt tca ggc gga      192
Gly Leu Leu Gly Ile Pro Phe Pro Glu Ser Tyr Gly Gly Ser Gly Gly
    50                  55                  60

gat acg att tcc tat gca ctt agc gtc gaa gaa atc ggc aaa gcg tgc      240
Asp Thr Ile Ser Tyr Ala Leu Ser Val Glu Glu Ile Gly Lys Ala Cys
65                  70                  75                  80

gga agc acc ggt ctt agc tat gct gcg gct gta tcg ctc ggg gct gcg      288
Gly Ser Thr Gly Leu Ser Tyr Ala Ala Ala Val Ser Leu Gly Ala Ala
                85                  90                  95

ccg att tat tat ttc ggc act gaa gaa caa aaa caa gaa tat ctc gtc      336
Pro Ile Tyr Tyr Phe Gly Thr Glu Glu Gln Lys Gln Glu Tyr Leu Val

            100                 105                 110

ccg ctt gcg acg ggc cgg gcg ctc gga gca ttt ggg ctg acc gaa ccg 384
Pro Leu Ala Thr Gly Arg Ala Leu Gly Ala Phe Gly Leu Thr Glu Pro
        115                 120                 125

aat gca ggt tcc gat gcg ggc ggc acc cgg aca aaa gcc cgc tcg gaa 432
Asn Ala Gly Ser Asp Ala Gly Gly Thr Arg Thr Lys Ala Arg Ser Glu
    130                 135                 140

ggg gac agc tat gtg atc agc ggt gag aaa tgc tgg atc aca aat gca 480
Gly Asp Ser Tyr Val Ile Ser Gly Glu Lys Cys Trp Ile Thr Asn Ala
145                 150                 155                 160

gga ttt gcc agg acc gtc atc gtc acc gcc gtc acc gga ata gat gac 528
Gly Phe Ala Arg Thr Val Ile Val Thr Ala Val Thr Gly Ile Asp Asp
                165                 170                 175

aac gga aaa aac atc att tcc gcc atc atc gtt ccg aca gat tcg gag 576
Asn Gly Lys Asn Ile Ile Ser Ala Ile Ile Val Pro Thr Asp Ser Glu
            180                 185                 190

ggc ttc acc att aaa agc gaa tat gac aaa atg ggt gtc cgc ggc tcc 624
Gly Phe Thr Ile Lys Ser Glu Tyr Asp Lys Met Gly Val Arg Gly Ser
        195                 200                 205

aat aca tca cag ctc ata ttg gac aat gtc cgc gta cca aaa caa aat 672
Asn Thr Ser Gln Leu Ile Leu Asp Asn Val Arg Val Pro Lys Gln Asn
    210                 215                 220

cta ttg gga agc ccg gaa aaa ggg ttt aaa caa ttt ctc aat aca ctt 720
Leu Leu Gly Ser Pro Glu Lys Gly Phe Lys Gln Phe Leu Asn Thr Leu
225                 230                 235                 240

gac ggc ggc aga att tcg atc gca gcg ctg gct gtc ggt att gcc caa 768
Asp Gly Gly Arg Ile Ser Ile Ala Ala Leu Ala Val Gly Ile Ala Gln
                245                 250                 255

ggc gca ttt gag gcg gcg ctc aca tac gcg cgc gaa cga aaa caa ttc 816
Gly Ala Phe Glu Ala Ala Leu Thr Tyr Ala Arg Glu Arg Lys Gln Phe
            260                 265                 270

ggc cga ccg atc tct tat ttc cag gcg att cag ttc aag ctt gcc gac 864
Gly Arg Pro Ile Ser Tyr Phe Gln Ala Ile Gln Phe Lys Leu Ala Asp
        275                 280                 285

atg gcc atg gaa att gag ctc gcc cgc aat atg gtg ctg aag gcc gcc 912
Met Ala Met Glu Ile Glu Leu Ala Arg Asn Met Val Leu Lys Ala Ala
    290                 295                 300

tgg ctg aaa gat caa gga cgt ccg ttt aca aaa gaa gcg gct ttt gcc 960
Trp Leu Lys Asp Gln Gly Arg Pro Phe Thr Lys Glu Ala Ala Phe Ala
305                 310                 315                 320

aag ctt tat gcc tca gaa atg gcg ttc agg aca tgc aat cag tcc att 1008
Lys Leu Tyr Ala Ser Glu Met Ala Phe Arg Thr Cys Asn Gln Ser Ile
                325                 330                 335

caa ata cac gga gga tac ggg tat atg aaa gag tat gga gtg gag cgc 1056

Gln Ile His Gly Gly Tyr Gly Tyr Met Lys Glu Tyr Gly Val Glu Arg
            340                 345                 350

atg ctg cgg gac gca aaa tta atg gaa atc ggt gaa ggc act tca gaa      1104
Met Leu Arg Asp Ala Lys Leu Met Glu Ile Gly Glu Gly Thr Ser Glu
        355                 360                 365

att caa cgg ctc gtc atc gca agg cag ctc ggc atc ggc aaa caa gcg      1152
Ile Gln Arg Leu Val Ile Ala Arg Gln Leu Gly Ile Gly Lys Gln Ala
    370                 375                 380

ctg aaa tga aaa                                                      1164
Leu Lys
385


<210>  28
<211>  386
<212>  PRT
<213>  Bacillus licheniformis DSM 13

<400>  28

Met Asn Phe Glu Leu Thr Arg Glu Gln Gln Met Ile Arg Glu Leu Ala
1               5                   10                  15

Arg Asp Phe Ala Lys Gln Glu Ile Ala Pro His Ala Glu His Val Asp
            20                  25                  30

Arg Thr Gly Glu Phe Pro Ile Glu Thr Phe Lys Lys Met Gly Glu Leu
        35                  40                  45

Gly Leu Leu Gly Ile Pro Phe Pro Glu Ser Tyr Gly Gly Ser Gly Gly
    50                  55                  60

Asp Thr Ile Ser Tyr Ala Leu Ser Val Glu Glu Ile Gly Lys Ala Cys
65                  70                  75                  80

Gly Ser Thr Gly Leu Ser Tyr Ala Ala Ala Val Ser Leu Gly Ala Ala
                85                  90                  95

Pro Ile Tyr Tyr Phe Gly Thr Glu Glu Gln Lys Gln Glu Tyr Leu Val
            100                 105                 110

Pro Leu Ala Thr Gly Arg Ala Leu Gly Ala Phe Gly Leu Thr Glu Pro
        115                 120                 125

Asn Ala Gly Ser Asp Ala Gly Gly Thr Arg Thr Lys Ala Arg Ser Glu
    130                 135                 140

Gly Asp Ser Tyr Val Ile Ser Gly Glu Lys Cys Trp Ile Thr Asn Ala
145                 150                 155                 160

Gly Phe Ala Arg Thr Val Ile Val Thr Ala Val Thr Gly Ile Asp Asp
                165                 170                 175

Asn Gly Lys Asn Ile Ile Ser Ala Ile Ile Val Pro Thr Asp Ser Glu
            180                 185                 190

Gly Phe Thr Ile Lys Ser Glu Tyr Asp Lys Met Gly Val Arg Gly Ser
        195                 200                 205

Asn Thr Ser Gln Leu Ile Leu Asp Asn Val Arg Val Pro Lys Gln Asn
    210                 215                 220

Leu Leu Gly Ser Pro Glu Lys Gly Phe Lys Gln Phe Leu Asn Thr Leu
225                 230                 235                 240

Asp Gly Gly Arg Ile Ser Ile Ala Ala Leu Ala Val Gly Ile Ala Gln
                245                 250                 255

Gly Ala Phe Glu Ala Ala Leu Thr Tyr Ala Arg Glu Arg Lys Gln Phe
            260                 265                 270

Gly Arg Pro Ile Ser Tyr Phe Gln Ala Ile Gln Phe Lys Leu Ala Asp
        275                 280                 285

Met Ala Met Glu Ile Glu Leu Ala Arg Asn Met Val Leu Lys Ala Ala
    290                 295                 300

Trp Leu Lys Asp Gln Gly Arg Pro Phe Thr Lys Glu Ala Ala Phe Ala
305                 310                 315                 320

Lys Leu Tyr Ala Ser Glu Met Ala Phe Arg Thr Cys Asn Gln Ser Ile
                325                 330                 335

Gln Ile His Gly Gly Tyr Gly Tyr Met Lys Glu Tyr Gly Val Glu Arg
            340                 345                 350

Met Leu Arg Asp Ala Lys Leu Met Glu Ile Gly Glu Gly Thr Ser Glu
        355                 360                 365

Ile Gln Arg Leu Val Ile Ala Arg Gln Leu Gly Ile Gly Lys Gln Ala
    370                 375                 380

Leu Lys
385

<210> 29
<211> 876
<212> DNA
<213> Bacillus licheniformis DSM 13

<220>
<221> gene
<222> (1)..(876)
<223> Hypothetical oxidoreductase (E.C. 1.1.-.-), ykwC

<220>
<221> CDS
<222> (1)..(873)
<223>

<400> 29
atg aaa aaa acg gtc gga ttt atc gga ctc ggt gta atg gga aac agc 48
Met Lys Lys Thr Val Gly Phe Ile Gly Leu Gly Val Met Gly Asn Ser
1               5                   10                  15

atg gcc tcg cac att tta gcc gcg ggc tat ccc gtc agc gcc tat acg 96
Met Ala Ser His Ile Leu Ala Ala Gly Tyr Pro Val Ser Ala Tyr Thr
            20                  25                  30

aga acg aaa cac aaa gcg gac agc ctc gtg gaa aaa ggg gcg gaa tgg 144
Arg Thr Lys His Lys Ala Asp Ser Leu Val Glu Lys Gly Ala Glu Trp
        35                  40                  45

aaa tca tcc gtc aaa gca ctg gcg cag tcg tct gat gtg atc atc aca 192
Lys Ser Ser Val Lys Ala Leu Ala Gln Ser Ser Asp Val Ile Ile Thr
    50                  55                  60

atg gtc ggc tac cca aaa gac gtt gaa gag gtt tac ttt gga agc gaa 240
Met Val Gly Tyr Pro Lys Asp Val Glu Glu Val Tyr Phe Gly Ser Glu
65                  70                  75                  80

ggc att att gaa aat gcc aaa aaa ggt tcc tac ctt atc gat atg acg 288
Gly Ile Ile Glu Asn Ala Lys Lys Gly Ser Tyr Leu Ile Asp Met Thr
                85                  90                  95

act tcc aaa cct tcg ctt gcc aaa caa atc gag act gcc gca aaa gag 336
Thr Ser Lys Pro Ser Leu Ala Lys Gln Ile Glu Thr Ala Ala Lys Glu
            100                 105                 110

aaa gga ctt tac gct ttg gat gct ccg gtt tca ggc ggg gac gtc ggc 384
Lys Gly Leu Tyr Ala Leu Asp Ala Pro Val Ser Gly Gly Asp Val Gly
        115                 120                 125

gcg agg aac ggc acg ctc gct atc atg gtc gga gga gaa cgg aaa gct 432
Ala Arg Asn Gly Thr Leu Ala Ile Met Val Gly Gly Glu Arg Lys Ala
    130                 135                 140

tat gat gaa tgc tac ccg ctc ttt tcg atc atg ggt gaa aac atc cag 480
Tyr Asp Glu Cys Tyr Pro Leu Phe Ser Ile Met Gly Glu Asn Ile Gln
145                 150                 155                 160

tat cag ggg ccg gcc gga agc ggc cag cat acg aaa atg tgc aac cag 528
Tyr Gln Gly Pro Ala Gly Ser Gly Gln His Thr Lys Met Cys Asn Gln
                165                 170                 175

att gcg att gcc gca ggg atg atc ggc gtc gca gaa gcg atg gcc tac 576
Ile Ala Ile Ala Ala Gly Met Ile Gly Val Ala Glu Ala Met Ala Tyr
            180                 185                 190

gcc gaa aaa tcc gga ctc gat ccc gac aac gtg ctg aaa agc att acg 624
Ala Glu Lys Ser Gly Leu Asp Pro Asp Asn Val Leu Lys Ser Ile Thr
        195                 200                 205

acc ggc gct gcg gga agc tgg tcg ctc tca aat cta gcg cct aga atg 672
Thr Gly Ala Ala Gly Ser Trp Ser Leu Ser Asn Leu Ala Pro Arg Met
    210                 215                 220

ctg aaa ggc gac ttt gaa ccg ggt ttt tac gtc aaa cac ttt gtt aaa 720
Leu Lys Gly Asp Phe Glu Pro Gly Phe Tyr Val Lys His Phe Val Lys
225                 230                 235                 240

gac atg ggc atc gcg ctt gaa gag gcg gag ctg atg ggc gag aaa atg 768
Asp Met Gly Ile Ala Leu Glu Glu Ala Glu Leu Met Gly Glu Lys Met
                245                 250                 255

ccg ggg ctc gag ctt gcg aaa agc ctt tac gac acc ctt gtt gaa aaa 816
Pro Gly Leu Glu Leu Ala Lys Ser Leu Tyr Asp Thr Leu Val Glu Lys
            260                 265                 270

ggc gaa gaa aac agc ggc acc caa agt ctg tac aag ctt tgg aca gaa 864
Gly Glu Glu Asn Ser Gly Thr Gln Ser Leu Tyr Lys Leu Trp Thr Glu
        275                 280                 285

tac aaa taa cga 876
Tyr Lys
    290


<210> 30
<211> 290
<212> PRT
<213> Bacillus licheniformis DSM 13

<400> 30

Met Lys Lys Thr Val Gly Phe Ile Gly Leu Gly Val Met Gly Asn Ser
1               5                   10                  15


Met Ala Ser His Ile Leu Ala Ala Gly Tyr Pro Val Ser Ala Tyr Thr
            20                  25                  30


Arg Thr Lys His Lys Ala Asp Ser Leu Val Glu Lys Gly Ala Glu Trp
        35                  40                  45

Lys Ser Ser Val Lys Ala Leu Ala Gln Ser Ser Asp Val Ile Ile Thr
    50                  55                  60

Met Val Gly Tyr Pro Lys Asp Val Glu Glu Val Tyr Phe Gly Ser Glu
65                  70                  75                  80

Gly Ile Ile Glu Asn Ala Lys Lys Gly Ser Tyr Leu Ile Asp Met Thr
                85                  90                  95

Thr Ser Lys Pro Ser Leu Ala Lys Gln Ile Glu Thr Ala Ala Lys Glu
            100                 105                 110

Lys Gly Leu Tyr Ala Leu Asp Ala Pro Val Ser Gly Gly Asp Val Gly
        115                 120                 125

Ala Arg Asn Gly Thr Leu Ala Ile Met Val Gly Gly Glu Arg Lys Ala
    130                 135                 140

Tyr Asp Glu Cys Tyr Pro Leu Phe Ser Ile Met Gly Glu Asn Ile Gln
145                 150                 155                 160

Tyr Gln Gly Pro Ala Gly Ser Gly Gln His Thr Lys Met Cys Asn Gln
                165                 170                 175

Ile Ala Ile Ala Ala Gly Met Ile Gly Val Ala Glu Ala Met Ala Tyr
            180                 185                 190

Ala Glu Lys Ser Gly Leu Asp Pro Asp Asn Val Leu Lys Ser Ile Thr
        195                 200                 205

Thr Gly Ala Ala Gly Ser Trp Ser Leu Ser Asn Leu Ala Pro Arg Met
    210                 215                 220

Leu Lys Gly Asp Phe Glu Pro Gly Phe Tyr Val Lys His Phe Val Lys
225                 230                 235                 240

Asp Met Gly Ile Ala Leu Glu Glu Ala Glu Leu Met Gly Glu Lys Met
                245                 250                 255

Pro Gly Leu Glu Leu Ala Lys Ser Leu Tyr Asp Thr Leu Val Glu Lys
            260                 265                 270

Gly Glu Glu Asn Ser Gly Thr Gln Ser Leu Tyr Lys Leu Trp Thr Glu
        275                 280                 285

Tyr Lys
    290

<210>  31
<211>  915
<212>  DNA
<213>  Bacillus licheniformis DSM 13

<220>
<221>  gene
<222>  (1)..(915)
<223>  Probable phosphate butyryltransferase (E.C. 2.3.1.19)

<220>
<221>  CDS
<222>  (1)..(912)
<223>

<400>  31
atg aag ctg aaa caa cta ttg caa aaa gcg gcg gag ctt gac aat aaa       48
Met Lys Leu Lys Gln Leu Leu Gln Lys Ala Ala Glu Leu Asp Asn Lys
1               5                   10                  15

acg gtc gcc gtc gca cat gcg gaa gat gac gaa gtg ctg caa gcg gtc       96
Thr Val Ala Val Ala His Ala Glu Asp Asp Glu Val Leu Gln Ala Val
            20                  25                  30

aaa ctt gcg gtc gac aaa caa ttt gcc cgg ttc ttg ctg atc ggg cac      144
Lys Leu Ala Val Asp Lys Gln Phe Ala Arg Phe Leu Leu Ile Gly His
        35                  40                  45

aga gaa aaa ctt aga cat atg atg aca gag cag aat att tca aaa cgg      192
Arg Glu Lys Leu Arg His Met Met Thr Glu Gln Asn Ile Ser Lys Arg
    50                  55                  60

cac gtg gat atc att cat tcg gaa tcg ccg gcg gat tct gcg aga att      240
His Val Asp Ile Ile His Ser Glu Ser Pro Ala Asp Ser Ala Arg Ile
65                  70                  75                  80

gcc gtt caa gct gtc aaa agc ggc aat gcg gat gtt ctt atg aag ggg      288
Ala Val Gln Ala Val Lys Ser Gly Asn Ala Asp Val Leu Met Lys Gly
                85                  90                  95

aat gtc ccg aca gct gtg cta tta aaa gcc gtt ttg aat aaa gag tac      336
Asn Val Pro Thr Ala Val Leu Leu Lys Ala Val Leu Asn Lys Glu Tyr
            100                 105                 110

ggg ctt cgt tcc tcg cac gtg ctg tca cat gta gca gca ttt gaa gtc      384
Gly Leu Arg Ser Ser His Val Leu Ser His Val Ala Ala Phe Glu Val
        115                 120                 125

agc ggg ttt gag agg ctg att tat gta aca gat gcg gcg atg aat atc      432
Ser Gly Phe Glu Arg Leu Ile Tyr Val Thr Asp Ala Ala Met Asn Ile
    130                 135                 140

agc ccc aag ctt gat gag ctg aag cag att tta gaa aac gca gtc ggc      480
Ser Pro Lys Leu Asp Glu Leu Lys Gln Ile Leu Glu Asn Ala Val Gly
145                 150                 155                 160

gtg gcg agg tcg gtc ggc gtg caa atg ccg aaa gtc gct tgt ctt gcc      528
Val Ala Arg Ser Val Gly Val Gln Met Pro Lys Val Ala Cys Leu Ala
                165                 170                 175

gca gtg gaa aca gtg aat ccc gcg atg gaa gcg aca ttg aat gca gct      576
Ala Val Glu Thr Val Asn Pro Ala Met Glu Ala Thr Leu Asn Ala Ala
            180                 185                 190

gcc ttg acg cag atg aat cat cgg ggc caa atc aaa aac tgc gtt gtt      624
Ala Leu Thr Gln Met Asn His Arg Gly Gln Ile Lys Asn Cys Val Val
        195                 200                 205

gac ggg cct ctt gca ttg gat aac gcg ata tcg ccg ctt gcc gcc cgg      672
Asp Gly Pro Leu Ala Leu Asp Asn Ala Ile Ser Pro Leu Ala Ala Arg
    210                 215                 220

cat aaa aac att tcc ggg atc gta gca ggc gag gcc gat atc ctg ctt      720
His Lys Asn Ile Ser Gly Ile Val Ala Gly Glu Ala Asp Ile Leu Leu
225                 230                 235                 240

gtt cct tca att gaa aca ggc aat gtc ctt tat aaa tca ttg att cat      768
Val Pro Ser Ile Glu Thr Gly Asn Val Leu Tyr Lys Ser Leu Ile His
                245                 250                 255

ttt gcg ggt gca aaa gtg gga gcc att tta gca ggg gca aaa gca ccc      816
Phe Ala Gly Ala Lys Val Gly Ala Ile Leu Ala Gly Ala Lys Ala Pro
            260                 265                 270

atc gcc ttg aca agc agg gcc gat tcc gca gaa aac aag ttg tat tcg      864
Ile Ala Leu Thr Ser Arg Ala Asp Ser Ala Glu Asn Lys Leu Tyr Ser
        275                 280                 285

att gct ttg gcg ctg tgt acg tct gaa gca cga cat gag gag gaa taa      912
Ile Ala Leu Ala Leu Cys Thr Ser Glu Ala Arg His Glu Glu Glu
    290                 295                 300

aaa                                                                  915

<210>  32
<211>  303
<212>  PRT
<213>  Bacillus licheniformis DSM 13

<400>  32

Met Lys Leu Lys Gln Leu Leu Gln Lys Ala Ala Glu Leu Asp Asn Lys
1               5                   10                  15

Thr Val Ala Val Ala His Ala Glu Asp Asp Glu Val Leu Gln Ala Val
            20                  25                  30

Lys Leu Ala Val Asp Lys Gln Phe Ala Arg Phe Leu Leu Ile Gly His
        35                  40                  45

Arg Glu Lys Leu Arg His Met Met Thr Glu Gln Asn Ile Ser Lys Arg
    50                  55                  60

His Val Asp Ile Ile His Ser Glu Ser Pro Ala Asp Ser Ala Arg Ile
65                  70                  75                  80

Ala Val Gln Ala Val Lys Ser Gly Asn Ala Asp Val Leu Met Lys Gly
                85                  90                  95

Asn Val Pro Thr Ala Val Leu Leu Lys Ala Val Leu Asn Lys Glu Tyr
            100                 105                 110

Gly Leu Arg Ser Ser His Val Leu Ser His Val Ala Ala Phe Glu Val
        115                 120                 125

Ser Gly Phe Glu Arg Leu Ile Tyr Val Thr Asp Ala Ala Met Asn Ile
    130                 135                 140

Ser Pro Lys Leu Asp Glu Leu Lys Gln Ile Leu Glu Asn Ala Val Gly
145                 150                 155                 160

Val Ala Arg Ser Val Gly Val Gln Met Pro Lys Val Ala Cys Leu Ala
                165                 170                 175

Ala Val Glu Thr Val Asn Pro Ala Met Glu Ala Thr Leu Asn Ala Ala
            180                 185                 190

Ala Leu Thr Gln Met Asn His Arg Gly Gln Ile Lys Asn Cys Val Val
        195                 200                 205

Asp Gly Pro Leu Ala Leu Asp Asn Ala Ile Ser Pro Leu Ala Ala Arg
    210                 215                 220

His Lys Asn Ile Ser Gly Ile Val Ala Gly Glu Ala Asp Ile Leu Leu
225                 230                 235                 240

Val Pro Ser Ile Glu Thr Gly Asn Val Leu Tyr Lys Ser Leu Ile His
                245                 250                 255

Phe Ala Gly Ala Lys Val Gly Ala Ile Leu Ala Gly Ala Lys Ala Pro
            260                 265                 270

Ile Ala Leu Thr Ser Arg Ala Asp Ser Ala Glu Asn Lys Leu Tyr Ser
        275                 280                 285

Ile Ala Leu Ala Leu Cys Thr Ser Glu Ala Arg His Glu Glu Glu
    290                 295                 300

<210>  33
<211>  1110
<212>  DNA
<213>  Bacillus licheniformis DSM 13

<220>
<221>  gene
<222>  (1)..(1110)
<223>  Probable butyrate kinase (E.C. 2.7.2.7)

<220>
<221>  CDS
<222>  (1)..(1107)
<223>

<400>  33
atg cag gta cag gaa aaa cgt att ctc gtc atc aat ccg gga tct aca       48
Met Gln Val Gln Glu Lys Arg Ile Leu Val Ile Asn Pro Gly Ser Thr
1               5                   10                  15

tct aca aag atc ggc gtt ttt cat gat gac cgt tcg att ttc gaa aaa       96
Ser Thr Lys Ile Gly Val Phe His Asp Asp Arg Ser Ile Phe Glu Lys
            20                  25                  30

tca atc cgt cat gac gag gct gag cta cag caa tat cag acc att att      144
Ser Ile Arg His Asp Glu Ala Glu Leu Gln Gln Tyr Gln Thr Ile Ile
        35                  40                  45

gat caa tat tcg ttc aga aaa cag gcg ata ctc gaa acc ctg cat gaa      192
Asp Gln Tyr Ser Phe Arg Lys Gln Ala Ile Leu Glu Thr Leu His Glu
    50                  55                  60

cag gga atc aat att tct aaa ttg gat gcc gtt tgc gcc agg gga ggg      240
Gln Gly Ile Asn Ile Ser Lys Leu Asp Ala Val Cys Ala Arg Gly Gly
65                  70                  75                  80

ctg ctt cgg ccg att gaa ggc ggc act tac gaa gtc aat gat gcg atg      288
Leu Leu Arg Pro Ile Glu Gly Gly Thr Tyr Glu Val Asn Asp Ala Met
                85                  90                  95

att gtc gat ttg aaa aac ggc tat gcg ggg cag cat gca tca aat ctc      336
Ile Val Asp Leu Lys Asn Gly Tyr Ala Gly Gln His Ala Ser Asn Leu
            100                 105                 110

ggg ggc atc atc gcc agg gag att gcc gac ggg tta aat att ccc gct      384
Gly Gly Ile Ile Ala Arg Glu Ile Ala Asp Gly Leu Asn Ile Pro Ala
        115                 120                 125

ttt atc gtc gac ccc gtt gtt gtg gat gaa atg gct cct atc gca aaa      432
Phe Ile Val Asp Pro Val Val Val Asp Glu Met Ala Pro Ile Ala Lys
    130                 135                 140

att tcc ggc acc ccg gct att gaa agg cgc agc att ttt cac gcg ctc      480
Ile Ser Gly Thr Pro Ala Ile Glu Arg Arg Ser Ile Phe His Ala Leu
145                 150                 155                 160

aac caa aaa gca gtt gca agg aaa gcg gct tgg cag ttt ggg aag cgt      528
Asn Gln Lys Ala Val Ala Arg Lys Ala Ala Trp Gln Phe Gly Lys Arg
                165                 170                 175

tat gaa gat atg aaa atg atc atc acc cac atg gga ggc ggc att acg      576
Tyr Glu Asp Met Lys Met Ile Ile Thr His Met Gly Gly Gly Ile Thr
            180                 185                 190

atc ggc gtc cat tgc cgc ggc cgg gtg atc gac gtc aac aac ggc ctc      624
Ile Gly Val His Cys Arg Gly Arg Val Ile Asp Val Asn Asn Gly Leu
        195                 200                 205

cac ggg gaa ggt ccg ctc agt cca gag cgg gcc gga acc att cct gcg      672
His Gly Glu Gly Pro Leu Ser Pro Glu Arg Ala Gly Thr Ile Pro Ala
    210                 215                 220

ggt gat ctg atc gat atg tgc ttt tcc ggc gaa tat acg aaa gac gag      720
Gly Asp Leu Ile Asp Met Cys Phe Ser Gly Glu Tyr Thr Lys Asp Glu
225                 230                 235                 240

ctg atg aaa atg ctt gtc ggc ggc gga ggg ctt gcc ggc tat ctc ggc      768
Leu Met Lys Met Leu Val Gly Gly Gly Gly Leu Ala Gly Tyr Leu Gly
                245                 250                 255

acg acg gat gcg gta aaa gtt gag aaa atg atc aag gaa ggc gat caa      816
Thr Thr Asp Ala Val Lys Val Glu Lys Met Ile Lys Glu Gly Asp Gln
            260                 265                 270

aaa gct gcg ctc atc tat gaa gcg atg gct tat caa atc gcc aaa gaa      864
Lys Ala Ala Leu Ile Tyr Glu Ala Met Ala Tyr Gln Ile Ala Lys Glu
        275                 280                 285

atc ggg gcg gcc agc gcc gtc tta aaa ggc gaa gtc gat gtc att att      912
Ile Gly Ala Ala Ser Ala Val Leu Lys Gly Glu Val Asp Val Ile Ile
    290                 295                 300

ttg aca gga gga ctg gca tat gga aaa tcg ttt att tcc tcg atc aga      960
Leu Thr Gly Gly Leu Ala Tyr Gly Lys Ser Phe Ile Ser Ser Ile Arg
305                 310                 315                 320

caa tac ata gac tgg att tcg gat gtc gtc gtc ttt cca gga gaa aat     1008
Gln Tyr Ile Asp Trp Ile Ser Asp Val Val Val Phe Pro Gly Glu Asn
                325                 330                 335

gaa ctt caa gca ttg gct gaa ggt gca ttt cgc gta ttg aac ggc gaa     1056
Glu Leu Gln Ala Leu Ala Glu Gly Ala Phe Arg Val Leu Asn Gly Glu
            340                 345                 350

gaa gag gca aaa cag tat ccg aac cag agg agg gaa agt cat ggc aac     1104
Glu Glu Ala Lys Gln Tyr Pro Asn Gln Arg Arg Glu Ser His Gly Asn
        355                 360                 365

tga ata                                                              1110

<210>  34
<211>  368
<212>  PRT
<213>  Bacillus licheniformis DSM 13

<400>  34

Met Gln Val Gln Glu Lys Arg Ile Leu Val Ile Asn Pro Gly Ser Thr
1               5                   10                  15

Ser Thr Lys Ile Gly Val Phe His Asp Asp Arg Ser Ile Phe Glu Lys
            20                  25                  30

Ser Ile Arg His Asp Glu Ala Glu Leu Gln Gln Tyr Gln Thr Ile Ile
        35                  40                  45

Asp Gln Tyr Ser Phe Arg Lys Gln Ala Ile Leu Glu Thr Leu His Glu
    50                  55                  60

Gln Gly Ile Asn Ile Ser Lys Leu Asp Ala Val Cys Ala Arg Gly Gly
65                  70                  75                  80

Leu Leu Arg Pro Ile Glu Gly Gly Thr Tyr Glu Val Asn Asp Ala Met
                85                  90                  95

Ile Val Asp Leu Lys Asn Gly Tyr Ala Gly Gln His Ala Ser Asn Leu
            100                 105                 110

Gly Gly Ile Ile Ala Arg Glu Ile Ala Asp Gly Leu Asn Ile Pro Ala
        115                 120                 125

Phe Ile Val Asp Pro Val Val Val Asp Glu Met Ala Pro Ile Ala Lys
    130                 135                 140

Ile Ser Gly Thr Pro Ala Ile Glu Arg Arg Ser Ile Phe His Ala Leu
145                 150                 155                 160

Asn Gln Lys Ala Val Ala Arg Lys Ala Ala Trp Gln Phe Gly Lys Arg
                165                 170                 175

Tyr Glu Asp Met Lys Met Ile Ile Thr His Met Gly Gly Gly Ile Thr
            180                 185                 190

Ile Gly Val His Cys Arg Gly Arg Val Ile Asp Val Asn Asn Gly Leu
        195                 200                 205

His Gly Glu Gly Pro Leu Ser Pro Glu Arg Ala Gly Thr Ile Pro Ala
    210                 215                 220

Gly Asp Leu Ile Asp Met Cys Phe Ser Gly Glu Tyr Thr Lys Asp Glu
225                 230                 235                 240

Leu Met Lys Met Leu Val Gly Gly Gly Gly Leu Ala Gly Tyr Leu Gly
                245                 250                 255

Thr Thr Asp Ala Val Lys Val Glu Lys Met Ile Lys Glu Gly Asp Gln
            260                 265                 270

Lys Ala Ala Leu Ile Tyr Glu Ala Met Ala Tyr Gln Ile Ala Lys Glu
        275                 280                 285

Ile Gly Ala Ala Ser Ala Val Leu Lys Gly Glu Val Asp Val Ile Ile
    290                 295                 300

Leu Thr Gly Gly Leu Ala Tyr Gly Lys Ser Phe Ile Ser Ser Ile Arg
305                 310                 315                 320

Gln Tyr Ile Asp Trp Ile Ser Asp Val Val Val Phe Pro Gly Glu Asn
                325                 330                 335

Glu Leu Gln Ala Leu Ala Glu Gly Ala Phe Arg Val Leu Asn Gly Glu
            340                 345                 350

Glu Glu Ala Lys Gln Tyr Pro Asn Gln Arg Arg Glu Ser His Gly Asn
        355                 360                 365

<210>  35
<211>  1722
<212>  DNA
<213>  Bacillus licheniformis DSM 13

<220>
<221>  gene
<222>  (1)..(1722)
<223>  Acetyl-coenzyme A synthetase (E.C. 6.2.1.1), acsA

<220>
<221>  CDS
<222>  (1)..(1719)
<223>

<400>  35
atg aaa ttg aaa gcg ctg cca gca gaa aag gga aat tac aac ttg aaa        48
Met Lys Leu Lys Ala Leu Pro Ala Glu Lys Gly Asn Tyr Asn Leu Lys
1               5                   10                  15

gac tat gat gaa aca tac cgg aca ttt gac tgg aag gat gcc gaa aag        96
Asp Tyr Asp Glu Thr Tyr Arg Thr Phe Asp Trp Lys Asp Ala Glu Lys
            20                  25                  30

cat ttt tca tgg cac aaa acg gga aaa atc aat gca gct tat gaa gct       144
His Phe Ser Trp His Lys Thr Gly Lys Ile Asn Ala Ala Tyr Glu Ala
        35                  40                  45

atc gac cgc cac gct gag tca aat ttg aaa aac aaa gtg gca ttt tac       192
Ile Asp Arg His Ala Glu Ser Asn Leu Lys Asn Lys Val Ala Phe Tyr
    50                  55                  60

tac aaa gat ccg gtc cgc gaa gaa aag tac act ttc aga gag atg aaa       240
Tyr Lys Asp Pro Val Arg Glu Glu Lys Tyr Thr Phe Arg Glu Met Lys
65                  70                  75                  80

aat gaa acc aac aaa gcc ggg aat gtc tta aag cag cat gcc gat gtg       288
Asn Glu Thr Asn Lys Ala Gly Asn Val Leu Lys Gln His Ala Asp Val
                85                  90                  95

gga aag gga gac cgt gtg ttt gtt ttt atg ccg aga tcg ccc gag ctt       336
Gly Lys Gly Asp Arg Val Phe Val Phe Met Pro Arg Ser Pro Glu Leu
            100                 105                 110

tat ttt att ctt ctc ggc gcc atc aaa ttg gga gcg atc gtc ggg ccg       384
Tyr Phe Ile Leu Leu Gly Ala Ile Lys Leu Gly Ala Ile Val Gly Pro
        115                 120                 125

tta ttt gaa gcg ttt atg gaa ggt gcc gtc aaa gac agg ctt gca aac       432
Leu Phe Glu Ala Phe Met Glu Gly Ala Val Lys Asp Arg Leu Ala Asn
    130                 135                 140

agc gga gcg aag gtc atc gtg acg acg ccg gaa ttg ctt gaa cgg gtg       480
Ser Gly Ala Lys Val Ile Val Thr Thr Pro Glu Leu Leu Glu Arg Val
145                 150                 155                 160

ccg gcc gat gaa ctt ccg gat ctt gaa tca atc att gtc gtt gga gaa       528
Pro Ala Asp Glu Leu Pro Asp Leu Glu Ser Ile Ile Val Val Gly Glu
                165                 170                 175

ggc gta aag gaa gaa gga cct gtc att gat tat tac gcg aaa gcg gcg       576
Gly Val Lys Glu Glu Gly Pro Val Ile Asp Tyr Tyr Ala Lys Ala Ala
            180                 185                 190

gaa gca ggc act gat ctt gag att gaa tgg gtg gat cag gaa gac ggg       624
Glu Ala Gly Thr Asp Leu Glu Ile Glu Trp Val Asp Gln Glu Asp Gly
        195                 200                 205

atg ctg ctt cac tat acg tcg ggt tcg acc ggc gcg cca aaa ggg gtt       672
Met Leu Leu His Tyr Thr Ser Gly Ser Thr Gly Ala Pro Lys Gly Val
    210                 215                 220

ctc cac gtc cat aaa gca atg atc cag cat tat caa aca gcc aaa tgg 720
Leu His Val His Lys Ala Met Ile Gln His Tyr Gln Thr Ala Lys Trp
225                 230                 235                 240

gtt ctt gat ctg cat gac gat gac atc tat tgg tgc acc gct gat ccc 768
Val Leu Asp Leu His Asp Asp Asp Ile Tyr Trp Cys Thr Ala Asp Pro
                245                 250                 255

ggc tgg gtc acc gga acg gtt tac ggg att ttc ggt ccg tgg ctg aat 816
Gly Trp Val Thr Gly Thr Val Tyr Gly Ile Phe Gly Pro Trp Leu Asn
            260                 265                 270

gga gct acg aat gtt gtc gta ggc ggc aga ttc agt cct gag gca tgg 864
Gly Ala Thr Asn Val Val Val Gly Gly Arg Phe Ser Pro Glu Ala Trp
        275                 280                 285

tac gaa acg att gaa aaa atg gaa gtg acg gta tgg tac agc gcg cca 912
Tyr Glu Thr Ile Glu Lys Met Glu Val Thr Val Trp Tyr Ser Ala Pro
    290                 295                 300

acg gct ttc cgg atg ctg atg ggt gca ggc gac gat ctt gtg aat aaa 960
Thr Ala Phe Arg Met Leu Met Gly Ala Gly Asp Asp Leu Val Asn Lys
305                 310                 315                 320

tat aat cta agc tcc ttg cgg cat att tta agc gta ggg gag ccg tta 1008
Tyr Asn Leu Ser Ser Leu Arg His Ile Leu Ser Val Gly Glu Pro Leu
                325                 330                 335

aat ccc gaa gtc atc agg tgg ggg cat aaa gtc ttc ggc aac cgg att 1056
Asn Pro Glu Val Ile Arg Trp Gly His Lys Val Phe Gly Asn Arg Ile
            340                 345                 350

cat gat act tgg tgg atg act gaa aca gga tcg cag ctc atc tgc aat 1104
His Asp Thr Trp Trp Met Thr Glu Thr Gly Ser Gln Leu Ile Cys Asn
        355                 360                 365

tac ccg tgc atg gaa att aaa ccg gga tca atg ggc aag ccg att ccc 1152
Tyr Pro Cys Met Glu Ile Lys Pro Gly Ser Met Gly Lys Pro Ile Pro
    370                 375                 380

ggt gta gag gct gca atc gtc gac aac cag gga aat gaa ctg cct cct 1200
Gly Val Glu Ala Ala Ile Val Asp Asn Gln Gly Asn Glu Leu Pro Pro
385                 390                 395                 400

tac aga atg gga aat ctc gcc att aaa aaa ggc tgg ccg tcg atg atg 1248
Tyr Arg Met Gly Asn Leu Ala Ile Lys Lys Gly Trp Pro Ser Met Met
                405                 410                 415

cat tcg atc tgg aac aat cct gaa aaa tat agc tcc tat ttt atg ccg 1296
His Ser Ile Trp Asn Asn Pro Glu Lys Tyr Ser Ser Tyr Phe Met Pro
            420                 425                 430

ggc gat tgg tat gtg tca gga gat tcc gcc tac atg gat gaa gac ggg 1344
Gly Asp Trp Tyr Val Ser Gly Asp Ser Ala Tyr Met Asp Glu Asp Gly
        435                 440                 445

tac ttc tgg ttc cag gga cgg atc gac gat gtc atc atg aca tcg ggc 1392
Tyr Phe Trp Phe Gln Gly Arg Ile Asp Asp Val Ile Met Thr Ser Gly
    450                 455                 460

gaa cgc gtc ggc ccg ttt gaa gtc gag agc aag ctt gtt gag cat cag     1440
Glu Arg Val Gly Pro Phe Glu Val Glu Ser Lys Leu Val Glu His Gln
465                 470                 475                 480

gcc gtc gct gaa gca ggc gtc atc ggc aaa ccg gat ccc gtc cgg ggt     1488
Ala Val Ala Glu Ala Gly Val Ile Gly Lys Pro Asp Pro Val Arg Gly
                485                 490                 495

gaa att att aaa gcg ttc atc gcc ttg agg gac ggt tat gaa ccg tca     1536
Glu Ile Ile Lys Ala Phe Ile Ala Leu Arg Asp Gly Tyr Glu Pro Ser
            500                 505                 510

gat gcg tta aaa gaa gaa atc agg cag ttc gtg aaa caa ggc ttg gcc     1584
Asp Ala Leu Lys Glu Glu Ile Arg Gln Phe Val Lys Gln Gly Leu Ala
        515                 520                 525

gca cat gcc gcg cca agg gaa atc gaa ttt aaa gat aaa ctg ccg aag     1632
Ala His Ala Ala Pro Arg Glu Ile Glu Phe Lys Asp Lys Leu Pro Lys
    530                 535                 540

aca aga agc gga aag atc atg aga cgc gtc ctg aaa gca tgg gag ctg     1680
Thr Arg Ser Gly Lys Ile Met Arg Arg Val Leu Lys Ala Trp Glu Leu
545                 550                 555                 560

aac ctt ccg gca ggc gat ctc tca tcg atg gaa gac tga tgt             1722
Asn Leu Pro Ala Gly Asp Leu Ser Ser Met Glu Asp
                565                 570


<210>  36
<211>  572
<212>  PRT
<213>  Bacillus licheniformis DSM 13

<400>  36

Met Lys Leu Lys Ala Leu Pro Ala Glu Lys Gly Asn Tyr Asn Leu Lys
1               5                   10                  15


Asp Tyr Asp Glu Thr Tyr Arg Thr Phe Asp Trp Lys Asp Ala Glu Lys
            20                  25                  30


His Phe Ser Trp His Lys Thr Gly Lys Ile Asn Ala Ala Tyr Glu Ala
        35                  40                  45


Ile Asp Arg His Ala Glu Ser Asn Leu Lys Asn Lys Val Ala Phe Tyr
    50                  55                  60


Tyr Lys Asp Pro Val Arg Glu Glu Lys Tyr Thr Phe Arg Glu Met Lys
65                  70                  75                  80


Asn Glu Thr Asn Lys Ala Gly Asn Val Leu Lys Gln His Ala Asp Val
                85                  90                  95

Gly Lys Gly Asp Arg Val Phe Val Phe Met Pro Arg Ser Pro Glu Leu
            100                 105                 110

Tyr Phe Ile Leu Leu Gly Ala Ile Lys Leu Gly Ala Ile Val Gly Pro
        115                 120                 125

Leu Phe Glu Ala Phe Met Glu Gly Ala Val Lys Asp Arg Leu Ala Asn
    130                 135                 140

Ser Gly Ala Lys Val Ile Val Thr Thr Pro Glu Leu Leu Glu Arg Val
145                 150                 155                 160

Pro Ala Asp Glu Leu Pro Asp Leu Glu Ser Ile Ile Val Val Gly Glu
                165                 170                 175

Gly Val Lys Glu Glu Gly Pro Val Ile Asp Tyr Tyr Ala Lys Ala Ala
            180                 185                 190

Glu Ala Gly Thr Asp Leu Glu Ile Glu Trp Val Asp Gln Glu Asp Gly
        195                 200                 205

Met Leu Leu His Tyr Thr Ser Gly Ser Thr Gly Ala Pro Lys Gly Val
    210                 215                 220

Leu His Val His Lys Ala Met Ile Gln His Tyr Gln Thr Ala Lys Trp
225                 230                 235                 240

Val Leu Asp Leu His Asp Asp Asp Ile Tyr Trp Cys Thr Ala Asp Pro
                245                 250                 255

Gly Trp Val Thr Gly Thr Val Tyr Gly Ile Phe Gly Pro Trp Leu Asn
            260                 265                 270

Gly Ala Thr Asn Val Val Val Gly Gly Arg Phe Ser Pro Glu Ala Trp
        275                 280                 285

Tyr Glu Thr Ile Glu Lys Met Glu Val Thr Val Trp Tyr Ser Ala Pro
    290                 295                 300

Thr Ala Phe Arg Met Leu Met Gly Ala Gly Asp Asp Leu Val Asn Lys
305                 310                 315                 320

Tyr Asn Leu Ser Ser Leu Arg His Ile Leu Ser Val Gly Glu Pro Leu

325                 330                 335

Asn Pro Glu Val Ile Arg Trp Gly His Lys Val Phe Gly Asn Arg Ile
            340                 345                 350

His Asp Thr Trp Trp Met Thr Glu Thr Gly Ser Gln Leu Ile Cys Asn
        355                 360                 365

Tyr Pro Cys Met Glu Ile Lys Pro Gly Ser Met Gly Lys Pro Ile Pro
    370                 375                 380

Gly Val Glu Ala Ala Ile Val Asp Asn Gln Gly Asn Glu Leu Pro Pro
385                 390                 395                 400

Tyr Arg Met Gly Asn Leu Ala Ile Lys Lys Gly Trp Pro Ser Met Met
                405                 410                 415

His Ser Ile Trp Asn Asn Pro Glu Lys Tyr Ser Ser Tyr Phe Met Pro
            420                 425                 430

Gly Asp Trp Tyr Val Ser Gly Asp Ser Ala Tyr Met Asp Glu Asp Gly
        435                 440                 445

Tyr Phe Trp Phe Gln Gly Arg Ile Asp Asp Val Ile Met Thr Ser Gly
    450                 455                 460

Glu Arg Val Gly Pro Phe Glu Val Glu Ser Lys Leu Val Glu His Gln
465                 470                 475                 480

Ala Val Ala Glu Ala Gly Val Ile Gly Lys Pro Asp Pro Val Arg Gly
                485                 490                 495

Glu Ile Ile Lys Ala Phe Ile Ala Leu Arg Asp Gly Tyr Glu Pro Ser
            500                 505                 510

Asp Ala Leu Lys Glu Glu Ile Arg Gln Phe Val Lys Gln Gly Leu Ala
        515                 520                 525

Ala His Ala Ala Pro Arg Glu Ile Glu Phe Lys Asp Lys Leu Pro Lys
    530                 535                 540

Thr Arg Ser Gly Lys Ile Met Arg Arg Val Leu Lys Ala Trp Glu Leu
545                 550                 555                 560

Asn Leu Pro Ala Gly Asp Leu Ser Ser Met Glu Asp
                565                 570

<210>  37
<211>  1593
<212>  DNA
<213>  Bacillus licheniformis DSM 13

<220>
<221>  gene
<222>  (1)..(1593)
<223>  acetate-CoA ligase (E.C. 6.2.1.1), ytcI

<220>
<221>  misc_feature
<222>  (1)..(3)
<223>  First codon translated as Met.

<220>
<221>  CDS
<222>  (1)..(1590)
<223>

<400>  37
ttg aga aga gaa gat ttg att gcg ccg gag aag tat aat gcg gtt gat       48
Leu Arg Arg Glu Asp Leu Ile Ala Pro Glu Lys Tyr Asn Ala Val Asp
1               5                   10                  15

gaa att gaa aaa ttt aaa tct tcc cgc gat aag acc gca ttg atc tgg       96
Glu Ile Glu Lys Phe Lys Ser Ser Arg Asp Lys Thr Ala Leu Ile Trp
            20                  25                  30

gaa gat gaa tca ggg cgt caa gtg tca tgg tcc tat gaa aaa ttg att      144
Glu Asp Glu Ser Gly Arg Gln Val Ser Trp Ser Tyr Glu Lys Leu Ile
        35                  40                  45

gaa aag gct tac aaa atc ggc agc ata ttg acc cgt tct gga ctg aaa      192
Glu Lys Ala Tyr Lys Ile Gly Ser Ile Leu Thr Arg Ser Gly Leu Lys
    50                  55                  60

aaa ggt gac aag ctt atc gtg atg atg ccg cgg ata ccg gaa acg tat      240
Lys Gly Asp Lys Leu Ile Val Met Met Pro Arg Ile Pro Glu Thr Tyr
65                  70                  75                  80

gcc gtg tac atg gcc att tta aaa gct gga atg gtg gtc atc cca tgt      288
Ala Val Tyr Met Ala Ile Leu Lys Ala Gly Met Val Val Ile Pro Cys
                85                  90                  95

tcc gaa atg ctt cgg gcg aaa gac ttg gat tac agg atc aag cat gca      336
Ser Glu Met Leu Arg Ala Lys Asp Leu Asp Tyr Arg Ile Lys His Ala
            100                 105                 110

ggc gtc aaa gga gcc gtc gta tat tca gca ttt ctt gat gct ttt cta      384
Gly Val Lys Gly Ala Val Val Tyr Ser Ala Phe Leu Asp Ala Phe Leu
        115                 120                 125

gat gtt cgt tca aaa gag gca ctg tcg tta ttt gcc gtc gga gaa agc 432
Asp Val Arg Ser Lys Glu Ala Leu Ser Leu Phe Ala Val Gly Glu Ser
    130                 135                 140

agc gaa ggg tgg atc aat ctg ctc gaa aaa atg aat cag gcc att gca 480
Ser Glu Gly Trp Ile Asn Leu Leu Glu Lys Met Asn Gln Ala Ile Ala
145                 150                 155                 160

gcg gat ttt caa gcg gcg gat acc tct cgc gat gac atc gca ttt tta 528
Ala Asp Phe Gln Ala Ala Asp Thr Ser Arg Asp Asp Ile Ala Phe Leu
                165                 170                 175

tct tac aca tcc ggc acg acc ggt cag cca aaa ggg gtt gta cat aca 576
Ser Tyr Thr Ser Gly Thr Thr Gly Gln Pro Lys Gly Val Val His Thr
            180                 185                 190

cac ggc tgg gct tat gct cac tta aga acg aca gct tcc gca tgg ctt 624
His Gly Trp Ala Tyr Ala His Leu Arg Thr Thr Ala Ser Ala Trp Leu
        195                 200                 205

gac att tcg gaa ggg gat ctc gtc tgg gcg aca gca ggg ccg ggc tgg 672
Asp Ile Ser Glu Gly Asp Leu Val Trp Ala Thr Ala Gly Pro Gly Trp
    210                 215                 220

caa aaa tgg gta tgg agc ccg ttt tta gca gtg ctc ggt agc ggt gca 720
Gln Lys Trp Val Trp Ser Pro Phe Leu Ala Val Leu Gly Ser Gly Ala
225                 230                 235                 240

acc ggc ttt atc tat cat gga aaa ttc acg ccg gaa acg tat ttg cgg 768
Thr Gly Phe Ile Tyr His Gly Lys Phe Thr Pro Glu Thr Tyr Leu Arg
                245                 250                 255

ctg att gag cgc cat caa gtc aat gtg ctg tgc tgt acg ccg aca gag 816
Leu Ile Glu Arg His Gln Val Asn Val Leu Cys Cys Thr Pro Thr Glu
            260                 265                 270

tac cgg ttc atg gcg aaa gtc aat gat ttg tcc cga ttt gac ctg tct 864
Tyr Arg Phe Met Ala Lys Val Asn Asp Leu Ser Arg Phe Asp Leu Ser
        275                 280                 285

tct ctg cac agc gct gtt tcg gcc gga gag ccg ttg aac agg gaa gtc 912
Ser Leu His Ser Ala Val Ser Ala Gly Glu Pro Leu Asn Arg Glu Val
    290                 295                 300

atc gat aca ttt aaa aag cat ttt cat att gct gtg cgg gac gga tac 960
Ile Asp Thr Phe Lys Lys His Phe His Ile Ala Val Arg Asp Gly Tyr
305                 310                 315                 320

gga caa acg gag agc acg ctg ttg gtg ggg att tta aaa ggt atg aaa 1008
Gly Gln Thr Glu Ser Thr Leu Leu Val Gly Ile Leu Lys Gly Met Lys
                325                 330                 335

atc aag cct gga agc atg gga aaa ccg acg cct gga aac ttg gtt gat 1056
Ile Lys Pro Gly Ser Met Gly Lys Pro Thr Pro Gly Asn Leu Val Asp
            340                 345                 350

att att gac ggg aat gga aag agc tgt ccc ccg ggc gaa aca ggc gat 1104
Ile Ile Asp Gly Asn Gly Lys Ser Cys Pro Pro Gly Glu Thr Gly Asp

355                 360                 365

att gcc gtt cac tta agc acg ccg gct ctt ttt aaa gaa tat tac aaa      1152
Ile Ala Val His Leu Ser Thr Pro Ala Leu Phe Lys Glu Tyr Tyr Lys
    370                 375                 380

gat caa gaa cga acg ctc cga caa aga aga ggg gat tac ttt ata aca      1200
Asp Gln Glu Arg Thr Leu Arg Gln Arg Arg Gly Asp Tyr Phe Ile Thr
385                 390                 395                 400

ggg gat aag gcg cga aag gat gaa gac ggc tat ttt tgg ttt gaa agc      1248
Gly Asp Lys Ala Arg Lys Asp Glu Asp Gly Tyr Phe Trp Phe Glu Ser
                405                 410                 415

cgc aat gac gat gtg atc atc agc tca gga tat aca atc ggc ccg ttt      1296
Arg Asn Asp Asp Val Ile Ile Ser Ser Gly Tyr Thr Ile Gly Pro Phe
            420                 425                 430

gaa gtt gaa gat gcg ctg atc aag cat cct gag gtc aaa gaa tgt gct      1344
Glu Val Glu Asp Ala Leu Ile Lys His Pro Glu Val Lys Glu Cys Ala
        435                 440                 445

gtt gtg gca agc cct gat gaa atc agg gga tcg atc gtg aaa gca tac      1392
Val Val Ala Ser Pro Asp Glu Ile Arg Gly Ser Ile Val Lys Ala Tyr
    450                 455                 460

gtt gtc tta aag gac cca tcc cgc gga aat gaa cac ctg aca aag gaa      1440
Val Val Leu Lys Asp Pro Ser Arg Gly Asn Glu His Leu Thr Lys Glu
465                 470                 475                 480

tta caa gac cat gta aaa gcc atg acg gcc cct tat aaa tac ccc cgg      1488
Leu Gln Asp His Val Lys Ala Met Thr Ala Pro Tyr Lys Tyr Pro Arg
                485                 490                 495

gag ata gaa ttc atc gaa gaa ctg ccg aaa acg cct tcg gcg aaa atc      1536
Glu Ile Glu Phe Ile Glu Glu Leu Pro Lys Thr Pro Ser Ala Lys Ile
            500                 505                 510

aag cgc ttt gaa cta aga caa cgg gaa atc gca ctt aaa acg aaa gct      1584
Lys Arg Phe Glu Leu Arg Gln Arg Glu Ile Ala Leu Lys Thr Lys Ala
        515                 520                 525

gaa taa cca                                                          1593
Glu

<210>  38
<211>  529
<212>  PRT
<213>  Bacillus licheniformis DSM 13

<220>
<221>  misc_feature
<222>  (1)..(3)
<223>  First codon translated as Met.

<400>  38

Leu Arg Arg Glu Asp Leu Ile Ala Pro Glu Lys Tyr Asn Ala Val Asp
1               5                   10                  15

Glu Ile Glu Lys Phe Lys Ser Ser Arg Asp Lys Thr Ala Leu Ile Trp
            20                  25                  30

Glu Asp Glu Ser Gly Arg Gln Val Ser Trp Ser Tyr Glu Lys Leu Ile
        35                  40                  45

Glu Lys Ala Tyr Lys Ile Gly Ser Ile Leu Thr Arg Ser Gly Leu Lys
    50                  55                  60

Lys Gly Asp Lys Leu Ile Val Met Met Pro Arg Ile Pro Glu Thr Tyr
65                  70                  75                  80

Ala Val Tyr Met Ala Ile Leu Lys Ala Gly Met Val Val Ile Pro Cys
                85                  90                  95

Ser Glu Met Leu Arg Ala Lys Asp Leu Asp Tyr Arg Ile Lys His Ala
            100                 105                 110

Gly Val Lys Gly Ala Val Val Tyr Ser Ala Phe Leu Asp Ala Phe Leu
        115                 120                 125

Asp Val Arg Ser Lys Glu Ala Leu Ser Leu Phe Ala Val Gly Glu Ser
    130                 135                 140

Ser Glu Gly Trp Ile Asn Leu Leu Glu Lys Met Asn Gln Ala Ile Ala
145                 150                 155                 160

Ala Asp Phe Gln Ala Ala Asp Thr Ser Arg Asp Asp Ile Ala Phe Leu
                165                 170                 175

Ser Tyr Thr Ser Gly Thr Thr Gly Gln Pro Lys Gly Val Val His Thr
            180                 185                 190

His Gly Trp Ala Tyr Ala His Leu Arg Thr Thr Ala Ser Ala Trp Leu
        195                 200                 205

Asp Ile Ser Glu Gly Asp Leu Val Trp Ala Thr Ala Gly Pro Gly Trp
    210                 215                 220

Gln Lys Trp Val Trp Ser Pro Phe Leu Ala Val Leu Gly Ser Gly Ala
225                 230                 235                 240

Thr Gly Phe Ile Tyr His Gly Lys Phe Thr Pro Glu Thr Tyr Leu Arg
                245                 250                 255

Leu Ile Glu Arg His Gln Val Asn Val Leu Cys Cys Thr Pro Thr Glu
            260                 265                 270

Tyr Arg Phe Met Ala Lys Val Asn Asp Leu Ser Arg Phe Asp Leu Ser
        275                 280                 285

Ser Leu His Ser Ala Val Ser Ala Gly Glu Pro Leu Asn Arg Glu Val
    290                 295                 300

Ile Asp Thr Phe Lys Lys His Phe His Ile Ala Val Arg Asp Gly Tyr
305                 310                 315                 320

Gly Gln Thr Glu Ser Thr Leu Leu Val Gly Ile Leu Lys Gly Met Lys
                325                 330                 335

Ile Lys Pro Gly Ser Met Gly Lys Pro Thr Pro Gly Asn Leu Val Asp
            340                 345                 350

Ile Ile Asp Gly Asn Gly Lys Ser Cys Pro Pro Gly Glu Thr Gly Asp
        355                 360                 365

Ile Ala Val His Leu Ser Thr Pro Ala Leu Phe Lys Glu Tyr Tyr Lys
    370                 375                 380

Asp Gln Glu Arg Thr Leu Arg Gln Arg Arg Gly Asp Tyr Phe Ile Thr
385                 390                 395                 400

Gly Asp Lys Ala Arg Lys Asp Glu Asp Gly Tyr Phe Trp Phe Glu Ser
                405                 410                 415

Arg Asn Asp Asp Val Ile Ile Ser Ser Gly Tyr Thr Ile Gly Pro Phe
            420                 425                 430

Glu Val Glu Asp Ala Leu Ile Lys His Pro Glu Val Lys Glu Cys Ala
        435                 440                 445

Val Val Ala Ser Pro Asp Glu Ile Arg Gly Ser Ile Val Lys Ala Tyr
    450                 455                 460

Val Val Leu Lys Asp Pro Ser Arg Gly Asn Glu His Leu Thr Lys Glu
465                 470                 475                 480

Leu Gln Asp His Val Lys Ala Met Thr Ala Pro Tyr Lys Tyr Pro Arg
                485                 490                 495

Glu Ile Glu Phe Ile Glu Glu Leu Pro Lys Thr Pro Ser Ala Lys Ile
            500                 505                 510

Lys Arg Phe Glu Leu Arg Gln Arg Glu Ile Ala Leu Lys Thr Lys Ala
        515                 520                 525

Glu

<210>  39
<211>  1524
<212>  DNA
<213>  Bacillus licheniformis DSM 13

<220>
<221>  gene
<222>  (1)..(1524)
<223>  Lysine and/or arginine decarboxylase (E.C. 4.1.1.18 or E.C. 4.1.1
       .19, respectively), speA

<220>
<221>  CDS
<222>  (1)..(1521)
<223>

<400>  39
atg cag aat gtc ata tcg gca tcc ggc agc gcc tgg atg ccg aag aaa       48
Met Gln Asn Val Ile Ser Ala Ser Gly Ser Ala Trp Met Pro Lys Lys
1               5                   10                  15

ata gag aag atg act aaa gga tcg gtt tat atg aag aca cct tta tat       96
Ile Glu Lys Met Thr Lys Gly Ser Val Tyr Met Lys Thr Pro Leu Tyr
            20                  25                  30

aca gca ttg gtg aac cat gct gaa gga cat cat tat tct ttt cac gtt      144
Thr Ala Leu Val Asn His Ala Glu Gly His His Tyr Ser Phe His Val
        35                  40                  45

ccg gga cat cac aat gga gat gtc ttt ttt gat gaa gca aaa act ttt      192
Pro Gly His His Asn Gly Asp Val Phe Phe Asp Glu Ala Lys Thr Phe
    50                  55                  60

ttt gaa acg att ctc aaa gtg gat cta act gaa ctg aca gga ctg gat      240
Phe Glu Thr Ile Leu Lys Val Asp Leu Thr Glu Leu Thr Gly Leu Asp
65                  70                  75                  80

gat tta cat gag cca tct ggc gtc atc aag gaa gca cag gat tta gtt      288
Asp Leu His Glu Pro Ser Gly Val Ile Lys Glu Ala Gln Asp Leu Val

85                  90                  95

tcg cgg ctt tac gga gcg gaa gaa agc ttt ttt ctc gtc aat ggt tcg      336
Ser Arg Leu Tyr Gly Ala Glu Glu Ser Phe Phe Leu Val Asn Gly Ser
            100                 105                 110

acc gtt ggg aat ctg gct atg atc ctg gct gtt tgt cag ccg ggt gat      384
Thr Val Gly Asn Leu Ala Met Ile Leu Ala Val Cys Gln Pro Gly Asp
        115                 120                 125

acg ata ctc gtt cag cgt aac tgc cat aag tct gta ttt cat gcg att      432
Thr Ile Leu Val Gln Arg Asn Cys His Lys Ser Val Phe His Ala Ile
    130                 135                 140

gaa ctt tca ggc gcc cat ccg gtt ttt ttg aca ccg gaa att gat gag      480
Glu Leu Ser Gly Ala His Pro Val Phe Leu Thr Pro Glu Ile Asp Glu
145                 150                 155                 160

gcg atg gct gtt ccc aca cat ata ctg tac gaa aca gtt gaa gat gca      528
Ala Met Ala Val Pro Thr His Ile Leu Tyr Glu Thr Val Glu Asp Ala
                165                 170                 175

att tca caa tat ccg cac gca aaa ggg atc gtg ttg aca tat cca aat      576
Ile Ser Gln Tyr Pro His Ala Lys Gly Ile Val Leu Thr Tyr Pro Asn
            180                 185                 190

tac tac ggc cat gca gtt gat ctg aag ccg atc ata gaa aag gcc cac      624
Tyr Tyr Gly His Ala Val Asp Leu Lys Pro Ile Ile Glu Lys Ala His
        195                 200                 205

caa cac gat att tcc gtt tta gtc gac gag gct cac ggc gcc cat ttt      672
Gln His Asp Ile Ser Val Leu Val Asp Glu Ala His Gly Ala His Phe
    210                 215                 220

gtg ctg ggc cat cca ttt ccc cag tcg tcg ttg aaa gcg ggc gct gat      720
Val Leu Gly His Pro Phe Pro Gln Ser Ser Leu Lys Ala Gly Ala Asp
225                 230                 235                 240

gcc gtc gtc cag tcc gcg cat aaa acg ctc ccg gcg atg acg atg ggg      768
Ala Val Val Gln Ser Ala His Lys Thr Leu Pro Ala Met Thr Met Gly
                245                 250                 255

tcc tat ctg cat ctt aac agc ggc agg atc aac agg gac aga ctg gca      816
Ser Tyr Leu His Leu Asn Ser Gly Arg Ile Asn Arg Asp Arg Leu Ala
            260                 265                 270

tac tat ctg tcc gtg ctt caa agc agc agt cct tcc tac cca att atg      864
Tyr Tyr Leu Ser Val Leu Gln Ser Ser Ser Pro Ser Tyr Pro Ile Met
        275                 280                 285

gca tct ttg gat ata gct agg gca tac gcc gaa gat atc ttg aaa aca      912
Ala Ser Leu Asp Ile Ala Arg Ala Tyr Ala Glu Asp Ile Leu Lys Thr
    290                 295                 300

aat cga aca gcc gat att gag aaa gag ctc atc aat atg aga gaa gtc      960
Asn Arg Thr Ala Asp Ile Glu Lys Glu Leu Ile Asn Met Arg Glu Val
305                 310                 315                 320

ttt tct caa ata aac gga gct gat att gtc gag ccg gct gat gcc cgt     1008

Phe Ser Gln Ile Asn Gly Ala Asp Ile Val Glu Pro Ala Asp Ala Arg
                325                 330                 335

atc cgg caa gat ccg ctt aag ctg tgt atc aga tca gcg tac ggc cac      1056
Ile Arg Gln Asp Pro Leu Lys Leu Cys Ile Arg Ser Ala Tyr Gly His
            340                 345                 350

tcc ggt ttt gaa ctg aaa tcc ata ttt gaa gca aac ggc atc cat cct      1104
Ser Gly Phe Glu Leu Lys Ser Ile Phe Glu Ala Asn Gly Ile His Pro
        355                 360                 365

gaa ttg gca gat gaa agg caa gtc ctg ttg atc ctg ccg ctt gaa gga      1152
Glu Leu Ala Asp Glu Arg Gln Val Leu Leu Ile Leu Pro Leu Glu Gly
    370                 375                 380

aaa aat atg ccc gcg ccg gaa ctg atc agc aca atc agt aaa gac atg      1200
Lys Asn Met Pro Ala Pro Glu Leu Ile Ser Thr Ile Ser Lys Asp Met
385                 390                 395                 400

aaa gat aca gca gtt cga aat gat ctt cct gcc gga atc gga atc cct      1248
Lys Asp Thr Ala Val Arg Asn Asp Leu Pro Ala Gly Ile Gly Ile Pro
                405                 410                 415

tct gaa aaa gtc acg gct ttg cct tac cgg aaa agc aag ttg tct gcc      1296
Ser Glu Lys Val Thr Ala Leu Pro Tyr Arg Lys Ser Lys Leu Ser Ala
            420                 425                 430

ttc aaa aaa gaa tcg gtg cct ttt acc gaa gca gcg ggc aga ata agc      1344
Phe Lys Lys Glu Ser Val Pro Phe Thr Glu Ala Ala Gly Arg Ile Ser
        435                 440                 445

gcc gaa tcg gtc aca cct tat cct ccg ggc att ccg ttg atc atg gcg      1392
Ala Glu Ser Val Thr Pro Tyr Pro Pro Gly Ile Pro Leu Ile Met Ala
    450                 455                 460

ggt gag aga ata aca aaa gag acg atc agc cgg ctg acg cgg ctc gtc      1440
Gly Glu Arg Ile Thr Lys Glu Thr Ile Ser Arg Leu Thr Arg Leu Val
465                 470                 475                 480

gat ctg aat gta cac att cag ggc agc aat cag ctc aaa caa aag caa      1488
Asp Leu Asn Val His Ile Gln Gly Ser Asn Gln Leu Lys Gln Lys Gln
                485                 490                 495

tta act gta tat ata gaa gag gag aaa tca tga acg                      1524
Leu Thr Val Tyr Ile Glu Glu Glu Lys Ser
            500                 505


<210>  40
<211>  506
<212>  PRT
<213>  Bacillus licheniformis DSM 13

<400>  40

Met Gln Asn Val Ile Ser Ala Ser Gly Ser Ala Trp Met Pro Lys Lys
1               5                   10                  15

Ile Glu Lys Met Thr Lys Gly Ser Val Tyr Met Lys Thr Pro Leu Tyr
            20                  25                  30

Thr Ala Leu Val Asn His Ala Glu Gly His His Tyr Ser Phe His Val
        35                  40                  45

Pro Gly His His Asn Gly Asp Val Phe Phe Asp Glu Ala Lys Thr Phe
    50                  55                  60

Phe Glu Thr Ile Leu Lys Val Asp Leu Thr Glu Leu Thr Gly Leu Asp
65                  70                  75                  80

Asp Leu His Glu Pro Ser Gly Val Ile Lys Glu Ala Gln Asp Leu Val
                85                  90                  95

Ser Arg Leu Tyr Gly Ala Glu Glu Ser Phe Phe Leu Val Asn Gly Ser
            100                 105                 110

Thr Val Gly Asn Leu Ala Met Ile Leu Ala Val Cys Gln Pro Gly Asp
        115                 120                 125

Thr Ile Leu Val Gln Arg Asn Cys His Lys Ser Val Phe His Ala Ile
    130                 135                 140

Glu Leu Ser Gly Ala His Pro Val Phe Leu Thr Pro Glu Ile Asp Glu
145                 150                 155                 160

Ala Met Ala Val Pro Thr His Ile Leu Tyr Glu Thr Val Glu Asp Ala
                165                 170                 175

Ile Ser Gln Tyr Pro His Ala Lys Gly Ile Val Leu Thr Tyr Pro Asn
            180                 185                 190

Tyr Tyr Gly His Ala Val Asp Leu Lys Pro Ile Ile Glu Lys Ala His
        195                 200                 205

Gln His Asp Ile Ser Val Leu Val Asp Glu Ala His Gly Ala His Phe
    210                 215                 220

Val Leu Gly His Pro Phe Pro Gln Ser Ser Leu Lys Ala Gly Ala Asp
225                 230                 235                 240

Ala Val Val Gln Ser Ala His Lys Thr Leu Pro Ala Met Thr Met Gly
                245                 250                 255

Ser Tyr Leu His Leu Asn Ser Gly Arg Ile Asn Arg Asp Arg Leu Ala
            260                 265                 270

Tyr Tyr Leu Ser Val Leu Gln Ser Ser Ser Pro Ser Tyr Pro Ile Met
        275                 280                 285

Ala Ser Leu Asp Ile Ala Arg Ala Tyr Ala Glu Asp Ile Leu Lys Thr
    290                 295                 300

Asn Arg Thr Ala Asp Ile Glu Lys Glu Leu Ile Asn Met Arg Glu Val
305                 310                 315                 320

Phe Ser Gln Ile Asn Gly Ala Asp Ile Val Glu Pro Ala Asp Ala Arg
                325                 330                 335

Ile Arg Gln Asp Pro Leu Lys Leu Cys Ile Arg Ser Ala Tyr Gly His
            340                 345                 350

Ser Gly Phe Glu Leu Lys Ser Ile Phe Glu Ala Asn Gly Ile His Pro
        355                 360                 365

Glu Leu Ala Asp Glu Arg Gln Val Leu Leu Ile Leu Pro Leu Glu Gly
    370                 375                 380

Lys Asn Met Pro Ala Pro Glu Leu Ile Ser Thr Ile Ser Lys Asp Met
385                 390                 395                 400

Lys Asp Thr Ala Val Arg Asn Asp Leu Pro Ala Gly Ile Gly Ile Pro
                405                 410                 415

Ser Glu Lys Val Thr Ala Leu Pro Tyr Arg Lys Ser Lys Leu Ser Ala
            420                 425                 430

Phe Lys Lys Glu Ser Val Pro Phe Thr Glu Ala Ala Gly Arg Ile Ser
        435                 440                 445

Ala Glu Ser Val Thr Pro Tyr Pro Pro Gly Ile Pro Leu Ile Met Ala
    450                 455                 460

Gly Glu Arg Ile Thr Lys Glu Thr Ile Ser Arg Leu Thr Arg Leu Val
465                 470                 475                 480

Asp Leu Asn Val His Ile Gln Gly Ser Asn Gln Leu Lys Gln Lys Gln
                485                 490                 495

Leu Thr Val Tyr Ile Glu Glu Glu Lys Ser
            500                 505

<210>  41
<211>  780
<212>  DNA
<213>  Bacillus licheniformis DSM 13

<220>
<221>  gene
<222>  (1)..(780)
<223>  probable enoyl-CoA hydratase (E.C. 4.2.1.17), ysiB

<220>
<221>  CDS
<222>  (1)..(777)
<223>

<400>  41
atg gca gca tta tca tac gcc gta gac ggc cat gtt gcg acg att acg       48
Met Ala Ala Leu Ser Tyr Ala Val Asp Gly His Val Ala Thr Ile Thr
1               5                   10                  15

att cag cac ccg ccg gca aat gcg cta tcg acg caa gtg ctt gaa gat       96
Ile Gln His Pro Pro Ala Asn Ala Leu Ser Thr Gln Val Leu Glu Asp
            20                  25                  30

ctt tcg gca tgc ctt gat gaa ctt tca gaa cgt cag gat gtc aga agc      144
Leu Ser Ala Cys Leu Asp Glu Leu Ser Glu Arg Gln Asp Val Arg Ser
        35                  40                  45

gtc gtt att cac ggg gaa gga aga ttt ttc tcg gca ggc gct gat att      192
Val Val Ile His Gly Glu Gly Arg Phe Phe Ser Ala Gly Ala Asp Ile
    50                  55                  60

aaa gag ttt aca tca ttg atg gac ggg tct gat tat gca aat ttg gct      240
Lys Glu Phe Thr Ser Leu Met Asp Gly Ser Asp Tyr Ala Asn Leu Ala
65                  70                  75                  80

gat aag ggc cag cag att ttc gaa aaa gta gaa tct ttt cca aaa ccg      288
Asp Lys Gly Gln Gln Ile Phe Glu Lys Val Glu Ser Phe Pro Lys Pro
                85                  90                  95

gtg atc gcc gcg att cac ggg gcc gct ctt gga ggc ggt ctt gag cta      336
Val Ile Ala Ala Ile His Gly Ala Ala Leu Gly Gly Gly Leu Glu Leu
            100                 105                 110

gcg atg gca tgc cac atc cgg att gcc gag gaa agc gca aag ctc ggc      384
Ala Met Ala Cys His Ile Arg Ile Ala Glu Glu Ser Ala Lys Leu Gly
        115                 120                 125

ctt ccc gaa ctg aat ctc gga atc att ccc ggc ttt gcg gga acg cag      432
Leu Pro Glu Leu Asn Leu Gly Ile Ile Pro Gly Phe Ala Gly Thr Gln
    130                 135                 140

cgc ctt ccc aag tat gtg ggc acc gcc aaa gcg ctt gaa atg atc ggg      480
Arg Leu Pro Lys Tyr Val Gly Thr Ala Lys Ala Leu Glu Met Ile Gly
145                 150                 155                 160

aca tca gag ccg ata tcc ggc aag gaa gct ttt gaa tac ggt ctt gtc      528
Thr Ser Glu Pro Ile Ser Gly Lys Glu Ala Phe Glu Tyr Gly Leu Val
                165                 170                 175

acg att ttg gcg gcg aat gaa gaa gaa gtg ctg caa aag gca aaa gag      576
Thr Ile Leu Ala Ala Asn Glu Glu Glu Val Leu Gln Lys Ala Lys Glu
            180                 185                 190

ctc gca caa aaa ttc gct gaa aaa agt ccg cag acg atg gct tat gtc      624
Leu Ala Gln Lys Phe Ala Glu Lys Ser Pro Gln Thr Met Ala Tyr Val
        195                 200                 205

att gaa ctc ctg aat tcg agc aaa gtg tat tcg tat gaa gga ggc ctc      672
Ile Glu Leu Leu Asn Ser Ser Lys Val Tyr Ser Tyr Glu Gly Gly Leu
    210                 215                 220

aag cta gaa ggg aaa tac ttc ggc gaa gtg ttc caa tcc gag gat gcg      720
Lys Leu Glu Gly Lys Tyr Phe Gly Glu Val Phe Gln Ser Glu Asp Ala
225                 230                 235                 240

aag gaa ggg att cag gca ttt ctt gaa aag cgg aag ccg cat ttt aaa      768
Lys Glu Gly Ile Gln Ala Phe Leu Glu Lys Arg Lys Pro His Phe Lys
                245                 250                 255

ggg aaa taa caa                                                      780
Gly Lys

<210>  42
<211>  258
<212>  PRT
<213>  Bacillus licheniformis DSM 13

<400>  42

Met Ala Ala Leu Ser Tyr Ala Val Asp Gly His Val Ala Thr Ile Thr
1               5                   10                  15

Ile Gln His Pro Pro Ala Asn Ala Leu Ser Thr Gln Val Leu Glu Asp
            20                  25                  30

Leu Ser Ala Cys Leu Asp Glu Leu Ser Glu Arg Gln Asp Val Arg Ser
        35                  40                  45

Val Val Ile His Gly Glu Gly Arg Phe Phe Ser Ala Gly Ala Asp Ile
    50                  55                  60

Lys Glu Phe Thr Ser Leu Met Asp Gly Ser Asp Tyr Ala Asn Leu Ala
65                  70                  75                  80

Asp Lys Gly Gln Gln Ile Phe Glu Lys Val Glu Ser Phe Pro Lys Pro
                85                  90                  95

Val Ile Ala Ala Ile His Gly Ala Ala Leu Gly Gly Gly Leu Glu Leu
            100                 105                 110

Ala Met Ala Cys His Ile Arg Ile Ala Glu Glu Ser Ala Lys Leu Gly
        115                 120                 125

Leu Pro Glu Leu Asn Leu Gly Ile Ile Pro Gly Phe Ala Gly Thr Gln
    130                 135                 140

Arg Leu Pro Lys Tyr Val Gly Thr Ala Lys Ala Leu Glu Met Ile Gly
145                 150                 155                 160

Thr Ser Glu Pro Ile Ser Gly Lys Glu Ala Phe Glu Tyr Gly Leu Val
                165                 170                 175

Thr Ile Leu Ala Ala Asn Glu Glu Glu Val Leu Gln Lys Ala Lys Glu
            180                 185                 190

Leu Ala Gln Lys Phe Ala Glu Lys Ser Pro Gln Thr Met Ala Tyr Val
        195                 200                 205

Ile Glu Leu Leu Asn Ser Ser Lys Val Tyr Ser Tyr Glu Gly Gly Leu
    210                 215                 220

Lys Leu Glu Gly Lys Tyr Phe Gly Glu Val Phe Gln Ser Glu Asp Ala
225                 230                 235                 240

Lys Glu Gly Ile Gln Ala Phe Leu Glu Lys Arg Lys Pro His Phe Lys
                245                 250                 255

Gly Lys

<210>  43
<211>  2394
<212>  DNA
<213>  Bacillus licheniformis DSM 13

<220>
<221>  gene
<222>  (1)..(2394)
<223>  similar to 3-hydroxyacyl-CoA dehydrogenase (E.C. 1.1.1.35)

<220>
<221>  CDS
<222>  (1)..(2391)
<223>

<400>  43
atg gtc aag cat att cga aaa gcc gcc gtt atc ggt tcc ggt gtt atg        48
Met Val Lys His Ile Arg Lys Ala Ala Val Ile Gly Ser Gly Val Met
1               5                   10                  15

ggt tcc ggc atc gcg gct cac tta gcc aat atc ggg att cct gta tta        96
Gly Ser Gly Ile Ala Ala His Leu Ala Asn Ile Gly Ile Pro Val Leu
            20                  25                  30

ctg ctc gat atg gtg ccg cat gaa tta aca gat gag gaa agg aaa aaa       144
Leu Leu Asp Met Val Pro His Glu Leu Thr Asp Glu Glu Arg Lys Lys
        35                  40                  45

ggc cgc acc ctt gaa gac ccg gat gtg cgc aat cgg ctg gca cgg aca       192
Gly Arg Thr Leu Glu Asp Pro Asp Val Arg Asn Arg Leu Ala Arg Thr
    50                  55                  60

gcc gta caa aag ctg cta aag caa aaa ccg gct ccg ctg act tca aag       240
Ala Val Gln Lys Leu Leu Lys Gln Lys Pro Ala Pro Leu Thr Ser Lys
65                  70                  75                  80

aag aat atc agc cgt atc agc aca ggg aac atg gaa gac gat ttt gaa       288
Lys Asn Ile Ser Arg Ile Ser Thr Gly Asn Met Glu Asp Asp Phe Glu
                85                  90                  95

aag att aaa gat gcc gac tgg att att gaa gtt gtt gtc gaa aac ctg       336
Lys Ile Lys Asp Ala Asp Trp Ile Ile Glu Val Val Val Glu Asn Leu
            100                 105                 110

gag atc aaa aag caa gtg ttt tca aag gtc gat caa tac aga aaa caa       384
Glu Ile Lys Lys Gln Val Phe Ser Lys Val Asp Gln Tyr Arg Lys Gln
        115                 120                 125

gga agc atc gtc agc agc aac aca tca ggc atc tcc gtc agg cag atg       432
Gly Ser Ile Val Ser Ser Asn Thr Ser Gly Ile Ser Val Arg Gln Met
    130                 135                 140

gct gag gga agg tcc gct gat ttt aaa aag cac ttt tta gga acg cac       480
Ala Glu Gly Arg Ser Ala Asp Phe Lys Lys His Phe Leu Gly Thr His
145                 150                 155                 160

ttc ttc aat ccg gcc cgc tat tta aag ctc ctt gag gtg att ccg att       528
Phe Phe Asn Pro Ala Arg Tyr Leu Lys Leu Leu Glu Val Ile Pro Ile
                165                 170                 175

gac gaa acg gag ccc gaa gtg ctg tcc ttt atg aag aaa ttc ggc gaa       576
Asp Glu Thr Glu Pro Glu Val Leu Ser Phe Met Lys Lys Phe Gly Glu
            180                 185                 190

gac gtt ctt ggc aaa ggg gtt gtt gaa gcg aaa gac acg cca aac ttt       624
Asp Val Leu Gly Lys Gly Val Val Glu Ala Lys Asp Thr Pro Asn Phe

195                 200                 205

atc gcc aac cgc atc gga acc tac ggc ttg ctt gtg acg gtt cag gaa       672
Ile Ala Asn Arg Ile Gly Thr Tyr Gly Leu Leu Val Thr Val Gln Glu
    210                 215                 220

atg ctg aaa ggc ggc tac acg ata ggc gaa gtc gat tcg att aca ggt       720
Met Leu Lys Gly Gly Tyr Thr Ile Gly Glu Val Asp Ser Ile Thr Gly
225                 230                 235                 240

ccg ctg atc gga cgc ccg aaa agc gcg acc ttc aga acg ctc gat gtc       768
Pro Leu Ile Gly Arg Pro Lys Ser Ala Thr Phe Arg Thr Leu Asp Val
                245                 250                 255

gtc ggc ctt gat aca ttt tca cac gtt gcc aaa aat gtc tat aac caa       816
Val Gly Leu Asp Thr Phe Ser His Val Ala Lys Asn Val Tyr Asn Gln
            260                 265                 270

gtc acg ggc gaa gaa aaa aac gtt ttc cgg ctc cct gaa ttt att gaa       864
Val Thr Gly Glu Glu Lys Asn Val Phe Arg Leu Pro Glu Phe Ile Glu
        275                 280                 285

caa atg ctc gaa aaa ggc tgg atc ggg agc aaa gcg aaa caa ggt ttt       912
Gln Met Leu Glu Lys Gly Trp Ile Gly Ser Lys Ala Lys Gln Gly Phe
    290                 295                 300

tac aaa aaa gaa gga aaa gac atc ctt gag ctc gat ccg atg acg atg       960
Tyr Lys Lys Glu Gly Lys Asp Ile Leu Glu Leu Asp Pro Met Thr Met
305                 310                 315                 320

acc tac agc gcg cgt caa aca tta aaa aca tcg gga ctg gaa gcg gcc      1008
Thr Tyr Ser Ala Arg Gln Thr Leu Lys Thr Ser Gly Leu Glu Ala Ala
                325                 330                 335

aag caa atg aaa ggc gca gga gcg aga ttg aaa gcg ctt gtt tat tcc      1056
Lys Gln Met Lys Gly Ala Gly Ala Arg Leu Lys Ala Leu Val Tyr Ser
            340                 345                 350

gat gac agg gcg gga agc ctc ctt tgg aag att aca gct ccg acg ctt      1104
Asp Asp Arg Ala Gly Ser Leu Leu Trp Lys Ile Thr Ala Pro Thr Leu
        355                 360                 365

gta tat tcg gcg gag ctg aca ggg gaa atc gcc gac agc ata acg gcg      1152
Val Tyr Ser Ala Glu Leu Thr Gly Glu Ile Ala Asp Ser Ile Thr Ala
    370                 375                 380

gtt gat cag gcg atg aaa tgg gga ttc ggc tgg tcg gaa ggg ccg ttt      1200
Val Asp Gln Ala Met Lys Trp Gly Phe Gly Trp Ser Glu Gly Pro Phe
385                 390                 395                 400

gaa atg tgg gac agc atc ggt gtg aaa agc tcg gtg caa aag ctt gaa      1248
Glu Met Trp Asp Ser Ile Gly Val Lys Ser Ser Val Gln Lys Leu Glu
                405                 410                 415

gca gag ggc tgg aag gtc gcg gac tgg gtg aag gaa atg ctc gca aaa      1296
Ala Glu Gly Trp Lys Val Ala Asp Trp Val Lys Glu Met Leu Ala Lys
            420                 425                 430

gga cat gaa acc ttc tac ctt aag gaa aac gga aag acg ttc tac tac      1344

Gly His Glu Thr Phe Tyr Leu Lys Glu Asn Gly Lys Thr Phe Tyr Tyr
        435                 440                 445

tgt ttt gaa agc ggc gaa tac agg gcg ctt caa gaa aac aag aaa acg      1392
Cys Phe Glu Ser Gly Glu Tyr Arg Ala Leu Gln Glu Asn Lys Lys Thr
    450                 455                 460

atc cat ttg tcg tca ctc aaa gaa aca aaa ggc gtc atc aag aaa aac      1440
Ile His Leu Ser Ser Leu Lys Glu Thr Lys Gly Val Ile Lys Lys Asn
465                 470                 475                 480

agc ggg gca agt ttg atc gat tta ggc gat gat gtc gca ctg ctc gaa      1488
Ser Gly Ala Ser Leu Ile Asp Leu Gly Asp Asp Val Ala Leu Leu Glu
                485                 490                 495

ttt cac tcg aaa agc aac gca atc ggc ctg gac atc att caa atg ctg      1536
Phe His Ser Lys Ser Asn Ala Ile Gly Leu Asp Ile Ile Gln Met Leu
            500                 505                 510

aaa ttt gct ctc gag gaa gta gac gcc aac tat aaa ggg ctt gtg atc      1584
Lys Phe Ala Leu Glu Glu Val Asp Ala Asn Tyr Lys Gly Leu Val Ile
        515                 520                 525

ggc aac cag ggc aaa aat ttc tgc gtc gga gcc aat ctc gcg atg atg      1632
Gly Asn Gln Gly Lys Asn Phe Cys Val Gly Ala Asn Leu Ala Met Met
    530                 535                 540

ctg atg gaa gct caa gac gac aac tat atg gaa att gac atg atc atc      1680
Leu Met Glu Ala Gln Asp Asp Asn Tyr Met Glu Ile Asp Met Ile Ile
545                 550                 555                 560

cgt cag ttc caa gag acg atg atg aaa gtg aaa tac agt tcc aag ccg      1728
Arg Gln Phe Gln Glu Thr Met Met Lys Val Lys Tyr Ser Ser Lys Pro
                565                 570                 575

gtc gtc gct gct ccg ttc ggc atg acg ctt ggc ggg ggg act gag cta      1776
Val Val Ala Ala Pro Phe Gly Met Thr Leu Gly Gly Gly Thr Glu Leu
            580                 585                 590

tgc ctg cct gct gcc cgt gtt caa gcc tca agc gaa tca tac atg ggc      1824
Cys Leu Pro Ala Ala Arg Val Gln Ala Ser Ser Glu Ser Tyr Met Gly
        595                 600                 605

ctc gtt gaa aca ggc gtc ggt ctg att ccg ggc ggc gga ggc aat aaa      1872
Leu Val Glu Thr Gly Val Gly Leu Ile Pro Gly Gly Gly Gly Asn Lys
    610                 615                 620

gag ctt tat tta aat tac ttg aaa ggg ctg cct gaa ggc gtt aaa ccg      1920
Glu Leu Tyr Leu Asn Tyr Leu Lys Gly Leu Pro Glu Gly Val Lys Pro
625                 630                 635                 640

gat atc cag gag gcc gcc atc aag acg ttt gag aca att gcc ctt gca      1968
Asp Ile Gln Glu Ala Ala Ile Lys Thr Phe Glu Thr Ile Ala Leu Ala
                645                 650                 655

aaa acg tcg act tcg gct caa gaa gcg aaa gaa ctg aac atc tta acc      2016
Lys Thr Ser Thr Ser Ala Gln Glu Ala Lys Glu Leu Asn Ile Leu Thr
            660                 665                 670

gca gag gat caa atc agc atc aat cag gat cac ttg tta tac gat gcc      2064
Ala Glu Asp Gln Ile Ser Ile Asn Gln Asp His Leu Leu Tyr Asp Ala
        675                 680                 685

aaa aaa ctc gtc ctg aca ctt tcc gaa agc ggc tac cgt ccg ccg gtg      2112
Lys Lys Leu Val Leu Thr Leu Ser Glu Ser Gly Tyr Arg Pro Pro Val
    690                 695                 700

aaa gag aaa gtt ccg gtg acg ggc gaa acc ggc tat gcg gcg ctc ctg      2160
Lys Glu Lys Val Pro Val Thr Gly Glu Thr Gly Tyr Ala Ala Leu Leu
705                 710                 715                 720

ctc ggt gct gaa tcg tta aaa ctt tcc ggc gcc atc tct gaa cac gac      2208
Leu Gly Ala Glu Ser Leu Lys Leu Ser Gly Ala Ile Ser Glu His Asp
                725                 730                 735

atg aag att gcg aaa aaa ctg gcg ttt gtc atc gcg ggc ggc cga gtt      2256
Met Lys Ile Ala Lys Lys Leu Ala Phe Val Ile Ala Gly Gly Arg Val
            740                 745                 750

cca ttc ggc gcc gaa gta acg gaa gaa tac ttg ctc aat ttg gaa aga      2304
Pro Phe Gly Ala Glu Val Thr Glu Glu Tyr Leu Leu Asn Leu Glu Arg
        755                 760                 765

gaa gcg ttt ctg agc ctt gtg agc gaa ccg aaa tcg cag gcg aga atg      2352
Glu Ala Phe Leu Ser Leu Val Ser Glu Pro Lys Ser Gln Ala Arg Met
    770                 775                 780

cag cat atg ctt gtg aaa ggc aaa cct ttg cgt aat tag gag              2394
Gln His Met Leu Val Lys Gly Lys Pro Leu Arg Asn
785                 790                 795


<210>  44
<211>  796
<212>  PRT
<213>  Bacillus licheniformis DSM 13

<400>  44

Met Val Lys His Ile Arg Lys Ala Ala Val Ile Gly Ser Gly Val Met
1               5                   10                  15


Gly Ser Gly Ile Ala Ala His Leu Ala Asn Ile Gly Ile Pro Val Leu
            20                  25                  30


Leu Leu Asp Met Val Pro His Glu Leu Thr Asp Glu Glu Arg Lys Lys
        35                  40                  45


Gly Arg Thr Leu Glu Asp Pro Asp Val Arg Asn Arg Leu Ala Arg Thr
    50                  55                  60


Ala Val Gln Lys Leu Leu Lys Gln Lys Pro Ala Pro Leu Thr Ser Lys
65                  70                  75                  80

Lys Asn Ile Ser Arg Ile Ser Thr Gly Asn Met Glu Asp Asp Phe Glu
                85                  90                  95

Lys Ile Lys Asp Ala Asp Trp Ile Ile Glu Val Val Val Glu Asn Leu
            100                 105                 110

Glu Ile Lys Lys Gln Val Phe Ser Lys Val Asp Gln Tyr Arg Lys Gln
        115                 120                 125

Gly Ser Ile Val Ser Ser Asn Thr Ser Gly Ile Ser Val Arg Gln Met
    130                 135                 140

Ala Glu Gly Arg Ser Ala Asp Phe Lys Lys His Phe Leu Gly Thr His
145                 150                 155                 160

Phe Phe Asn Pro Ala Arg Tyr Leu Lys Leu Leu Glu Val Ile Pro Ile
                165                 170                 175

Asp Glu Thr Glu Pro Glu Val Leu Ser Phe Met Lys Lys Phe Gly Glu
            180                 185                 190

Asp Val Leu Gly Lys Gly Val Val Glu Ala Lys Asp Thr Pro Asn Phe
        195                 200                 205

Ile Ala Asn Arg Ile Gly Thr Tyr Gly Leu Leu Val Thr Val Gln Glu
    210                 215                 220

Met Leu Lys Gly Gly Tyr Thr Ile Gly Glu Val Asp Ser Ile Thr Gly
225                 230                 235                 240

Pro Leu Ile Gly Arg Pro Lys Ser Ala Thr Phe Arg Thr Leu Asp Val
                245                 250                 255

Val Gly Leu Asp Thr Phe Ser His Val Ala Lys Asn Val Tyr Asn Gln
            260                 265                 270

Val Thr Gly Glu Glu Lys Asn Val Phe Arg Leu Pro Glu Phe Ile Glu
        275                 280                 285

Gln Met Leu Glu Lys Gly Trp Ile Gly Ser Lys Ala Lys Gln Gly Phe
    290                 295                 300

Tyr Lys Lys Glu Gly Lys Asp Ile Leu Glu Leu Asp Pro Met Thr Met
305                 310                 315                 320

Thr Tyr Ser Ala Arg Gln Thr Leu Lys Thr Ser Gly Leu Glu Ala Ala
                325                 330                 335

Lys Gln Met Lys Gly Ala Gly Ala Arg Leu Lys Ala Leu Val Tyr Ser
            340                 345                 350

Asp Asp Arg Ala Gly Ser Leu Leu Trp Lys Ile Thr Ala Pro Thr Leu
        355                 360                 365

Val Tyr Ser Ala Glu Leu Thr Gly Glu Ile Ala Asp Ser Ile Thr Ala
    370                 375                 380

Val Asp Gln Ala Met Lys Trp Gly Phe Gly Trp Ser Glu Gly Pro Phe
385                 390                 395                 400

Glu Met Trp Asp Ser Ile Gly Val Lys Ser Ser Val Gln Lys Leu Glu
                405                 410                 415

Ala Glu Gly Trp Lys Val Ala Asp Trp Val Lys Glu Met Leu Ala Lys
            420                 425                 430

Gly His Glu Thr Phe Tyr Leu Lys Glu Asn Gly Lys Thr Phe Tyr Tyr
        435                 440                 445

Cys Phe Glu Ser Gly Glu Tyr Arg Ala Leu Gln Glu Asn Lys Lys Thr
    450                 455                 460

Ile His Leu Ser Ser Leu Lys Glu Thr Lys Gly Val Ile Lys Lys Asn
465                 470                 475                 480

Ser Gly Ala Ser Leu Ile Asp Leu Gly Asp Asp Val Ala Leu Leu Glu
                485                 490                 495

Phe His Ser Lys Ser Asn Ala Ile Gly Leu Asp Ile Ile Gln Met Leu
            500                 505                 510

Lys Phe Ala Leu Glu Glu Val Asp Ala Asn Tyr Lys Gly Leu Val Ile
        515                 520                 525

Gly Asn Gln Gly Lys Asn Phe Cys Val Gly Ala Asn Leu Ala Met Met
    530                 535                 540

Leu Met Glu Ala Gln Asp Asp Asn Tyr Met Glu Ile Asp Met Ile Ile

545                 550                 555                 560

Arg Gln Phe Gln Glu Thr Met Met Lys Val Lys Tyr Ser Ser Lys Pro
                565                 570                 575

Val Val Ala Ala Pro Phe Gly Met Thr Leu Gly Gly Gly Thr Glu Leu
            580                 585                 590

Cys Leu Pro Ala Ala Arg Val Gln Ala Ser Ser Glu Ser Tyr Met Gly
        595                 600                 605

Leu Val Glu Thr Gly Val Gly Leu Ile Pro Gly Gly Gly Gly Asn Lys
    610                 615                 620

Glu Leu Tyr Leu Asn Tyr Leu Lys Gly Leu Pro Glu Gly Val Lys Pro
625                 630                 635                 640

Asp Ile Gln Glu Ala Ala Ile Lys Thr Phe Glu Thr Ile Ala Leu Ala
                645                 650                 655

Lys Thr Ser Thr Ser Ala Gln Glu Ala Lys Glu Leu Asn Ile Leu Thr
            660                 665                 670

Ala Glu Asp Gln Ile Ser Ile Asn Gln Asp His Leu Leu Tyr Asp Ala
        675                 680                 685

Lys Lys Leu Val Leu Thr Leu Ser Glu Ser Gly Tyr Arg Pro Pro Val
    690                 695                 700

Lys Glu Lys Val Pro Val Thr Gly Glu Thr Gly Tyr Ala Ala Leu Leu
705                 710                 715                 720

Leu Gly Ala Glu Ser Leu Lys Leu Ser Gly Ala Ile Ser Glu His Asp
                725                 730                 735

Met Lys Ile Ala Lys Lys Leu Ala Phe Val Ile Ala Gly Gly Arg Val
            740                 745                 750

Pro Phe Gly Ala Glu Val Thr Glu Glu Tyr Leu Leu Asn Leu Glu Arg
        755                 760                 765

Glu Ala Phe Leu Ser Leu Val Ser Glu Pro Lys Ser Gln Ala Arg Met
    770                 775                 780

Gln His Met Leu Val Lys Gly Lys Pro Leu Arg Asn
785                 790                 795

<210>  45
<211>  2838
<212>  DNA
<213>  Bacillus licheniformis DSM 13

<220>
<221>  gene
<222>  (1)..(2838)
<223>  2-oxoglutarate dehydrogenase E1 component (E.C. 1.2.4.2)

<220>
<221>  CDS
<222>  (1)..(2835)
<223>

<400>  45
atg ttt caa aat agt atg aaa caa aga atg act tgg gaa gaa ttt cac       48
Met Phe Gln Asn Ser Met Lys Gln Arg Met Thr Trp Glu Glu Phe His
1               5                   10                  15

ggt ccg aac ctc ggc tat gtg ctg gag ctt tac gat cag tac gtc aag       96
Gly Pro Asn Leu Gly Tyr Val Leu Glu Leu Tyr Asp Gln Tyr Val Lys
            20                  25                  30

gat cca gaa agc ttg gat gct gat tta aaa gag atg ttt gac gaa ctt      144
Asp Pro Glu Ser Leu Asp Ala Asp Leu Lys Glu Met Phe Asp Glu Leu
        35                  40                  45

gga gct ccc ccg ggc gat att agg gcc gcc tct caa aaa aac gaa gag      192
Gly Ala Pro Pro Gly Asp Ile Arg Ala Ala Ser Gln Lys Asn Glu Glu
    50                  55                  60

gca gat ttc acg gct gga tct att caa aaa atc gca tca gcg gta aaa      240
Ala Asp Phe Thr Ala Gly Ser Ile Gln Lys Ile Ala Ser Ala Val Lys
65                  70                  75                  80

ctt gca gaa gat att aga acc tat ggc cat tta aac gct tcc gtc aat      288
Leu Ala Glu Asp Ile Arg Thr Tyr Gly His Leu Asn Ala Ser Val Asn
                85                  90                  95

cca ctg aga aaa aca caa gag aaa cag gag ctt ttt cct ctt gct gag      336
Pro Leu Arg Lys Thr Gln Glu Lys Gln Glu Leu Phe Pro Leu Ala Glu
            100                 105                 110

tac ggg tta act gag cag gat gtg aaa aaa atc ccg gcg tct gtc ata      384
Tyr Gly Leu Thr Glu Gln Asp Val Lys Lys Ile Pro Ala Ser Val Ile
        115                 120                 125

tgc aaa gat gcc cct aaa gaa gta acg aac ggt tta gaa gcc atc cag      432
Cys Lys Asp Ala Pro Lys Glu Val Thr Asn Gly Leu Glu Ala Ile Gln
    130                 135                 140

tac tta aga aac aca tac aaa aaa tcg att tct ttt gaa ttt gac cat      480

Tyr Leu Arg Asn Thr Tyr Lys Lys Ser Ile Ser Phe Glu Phe Asp His
145                 150                 155                 160

gtg cac att ttt gaa gag cgc aac tgg ctg atg aaa aag atc gaa tcc      528
Val His Ile Phe Glu Glu Arg Asn Trp Leu Met Lys Lys Ile Glu Ser
                165                 170                 175

ggg gaa tta ttc acc ccg aaa tcg aaa gaa aaa ctg gta gaa gtt tta      576
Gly Glu Leu Phe Thr Pro Lys Ser Lys Glu Lys Leu Val Glu Val Leu
            180                 185                 190

aga agg ctt aca gaa gtg gaa agc ctt gaa cag ttt ctc cac aaa acc      624
Arg Arg Leu Thr Glu Val Glu Ser Leu Glu Gln Phe Leu His Lys Thr
        195                 200                 205

ttt gtc ggg caa aaa cgc ttt tca ata gaa gga ctt gat gcg ctt gtg      672
Phe Val Gly Gln Lys Arg Phe Ser Ile Glu Gly Leu Asp Ala Leu Val
    210                 215                 220

ccc atg ctg gat gat att atc gcc aag tcc gtt tcg gca ggt acg aca      720
Pro Met Leu Asp Asp Ile Ile Ala Lys Ser Val Ser Ala Gly Thr Thr
225                 230                 235                 240

aac gtc aat atc gga atg gcg cac agg ggc cgc ctg aat gtt ctt gcg      768
Asn Val Asn Ile Gly Met Ala His Arg Gly Arg Leu Asn Val Leu Ala
                245                 250                 255

cat gtg ctc gga aaa cct tat gaa atc att ttt tct gaa ttc cag cat      816
His Val Leu Gly Lys Pro Tyr Glu Ile Ile Phe Ser Glu Phe Gln His
            260                 265                 270

gcg ccg aac aaa gat ctc gtt ccg tcg gaa ggt tcg acc ggg atc aat      864
Ala Pro Asn Lys Asp Leu Val Pro Ser Glu Gly Ser Thr Gly Ile Asn
        275                 280                 285

tac ggc tgg acg ggc gac gta aaa tac cac ctc ggc gcc aac cgc cag      912
Tyr Gly Trp Thr Gly Asp Val Lys Tyr His Leu Gly Ala Asn Arg Gln
    290                 295                 300

att cag gat gag cat acg aaa acg gcg cgc att gcg ctc gcg aac aat      960
Ile Gln Asp Glu His Thr Lys Thr Ala Arg Ile Ala Leu Ala Asn Asn
305                 310                 315                 320

ccg agc cac ctt gag ttc atc gat ccg atc gtt gag gga tcg aca aga     1008
Pro Ser His Leu Glu Phe Ile Asp Pro Ile Val Glu Gly Ser Thr Arg
                325                 330                 335

gcc gcc cag gaa acg aga acg gag agc ggc tat ccg gtt caa gac gtc     1056
Ala Ala Gln Glu Thr Arg Thr Glu Ser Gly Tyr Pro Val Gln Asp Val
            340                 345                 350

aaa aaa tcg atg gcg att ctg att cac ggc gat gcg gca ttc cca ggg     1104
Lys Lys Ser Met Ala Ile Leu Ile His Gly Asp Ala Ala Phe Pro Gly
        355                 360                 365

gaa ggc att gtc gcg gaa acg ctg aat tta agc cag ctt aaa ggg tat     1152
Glu Gly Ile Val Ala Glu Thr Leu Asn Leu Ser Gln Leu Lys Gly Tyr
    370                 375                 380

caa gtg ggc gga gcg att cac att atc gcc aat aac atg atc ggc ttt      1200
Gln Val Gly Gly Ala Ile His Ile Ile Ala Asn Asn Met Ile Gly Phe
385                 390                 395                 400

acg acg gaa agc aat gag tca aga tcg acg aaa tat gca agc gac ctt      1248
Thr Thr Glu Ser Asn Glu Ser Arg Ser Thr Lys Tyr Ala Ser Asp Leu
                405                 410                 415

gcg aaa ggt ttt gaa att ccg atc gtc cac gtc aat gct gat gat ccc      1296
Ala Lys Gly Phe Glu Ile Pro Ile Val His Val Asn Ala Asp Asp Pro
            420                 425                 430

gaa gca tgt ctt tca gcg gtt cag ctc gct gtt gaa tac cgc atg act      1344
Glu Ala Cys Leu Ser Ala Val Gln Leu Ala Val Glu Tyr Arg Met Thr
        435                 440                 445

ttc aac aaa gac ttt ttg atc gat ctg atc ggc tac cgc cgt ttt ggc      1392
Phe Asn Lys Asp Phe Leu Ile Asp Leu Ile Gly Tyr Arg Arg Phe Gly
    450                 455                 460

cac aat gaa atg gat gag ccg tcc gca acg cag ccg atg ctg tat gat      1440
His Asn Glu Met Asp Glu Pro Ser Ala Thr Gln Pro Met Leu Tyr Asp
465                 470                 475                 480

gcg gtc aga aag cat ccg acc gtc aaa aac atc ttt gct gaa aag ctg      1488
Ala Val Arg Lys His Pro Thr Val Lys Asn Ile Phe Ala Glu Lys Leu
                485                 490                 495

att cat aaa ggg atc gtc gat aaa gaa acc gtc ggc aaa atc aag gac      1536
Ile His Lys Gly Ile Val Asp Lys Glu Thr Val Gly Lys Ile Lys Asp
            500                 505                 510

gct gtc cag aag cgt tta gaa gaa gcc tat cgc aaa gtg ccg gcc aaa      1584
Ala Val Gln Lys Arg Leu Glu Glu Ala Tyr Arg Lys Val Pro Ala Lys
        515                 520                 525

aag gaa gac atg acg cat gaa atc gta ctt cca gag ccg gtc tcc aac      1632
Lys Glu Asp Met Thr His Glu Ile Val Leu Pro Glu Pro Val Ser Asn
    530                 535                 540

ggt ttt cct gat gtt gac aca tcg gtt gat ttt gaa act ttg cgc aaa      1680
Gly Phe Pro Asp Val Asp Thr Ser Val Asp Phe Glu Thr Leu Arg Lys
545                 550                 555                 560

atc aat cag gag ctt gtt tca tgg ccg gaa aac ttc aac gtt ttc gat      1728
Ile Asn Gln Glu Leu Val Ser Trp Pro Glu Asn Phe Asn Val Phe Asp
                565                 570                 575

aag cta aaa cga atc ctt gaa agg cgc gcc aaa gct ttc gaa gat gac      1776
Lys Leu Lys Arg Ile Leu Glu Arg Arg Ala Lys Ala Phe Glu Asp Asp
            580                 585                 590

cga aaa gtc gac tgg tcg ctt gca gag gcg atg gcg ttt gcg tcg att      1824
Arg Lys Val Asp Trp Ser Leu Ala Glu Ala Met Ala Phe Ala Ser Ile
        595                 600                 605

ttg aaa gac ggt acg ccg cta agg ctg acc ggg cag gat tca gaa cgc      1872
Leu Lys Asp Gly Thr Pro Leu Arg Leu Thr Gly Gln Asp Ser Glu Arg
    610                 615                 620

ggc aca ttc gca cac cgc aac ctt gtc ctt cac gac agc aag aca ggg     1920
Gly Thr Phe Ala His Arg Asn Leu Val Leu His Asp Ser Lys Thr Gly
625                 630                 635                 640

gac gaa ttc atc gcg ctg cat cac ctt gcc gat acg aaa gcg tca ttt     1968
Asp Glu Phe Ile Ala Leu His His Leu Ala Asp Thr Lys Ala Ser Phe
                645                 650                 655

gcg gtt cac aac agc ccg ctt tct gaa ggg tcc gtc ctc ggc ttc gaa     2016
Ala Val His Asn Ser Pro Leu Ser Glu Gly Ser Val Leu Gly Phe Glu
            660                 665                 670

tac ggc tat aac gtg tct tcg ccg gaa acg atg gtg atc tgg gaa gcg     2064
Tyr Gly Tyr Asn Val Ser Ser Pro Glu Thr Met Val Ile Trp Glu Ala
        675                 680                 685

cag ttt gga gat ttt gca aat gcg gcg caa gtt tac ttt gac cag ttc     2112
Gln Phe Gly Asp Phe Ala Asn Ala Ala Gln Val Tyr Phe Asp Gln Phe
    690                 695                 700

att tct gca gga aga gcg aag tgg ggt caa aaa tca ggg ctg gtt gtt     2160
Ile Ser Ala Gly Arg Ala Lys Trp Gly Gln Lys Ser Gly Leu Val Val
705                 710                 715                 720

ctc ttg ccg cac ggc tat gaa ggg cag ggg cct gag cat tca agc gga     2208
Leu Leu Pro His Gly Tyr Glu Gly Gln Gly Pro Glu His Ser Ser Gly
                725                 730                 735

aga aca gag cga ttc ctt caa ttg gcg gcg gaa aac aac tgg act gtc     2256
Arg Thr Glu Arg Phe Leu Gln Leu Ala Ala Glu Asn Asn Trp Thr Val
            740                 745                 750

gcc aac ctg acg agc gct gcc caa tac ttt cat att tta aga agg cag     2304
Ala Asn Leu Thr Ser Ala Ala Gln Tyr Phe His Ile Leu Arg Arg Gln
        755                 760                 765

gcg aag atg ctc ctt cgc gag gag atc cgc ccg ctg atc atc atg acg     2352
Ala Lys Met Leu Leu Arg Glu Glu Ile Arg Pro Leu Ile Ile Met Thr
    770                 775                 780

ccg aaa agc ctg ctg aga aat ccg aat acc gtg tca gaa gtg cag gag     2400
Pro Lys Ser Leu Leu Arg Asn Pro Asn Thr Val Ser Glu Val Gln Glu
785                 790                 795                 800

ctc agt aac agc agc ttt aag ccg gtc tat gaa atg tca gga ctt tcc     2448
Leu Ser Asn Ser Ser Phe Lys Pro Val Tyr Glu Met Ser Gly Leu Ser
                805                 810                 815

cat caa tat gac aaa gtg acg cgc ctc gtc ctt tca agc ggc aaa gtt     2496
His Gln Tyr Asp Lys Val Thr Arg Leu Val Leu Ser Ser Gly Lys Val
            820                 825                 830

tcg att gac atc agc gac cat ttc aat aaa atg gaa ggt gaa aag gat     2544
Ser Ile Asp Ile Ser Asp His Phe Asn Lys Met Glu Gly Glu Lys Asp
        835                 840                 845

tgg ctg cac att gca cgg gtt gaa gag ctg tat cct ttc cct gca aag     2592
Trp Leu His Ile Ala Arg Val Glu Glu Leu Tyr Pro Phe Pro Ala Lys

850                 855                 860

cat att aaa gcg atc ttc agc aaa ctt ccg aat ttg gag gag atc gtc      2640
His Ile Lys Ala Ile Phe Ser Lys Leu Pro Asn Leu Glu Glu Ile Val
865                 870                 875                 880

tgg gta cag gaa gaa ccg caa aat atg ggc gca tgg aac tat atc gag      2688
Trp Val Gln Glu Glu Pro Gln Asn Met Gly Ala Trp Asn Tyr Ile Glu
                885                 890                 895

cct tat tta aga gag gta gct cca aag gac gtg aag gtc cgc tat att      2736
Pro Tyr Leu Arg Glu Val Ala Pro Lys Asp Val Lys Val Arg Tyr Ile
            900                 905                 910

ggc aga aga aga cgt tca agt ccg gca gaa ggg gat ccg acg gtt cat      2784
Gly Arg Arg Arg Arg Ser Ser Pro Ala Glu Gly Asp Pro Thr Val His
        915                 920                 925

aaa aag gaa cag gaa cgc att gta tct gat agc ttg act cgc aaa aat      2832
Lys Lys Glu Gln Glu Arg Ile Val Ser Asp Ser Leu Thr Arg Lys Asn
    930                 935                 940

taa ggg                                                              2838


<210>  46
<211>  944
<212>  PRT
<213>  Bacillus licheniformis DSM 13

<400>  46

Met Phe Gln Asn Ser Met Lys Gln Arg Met Thr Trp Glu Glu Phe His
1               5                   10                  15


Gly Pro Asn Leu Gly Tyr Val Leu Glu Leu Tyr Asp Gln Tyr Val Lys
            20                  25                  30


Asp Pro Glu Ser Leu Asp Ala Asp Leu Lys Glu Met Phe Asp Glu Leu
        35                  40                  45


Gly Ala Pro Pro Gly Asp Ile Arg Ala Ala Ser Gln Lys Asn Glu Glu
    50                  55                  60


Ala Asp Phe Thr Ala Gly Ser Ile Gln Lys Ile Ala Ser Ala Val Lys
65                  70                  75                  80


Leu Ala Glu Asp Ile Arg Thr Tyr Gly His Leu Asn Ala Ser Val Asn
                85                  90                  95


Pro Leu Arg Lys Thr Gln Glu Lys Gln Glu Leu Phe Pro Leu Ala Glu
            100                 105                 110

Tyr Gly Leu Thr Glu Gln Asp Val Lys Lys Ile Pro Ala Ser Val Ile
        115                 120                 125

Cys Lys Asp Ala Pro Lys Glu Val Thr Asn Gly Leu Glu Ala Ile Gln
    130                 135                 140

Tyr Leu Arg Asn Thr Tyr Lys Lys Ser Ile Ser Phe Glu Phe Asp His
145                 150                 155                 160

Val His Ile Phe Glu Glu Arg Asn Trp Leu Met Lys Lys Ile Glu Ser
                165                 170                 175

Gly Glu Leu Phe Thr Pro Lys Ser Lys Glu Lys Leu Val Glu Val Leu
            180                 185                 190

Arg Arg Leu Thr Glu Val Glu Ser Leu Glu Gln Phe Leu His Lys Thr
        195                 200                 205

Phe Val Gly Gln Lys Arg Phe Ser Ile Glu Gly Leu Asp Ala Leu Val
    210                 215                 220

Pro Met Leu Asp Asp Ile Ile Ala Lys Ser Val Ser Ala Gly Thr Thr
225                 230                 235                 240

Asn Val Asn Ile Gly Met Ala His Arg Gly Arg Leu Asn Val Leu Ala
                245                 250                 255

His Val Leu Gly Lys Pro Tyr Glu Ile Ile Phe Ser Glu Phe Gln His
            260                 265                 270

Ala Pro Asn Lys Asp Leu Val Pro Ser Glu Gly Ser Thr Gly Ile Asn
        275                 280                 285

Tyr Gly Trp Thr Gly Asp Val Lys Tyr His Leu Gly Ala Asn Arg Gln
    290                 295                 300

Ile Gln Asp Glu His Thr Lys Thr Ala Arg Ile Ala Leu Ala Asn Asn
305                 310                 315                 320

Pro Ser His Leu Glu Phe Ile Asp Pro Ile Val Glu Gly Ser Thr Arg
                325                 330                 335

Ala Ala Gln Glu Thr Arg Thr Glu Ser Gly Tyr Pro Val Gln Asp Val
            340                 345                 350

Lys Lys Ser Met Ala Ile Leu Ile His Gly Asp Ala Ala Phe Pro Gly
        355                 360                 365

Glu Gly Ile Val Ala Glu Thr Leu Asn Leu Ser Gln Leu Lys Gly Tyr
    370                 375                 380

Gln Val Gly Gly Ala Ile His Ile Ile Ala Asn Asn Met Ile Gly Phe
385                 390                 395                 400

Thr Thr Glu Ser Asn Glu Ser Arg Ser Thr Lys Tyr Ala Ser Asp Leu
                405                 410                 415

Ala Lys Gly Phe Glu Ile Pro Ile Val His Val Asn Ala Asp Asp Pro
            420                 425                 430

Glu Ala Cys Leu Ser Ala Val Gln Leu Ala Val Glu Tyr Arg Met Thr
        435                 440                 445

Phe Asn Lys Asp Phe Leu Ile Asp Leu Ile Gly Tyr Arg Arg Phe Gly
    450                 455                 460

His Asn Glu Met Asp Glu Pro Ser Ala Thr Gln Pro Met Leu Tyr Asp
465                 470                 475                 480

Ala Val Arg Lys His Pro Thr Val Lys Asn Ile Phe Ala Glu Lys Leu
                485                 490                 495

Ile His Lys Gly Ile Val Asp Lys Glu Thr Val Gly Lys Ile Lys Asp
            500                 505                 510

Ala Val Gln Lys Arg Leu Glu Glu Ala Tyr Arg Lys Val Pro Ala Lys
        515                 520                 525

Lys Glu Asp Met Thr His Glu Ile Val Leu Pro Glu Pro Val Ser Asn
    530                 535                 540

Gly Phe Pro Asp Val Asp Thr Ser Val Asp Phe Glu Thr Leu Arg Lys
545                 550                 555                 560

Ile Asn Gln Glu Leu Val Ser Trp Pro Glu Asn Phe Asn Val Phe Asp
                565                 570                 575

Lys Leu Lys Arg Ile Leu Glu Arg Arg Ala Lys Ala Phe Glu Asp Asp

580                 585                 590

Arg Lys Val Asp Trp Ser Leu Ala Glu Ala Met Ala Phe Ala Ser Ile
        595                 600                 605

Leu Lys Asp Gly Thr Pro Leu Arg Leu Thr Gly Gln Asp Ser Glu Arg
    610                 615                 620

Gly Thr Phe Ala His Arg Asn Leu Val Leu His Asp Ser Lys Thr Gly
625                 630                 635                 640

Asp Glu Phe Ile Ala Leu His His Leu Ala Asp Thr Lys Ala Ser Phe
                645                 650                 655

Ala Val His Asn Ser Pro Leu Ser Glu Gly Ser Val Leu Gly Phe Glu
            660                 665                 670

Tyr Gly Tyr Asn Val Ser Ser Pro Glu Thr Met Val Ile Trp Glu Ala
        675                 680                 685

Gln Phe Gly Asp Phe Ala Asn Ala Ala Gln Val Tyr Phe Asp Gln Phe
    690                 695                 700

Ile Ser Ala Gly Arg Ala Lys Trp Gly Gln Lys Ser Gly Leu Val Val
705                 710                 715                 720

Leu Leu Pro His Gly Tyr Glu Gly Gln Gly Pro Glu His Ser Ser Gly
                725                 730                 735

Arg Thr Glu Arg Phe Leu Gln Leu Ala Ala Glu Asn Asn Trp Thr Val
            740                 745                 750

Ala Asn Leu Thr Ser Ala Ala Gln Tyr Phe His Ile Leu Arg Arg Gln
        755                 760                 765

Ala Lys Met Leu Leu Arg Glu Glu Ile Arg Pro Leu Ile Ile Met Thr
    770                 775                 780

Pro Lys Ser Leu Leu Arg Asn Pro Asn Thr Val Ser Glu Val Gln Glu
785                 790                 795                 800

Leu Ser Asn Ser Ser Phe Lys Pro Val Tyr Glu Met Ser Gly Leu Ser
                805                 810                 815

His Gln Tyr Asp Lys Val Thr Arg Leu Val Leu Ser Ser Gly Lys Val
            820                 825                 830

Ser Ile Asp Ile Ser Asp His Phe Asn Lys Met Glu Gly Glu Lys Asp
        835                 840                 845

Trp Leu His Ile Ala Arg Val Glu Glu Leu Tyr Pro Phe Pro Ala Lys
    850                 855                 860

His Ile Lys Ala Ile Phe Ser Lys Leu Pro Asn Leu Glu Glu Ile Val
865                 870                 875                 880

Trp Val Gln Glu Glu Pro Gln Asn Met Gly Ala Trp Asn Tyr Ile Glu
                885                 890                 895

Pro Tyr Leu Arg Glu Val Ala Pro Lys Asp Val Lys Val Arg Tyr Ile
            900                 905                 910

Gly Arg Arg Arg Arg Ser Ser Pro Ala Glu Gly Asp Pro Thr Val His
        915                 920                 925

Lys Lys Glu Gln Glu Arg Ile Val Ser Asp Ser Leu Thr Arg Lys Asn
    930                 935                 940

<210>  47
<211>  1545
<212>  DNA
<213>  Bacillus licheniformis DSM 13

<220>
<221>  gene
<222>  (1)..(1545)
<223>  probable acid-CoA ligase (E.C. 6.2.1.-), yhfL

<220>
<221>  CDS
<222>  (1)..(1542)
<223>

<400>  47
atg aat tta gtt tca aaa tta ggg gaa aca gct caa tca aag cct gac       48
Met Asn Leu Val Ser Lys Leu Gly Glu Thr Ala Gln Ser Lys Pro Asp
1               5                   10                  15

aga acc gct tat att ttt gga gag caa acg gaa aca tac ggg gga ttg       96
Arg Thr Ala Tyr Ile Phe Gly Glu Gln Thr Glu Thr Tyr Gly Gly Leu
            20                  25                  30

cag cag aaa att gat tgc ttt gcc gaa ggg ctg cgc gaa atc ggg gtg      144

Gln Gln Lys Ile Asp Cys Phe Ala Glu Gly Leu Arg Glu Ile Gly Val
        35                  40                  45

gaa aag gga gat cat gtc gct ttg cta ctc ggc aat aca ccg cat ttt      192
Glu Lys Gly Asp His Val Ala Leu Leu Leu Gly Asn Thr Pro His Phe
    50                  55                  60

gtg att gcg ttt tac ggc gcg ctg aag gcc ggg gct gtc gtc ata ccg      240
Val Ile Ala Phe Tyr Gly Ala Leu Lys Ala Gly Ala Val Val Ile Pro
65                  70                  75                  80

atc aat ccg gcc tat acg ccg act gag atc ggc tat atg ctg aca aat      288
Ile Asn Pro Ala Tyr Thr Pro Thr Glu Ile Gly Tyr Met Leu Thr Asn
                85                  90                  95

ggc gat gca aaa gtg atc gtg gct ctg gga cag ctg ctt ccg ctt tac      336
Gly Asp Ala Lys Val Ile Val Ala Leu Gly Gln Leu Leu Pro Leu Tyr
            100                 105                 110

gaa aag gtg cat gaa tcc ctt ccg aaa gtc ggc tgc gtc gtg ctt tgc      384
Glu Lys Val His Glu Ser Leu Pro Lys Val Gly Cys Val Val Leu Cys
        115                 120                 125

gaa act gga gag ccg ctt cag gaa ccg gaa aac aca gag gtt aag atg      432
Glu Thr Gly Glu Pro Leu Gln Glu Pro Glu Asn Thr Glu Val Lys Met
    130                 135                 140

aaa ttg aaa tcg ttt aca agc att atg aaa cct cct gtc cgg ccg ttt      480
Lys Leu Lys Ser Phe Thr Ser Ile Met Lys Pro Pro Val Arg Pro Phe
145                 150                 155                 160

cct gaa atc gat gac gaa gat acg gcc gcc atc ctc tat acg tca ggc      528
Pro Glu Ile Asp Asp Glu Asp Thr Ala Ala Ile Leu Tyr Thr Ser Gly
                165                 170                 175

acc acg gga aga cca aaa ggg gcg atg ctc aca cat caa aat cta ttt      576
Thr Thr Gly Arg Pro Lys Gly Ala Met Leu Thr His Gln Asn Leu Phe
            180                 185                 190

tcg aat gca aat gat aca gcc cgc tat ctg aca atg aat gaa tct gac      624
Ser Asn Ala Asn Asp Thr Ala Arg Tyr Leu Thr Met Asn Glu Ser Asp
        195                 200                 205

ctt gtc gtc gcc gcc ctg ccg atg ttc cac gtt ttt tgt tta acg gtc      672
Leu Val Val Ala Ala Leu Pro Met Phe His Val Phe Cys Leu Thr Val
    210                 215                 220

tgc atg aat gca ccg ctc atg aac ggc gca gcg att ttg atc gtg ccg      720
Cys Met Asn Ala Pro Leu Met Asn Gly Ala Ala Ile Leu Ile Val Pro
225                 230                 235                 240

aaa ttc agt ccc gcc gag gtt ttc aag ctg att aaa aag cat cag gcg      768
Lys Phe Ser Pro Ala Glu Val Phe Lys Leu Ile Lys Lys His Gln Ala
                245                 250                 255

acg atc ttc tca ggc gtt ccg aca atg tac aat tac ctg tac cag tat      816
Thr Ile Phe Ser Gly Val Pro Thr Met Tyr Asn Tyr Leu Tyr Gln Tyr
            260                 265                 270

gaa gga gcg gat gaa aca ggc ttt cgg tcc atc agg ctt tgc atc tca        864
Glu Gly Ala Asp Glu Thr Gly Phe Arg Ser Ile Arg Leu Cys Ile Ser
        275                 280                 285

ggc gga gcg gcc atg cct gtc gct ctc ctg aaa agc ttt gaa gaa agg        912
Gly Gly Ala Ala Met Pro Val Ala Leu Leu Lys Ser Phe Glu Glu Arg
    290                 295                 300

ttc ggc gtc ctc gtt tta gaa ggc tac ggc ttg tcg gag gct tct cct        960
Phe Gly Val Leu Val Leu Glu Gly Tyr Gly Leu Ser Glu Ala Ser Pro
305                 310                 315                 320

gtt aca tgc ttt aac ccg ttc agc acc ggc cgc aag cca gga tcg atc       1008
Val Thr Cys Phe Asn Pro Phe Ser Thr Gly Arg Lys Pro Gly Ser Ile
                325                 330                 335

ggc acg aac att ctc aat gtg aaa aac aaa gtc gtc aat gaa ctc ggg       1056
Gly Thr Asn Ile Leu Asn Val Lys Asn Lys Val Val Asn Glu Leu Gly
            340                 345                 350

gaa gag ctg cct gcc ggc caa gtc ggc gag ctg atc gtt aaa ggg cct       1104
Glu Glu Leu Pro Ala Gly Gln Val Gly Glu Leu Ile Val Lys Gly Pro
        355                 360                 365

aat gtc atg aaa ggg tac tac aaa atg ccg gat gag acg gcc cat acg       1152
Asn Val Met Lys Gly Tyr Tyr Lys Met Pro Asp Glu Thr Ala His Thr
    370                 375                 380

ata aaa gac gga tgg ctg tat aca ggt gat ttg gca aaa cgg gat gaa       1200
Ile Lys Asp Gly Trp Leu Tyr Thr Gly Asp Leu Ala Lys Arg Asp Glu
385                 390                 395                 400

gac ggt tat ttt tat atc gtc gac aga aaa aaa gat atg att atc gtc       1248
Asp Gly Tyr Phe Tyr Ile Val Asp Arg Lys Lys Asp Met Ile Ile Val
                405                 410                 415

ggc ggc tat aat gtc tat ccg agg gaa atc gaa gag gtg ctc tac ctc       1296
Gly Gly Tyr Asn Val Tyr Pro Arg Glu Ile Glu Glu Val Leu Tyr Leu
            420                 425                 430

cat cca aaa atc gca gaa gcg gtt gtg atc gga gtg ccc gat ccg aat       1344
His Pro Lys Ile Ala Glu Ala Val Val Ile Gly Val Pro Asp Pro Asn
        435                 440                 445

acg gga gaa gcc gtt cat tgc tat gtc gtt ccg aag gat aaa acg ctg       1392
Thr Gly Glu Ala Val His Cys Tyr Val Val Pro Lys Asp Lys Thr Leu
    450                 455                 460

aca gag gag gat gtc ttg tcg cac tgc aaa aag cat ctt gcc aaa tac       1440
Thr Glu Glu Asp Val Leu Ser His Cys Lys Lys His Leu Ala Lys Tyr
465                 470                 475                 480

aag cgc ccg tcg gcg atc gtt ttc atg gat gaa att ccg aaa aac tcg       1488
Lys Arg Pro Ser Ala Ile Val Phe Met Asp Glu Ile Pro Lys Asn Ser
                485                 490                 495

acc ggc aaa att tta agg cgc gct tta aaa gac att ctg aca aac aaa       1536
Thr Gly Lys Ile Leu Arg Arg Ala Leu Lys Asp Ile Leu Thr Asn Lys
            500                 505                 510

tct tga ccc                                                          1545
Ser

<210>  48
<211>  513
<212>  PRT
<213>  Bacillus licheniformis DSM 13

<400>  48

Met Asn Leu Val Ser Lys Leu Gly Glu Thr Ala Gln Ser Lys Pro Asp
1               5                   10                  15

Arg Thr Ala Tyr Ile Phe Gly Glu Gln Thr Glu Thr Tyr Gly Gly Leu
            20                  25                  30

Gln Gln Lys Ile Asp Cys Phe Ala Glu Gly Leu Arg Glu Ile Gly Val
        35                  40                  45

Glu Lys Gly Asp His Val Ala Leu Leu Leu Gly Asn Thr Pro His Phe
    50                  55                  60

Val Ile Ala Phe Tyr Gly Ala Leu Lys Ala Gly Ala Val Val Ile Pro
65                  70                  75                  80

Ile Asn Pro Ala Tyr Thr Pro Thr Glu Ile Gly Tyr Met Leu Thr Asn
                85                  90                  95

Gly Asp Ala Lys Val Ile Val Ala Leu Gly Gln Leu Leu Pro Leu Tyr
            100                 105                 110

Glu Lys Val His Glu Ser Leu Pro Lys Val Gly Cys Val Val Leu Cys
        115                 120                 125

Glu Thr Gly Glu Pro Leu Gln Glu Pro Glu Asn Thr Glu Val Lys Met
    130                 135                 140

Lys Leu Lys Ser Phe Thr Ser Ile Met Lys Pro Pro Val Arg Pro Phe
145                 150                 155                 160

Pro Glu Ile Asp Asp Glu Asp Thr Ala Ala Ile Leu Tyr Thr Ser Gly
                165                 170                 175

Thr Thr Gly Arg Pro Lys Gly Ala Met Leu Thr His Gln Asn Leu Phe
            180                 185                 190

Ser Asn Ala Asn Asp Thr Ala Arg Tyr Leu Thr Met Asn Glu Ser Asp
        195                 200                 205

Leu Val Val Ala Ala Leu Pro Met Phe His Val Phe Cys Leu Thr Val
    210                 215                 220

Cys Met Asn Ala Pro Leu Met Asn Gly Ala Ala Ile Leu Ile Val Pro
225                 230                 235                 240

Lys Phe Ser Pro Ala Glu Val Phe Lys Leu Ile Lys Lys His Gln Ala
                245                 250                 255

Thr Ile Phe Ser Gly Val Pro Thr Met Tyr Asn Tyr Leu Tyr Gln Tyr
            260                 265                 270

Glu Gly Ala Asp Glu Thr Gly Phe Arg Ser Ile Arg Leu Cys Ile Ser
        275                 280                 285

Gly Gly Ala Ala Met Pro Val Ala Leu Leu Lys Ser Phe Glu Glu Arg
    290                 295                 300

Phe Gly Val Leu Val Leu Glu Gly Tyr Gly Leu Ser Glu Ala Ser Pro
305                 310                 315                 320

Val Thr Cys Phe Asn Pro Phe Ser Thr Gly Arg Lys Pro Gly Ser Ile
                325                 330                 335

Gly Thr Asn Ile Leu Asn Val Lys Asn Lys Val Val Asn Glu Leu Gly
            340                 345                 350

Glu Glu Leu Pro Ala Gly Gln Val Gly Glu Leu Ile Val Lys Gly Pro
        355                 360                 365

Asn Val Met Lys Gly Tyr Tyr Lys Met Pro Asp Glu Thr Ala His Thr
    370                 375                 380

Ile Lys Asp Gly Trp Leu Tyr Thr Gly Asp Leu Ala Lys Arg Asp Glu
385                 390                 395                 400

Asp Gly Tyr Phe Tyr Ile Val Asp Arg Lys Lys Asp Met Ile Ile Val
                405                 410                 415

Gly Gly Tyr Asn Val Tyr Pro Arg Glu Ile Glu Glu Val Leu Tyr Leu

420                 425                 430

His Pro Lys Ile Ala Glu Ala Val Val Ile Gly Val Pro Asp Pro Asn
        435                 440                 445

Thr Gly Glu Ala Val His Cys Tyr Val Val Pro Lys Asp Lys Thr Leu
    450                 455                 460

Thr Glu Glu Asp Val Leu Ser His Cys Lys Lys His Leu Ala Lys Tyr
465                 470                 475                 480

Lys Arg Pro Ser Ala Ile Val Phe Met Asp Glu Ile Pro Lys Asn Ser
                485                 490                 495

Thr Gly Lys Ile Leu Arg Arg Ala Leu Lys Asp Ile Leu Thr Asn Lys
            500                 505                 510

Ser

<210>  49
<211>  876
<212>  DNA
<213>  Bacillus licheniformis DSM 13

<220>
<221>  gene
<222>  (1)..(876)
<223>  Agmatinase (E.C. 3.5.1.11), ywhG

<220>
<221>  CDS
<222>  (1)..(873)
<223>

<400>  49
atg aga ttt gat gaa gca tat tcc ggc aag gtg ttt atc gca agc cgc       48
Met Arg Phe Asp Glu Ala Tyr Ser Gly Lys Val Phe Ile Ala Ser Arg
1               5                   10                  15

ccc gat tgg gaa gag gcg gac gcc atc ctt tac ggc atg ccg atg gat       96
Pro Asp Trp Glu Glu Ala Asp Ala Ile Leu Tyr Gly Met Pro Met Asp
            20                  25                  30

tgg acg gtc agc tac cgt ccc ggc tcc cgc ttc ggt ccg gcg aga atc      144
Trp Thr Val Ser Tyr Arg Pro Gly Ser Arg Phe Gly Pro Ala Arg Ile
        35                  40                  45

cgc gag gtg tcc atc gga ctt gaa gaa tac agc cct tat ttg gac agg      192
Arg Glu Val Ser Ile Gly Leu Glu Glu Tyr Ser Pro Tyr Leu Asp Arg

50                  55                  60

gag ctg gaa gag gtt cat ttc ttt gac gcc ggc gac atc ccg ctg cct      240
Glu Leu Glu Glu Val His Phe Phe Asp Ala Gly Asp Ile Pro Leu Pro
65                  70                  75                  80

ttc ggg aac ccg cag aag agc ctc gac atg att gaa gaa tat gtc gac      288
Phe Gly Asn Pro Gln Lys Ser Leu Asp Met Ile Glu Glu Tyr Val Asp
                85                  90                  95

agc att tta gat aaa gga aaa ttc ccg ctc ggt atg ggg gga gag cat      336
Ser Ile Leu Asp Lys Gly Lys Phe Pro Leu Gly Met Gly Gly Glu His
            100                 105                 110

ctc gtt tca tgg ccg gtc att aaa gcg atg tac aaa aaa tat ccc gat      384
Leu Val Ser Trp Pro Val Ile Lys Ala Met Tyr Lys Lys Tyr Pro Asp
        115                 120                 125

ctt gcc atc atc cat atg gat gcg cac aca gat ctc cgc gtc gac tat      432
Leu Ala Ile Ile His Met Asp Ala His Thr Asp Leu Arg Val Asp Tyr
    130                 135                 140

gaa gga gag ccg ctc tcg cat tcg acg ccg atc cgt aaa gcc gcg gaa      480
Glu Gly Glu Pro Leu Ser His Ser Thr Pro Ile Arg Lys Ala Ala Glu
145                 150                 155                 160

ctg atc gga ccc ggc aat gtc tat tca ttc ggc atc cgc tcc ggc atg      528
Leu Ile Gly Pro Gly Asn Val Tyr Ser Phe Gly Ile Arg Ser Gly Met
                165                 170                 175

aaa gaa gaa ttt gaa tgg gca aaa gaa aac ggc atg cac att tct aaa      576
Lys Glu Glu Phe Glu Trp Ala Lys Glu Asn Gly Met His Ile Ser Lys
            180                 185                 190

ttt gaa gtg ctt gag ccg ctt aaa gcc gtc ctg ccg aaa ctc gcg ggg      624
Phe Glu Val Leu Glu Pro Leu Lys Ala Val Leu Pro Lys Leu Ala Gly
        195                 200                 205

cgt ccg gtt tat gtg acg atc gac atc gat gtc tta gat ccc gcg cat      672
Arg Pro Val Tyr Val Thr Ile Asp Ile Asp Val Leu Asp Pro Ala His
    210                 215                 220

gcg ccg gga acc ggt acg gtt gac gcc gga ggc atc aca tcc aaa gag      720
Ala Pro Gly Thr Gly Thr Val Asp Ala Gly Gly Ile Thr Ser Lys Glu
225                 230                 235                 240

ctg ctc gct tcg att cac gaa atc gcc cgc tca gac gtc aat gtc gtt      768
Leu Leu Ala Ser Ile His Glu Ile Ala Arg Ser Asp Val Asn Val Val
                245                 250                 255

gga gga gac ctt gtc gaa gtc gct cct gtc tat gac cat tcg gaa caa      816
Gly Gly Asp Leu Val Glu Val Ala Pro Val Tyr Asp His Ser Glu Gln
            260                 265                 270

acc gca aat acg gca agc aag ctg att cgc gaa atg ctg ctc ggc tgg      864
Thr Ala Asn Thr Ala Ser Lys Leu Ile Arg Glu Met Leu Leu Gly Trp
        275                 280                 285

gtg aaa taa aac                                                      876

Val Lys
    290


<210>  50
<211>  290
<212>  PRT
<213>  Bacillus licheniformis DSM 13

<400>  50

Met Arg Phe Asp Glu Ala Tyr Ser Gly Lys Val Phe Ile Ala Ser Arg
1               5                   10                  15

Pro Asp Trp Glu Glu Ala Asp Ala Ile Leu Tyr Gly Met Pro Met Asp
            20                  25                  30

Trp Thr Val Ser Tyr Arg Pro Gly Ser Arg Phe Gly Pro Ala Arg Ile
        35                  40                  45

Arg Glu Val Ser Ile Gly Leu Glu Glu Tyr Ser Pro Tyr Leu Asp Arg
    50                  55                  60

Glu Leu Glu Glu Val His Phe Phe Asp Ala Gly Asp Ile Pro Leu Pro
65                  70                  75                  80

Phe Gly Asn Pro Gln Lys Ser Leu Asp Met Ile Glu Glu Tyr Val Asp
                85                  90                  95

Ser Ile Leu Asp Lys Gly Lys Phe Pro Leu Gly Met Gly Gly Glu His
            100                 105                 110

Leu Val Ser Trp Pro Val Ile Lys Ala Met Tyr Lys Lys Tyr Pro Asp
        115                 120                 125

Leu Ala Ile Ile His Met Asp Ala His Thr Asp Leu Arg Val Asp Tyr
    130                 135                 140

Glu Gly Glu Pro Leu Ser His Ser Thr Pro Ile Arg Lys Ala Ala Glu
145                 150                 155                 160

Leu Ile Gly Pro Gly Asn Val Tyr Ser Phe Gly Ile Arg Ser Gly Met
                165                 170                 175

Lys Glu Glu Phe Glu Trp Ala Lys Glu Asn Gly Met His Ile Ser Lys
            180                 185                 190

Phe Glu Val Leu Glu Pro Leu Lys Ala Val Leu Pro Lys Leu Ala Gly
        195                 200                 205

Arg Pro Val Tyr Val Thr Ile Asp Ile Asp Val Leu Asp Pro Ala His
    210                 215                 220

Ala Pro Gly Thr Gly Thr Val Asp Ala Gly Gly Ile Thr Ser Lys Glu
225                 230                 235                 240

Leu Leu Ala Ser Ile His Glu Ile Ala Arg Ser Asp Val Asn Val Val
                245                 250                 255

Gly Gly Asp Leu Val Glu Val Ala Pro Val Tyr Asp His Ser Glu Gln
            260                 265                 270

Thr Ala Asn Thr Ala Ser Lys Leu Ile Arg Glu Met Leu Leu Gly Trp
        275                 280                 285

Val Lys
    290

## Beschreibung

Die vorliegende Erfindung betrifft ein Gen von *B. licheniformis* und ein davon abgeleitetes Genprodukt, das auf einem Stoffwechselweg an der Bildung von Geruchsstoffen beteiligt ist, sowie biotechnologische Produktionsverfahren, die insofern verbessert sind, als aufgrund der Identifizierung dieses Gens die Bildung dieser Geruchsstoffe verringert werden kann.

Die vorliegende Erfindung liegt auf dem Gebiet der Biotechnologie, insbesondere der Herstellung von Wertstoffen durch Fermentation von Mikroorganismen, die zur Bildung der interessierenden Wertstoffe in der Lage sind. Hierzu zählt beispielsweise die Herstellung niedermolekularer Verbindungen, etwa von Nahrungsmittelergänzungsstoffen oder pharmazeutisch relevanten Verbindungen, oder von Proteinen, für welche aufgrund ihrer Diversität wiederum ein großes technisches Einsatzgebiet besteht. Im ersten Fall werden die Stoffwechseleigenschaften der betreffenden Mikroorganismen zur Herstellung der Wertstoffe ausgenutzt und/oder verändert; im zweiten Fall werden Zellen eingesetzt, die die Gene der interessierenden Proteine exprimieren. In beiden Fällen handelt es sich zumeist also um gentechnisch veränderte Organismen (GVO).

Zur Fermentation von Mikroorganismen besteht ein reichhaltiger Stand der Technik, insbesondere auch im großtechnischen Maßstab; er reicht von der Optimierung der betreffenden Stämme hinsichtlich der Bildungsrate und der Nährstoffausnutzung über die technische Gestaltung der Fermenter bis hin zur Gewinnnung der Wertstoffe aus den betreffenden Zellen selbst und/oder dem Fermentationsmedium. Hierfür kommen sowohl genetische und mikrobiologische als auch verfahrenstechnische und biochemische Ansätze zu tragen. Ziel der vorliegenden Erfindung ist es, diesen Prozeß hinsichtlich einer häufigen, den eigentlichen Fermentationschritt beeinträchtigenden Eigenschaft der eingesetzten Mikroorganismen zu verbessern, und zwar auf der Ebene der genetischen Eigenschaften der eingesetzten Stämme.

Für die großtechnische, biotechnologische Produktion werden die betreffenden Mikroorganismen in Fermentern kultiviert, die ihren Stoffwechseleigenschaften entsprechend ausgelegt sind. Während der Kultivierung verstoffwechseln sie das angebotene Substrat und bilden neben dem eigentlichen Produkt üblicherweise eine Vielzahl weiterer Substanzen, an denen in der Regel kein Interesse besteht und/oder die zu unerwünschten Begleiterscheinungen führen können.

Hierzu gehören Geruchs- und/oder Giftstoffe, die lästig und/oder schädlich sind und bereits während der Fermentation über die Abluft ausgetragen werden und/oder bei der nachfolgenden Aufarbeitung des Wertstoffs nur unvollständig abgetrennt werden und somit die Qualität des Produkts beeinträchtigen. Die begleitenden Geruchs- und/oder Giftstoffe belasten somit zum einen den Produktionsprozeß, das heißt das beteiligte Personal und die Umgebung der Anlage. Zum anderen kann das Nichterreichen einer erwünschten Qualität (Spezifikation) des Produkts dazu führen, daß es für das erhoffte Einsatzgebiet (beispielsweise der Lebensmittelherstellung) nicht zur Verfügung steht, was einen erheblichen wirtschaftlichen Nachteil bedeutet. Umgekehrt könnten durch Verringerung der Bildung von Geruchs- und/oder Giftsstoffen der Arbeits- und der Umweltschutz gesteigert und für das Produkt zusätzliche Einsatzgebiete und Absatzmärkte erschlossen werden.

Häufig bei der Fermentation von Mikroorganismen festgestellte Gerüche werden durch kleine organische Moleküle aus den Klassen der flüchtigen, verzweigten und unverzeigten Fettsäuren, Alkoholen und Diaminen hervorgerufen. Dazu gehören Isovaleriansäure, 2-Methylbuttersäure, Isobuttersäure aus der Klasse der verzweigten Fettsäuren, Buttersäure, Propylsäure (unverzweigte Fettsäuren), Butanol (Alkohol), Cadaverin und Putrescin (Diamine).

Ein Teil dieser flüchtigen Substanzen ist zudem toxisch für Mensch und Tier, zum Beispiel Cadaverin und Putrescin, die auch als Leichengifte bekannt sind. Sie können deshalb nicht nur als Geruchsstoffe, sondern je nach Konzentration und Einwirkzeit für den betreffenden Organismus bereits als Giftstoffe definiert werden.

Bereits zum gegenwärtigen Zeitpunkt bemüht man sich, derartige Verbindungen aus den Fermentationsprodukten nachträglich zu entfernen. Zu diesem Zweck enthält die übliche Aufarbeitung der gebildeten Wertstoffe neben Schritten zur Entfernung von Zelltrümmern und höhermolekularen Verbindungen auch zusätzliche Prozeßschritte, die als Desodorierung bezeichnet werden. Hinzu kommen in der Regel Filtrationen, Fällungsschritte und/oder Chromatographieschritte, die jeweils zu einem gewissen Anteil auch zur Desodorierung beitragen. Dennoch führen all diese zur Abtrennung durchgeführten Schritte nur zu einer, nach den oben genannten Maßstäben unzureichenden Reinheit.

Die Abluft der Fermenter wird ebenfalls kontrolliert, um die Belastung während des Produktionsprozesses geringzuhalten.

Dennoch wäre es wünschenswert, eine möglichst ursächliche Geruchsbekämpfung vorzunehmen, das heißt zu verhindern, daß die betreffenden Stoffe überhaupt erst entstehen. Denn damit könnte zum einen die Zahl der nachfolgenden Reinigungs- und Aufarbeitungsschritte geringgehalten werden, was deshalb vorteilhaft erscheint, weil sie jeweils eine physikochemische Belastung für das erwünschte Produkt darstellen und die Ausbeute verringern. Insgesamt würde also eine bessere Produktqualität erhalten. Zum anderen würden die Produktionsbedingungen an sich verbessert, und die Anlagen zur Filtration der Fermenterabluft könnten einfacher gehalten werden. Eine derartige ursächliche Geruchsbekämpfung würde, wenn dadurch die Eigenschaften des Mikroorganismus selbst verändert würden, auch dessen Verträglichkeit für weitere Arbeiten an diesem Mikroorganismus erhöhen.

Es stellte sich somit die Aufgabe, die bei der Fermentation von Mikroorganismen, insbesondere grampositiven Bakterien der Spezies *Bacillus* auftretende und auf diese selbst zurückzuführende Bildung unangenehmer Gerüche und/oder giftiger Verbindungen zu verringern. Vorzugsweise sollte dies auf genetischer Ebene erfolgen, um geruchs- und/oder giftstoffärmere Mikroorganismen zu erhalten. In Teilaufgaben bedeutete dies, hierfür relevante Stoffwechselwege zu identifizieren, Gene zu finden, die für Proteine und/oder Enzyme codieren, welche auf diesen Wegen liegende Reaktionen katalysieren und sich als mögliche Ansatzpunkte zur Lösung der Aufgabe eignen, und über Identifizierung der zugehörigen Nukleotidsequenzen Werkzeuge für die gewünschte gentechnische Modifizierung in die Hand zu bekommen und entsprechenden Anwendungen zur Verfügung zu stellen.

Zur Lösung dieser Aufgabe wurde folgender Stoffwechselweg identifiziert:
der Stoffwechselweg zur Synthese von Butanol und/oder Buttersäure (als Teil des Buttersäure-Metabolismus).

Sodann wurde folgendes Gen gefunden, das für ein Protein und/oder Enzym codiert, welches auf diesem Weg liegende Reaktionen katalysiert und sich als Ansatzpunkt für erfindungsgemäße biotechnologische Produktionsverfahren eignet:
Phosphat-Butyryl-Transferase (E.C. 2.3.1.19).

Schließlich wurden über Sequenzierung zugehöriger Gene in *B. licheniformis* DSM 13 für dieses Protein/Enzym codierende Nukleotid- und Aminosäuresequenzen vollständig bestimmt und somit für die erwünschte Modifizierung der interessierenden Mikroorganismen zur Verfügung gestellt. Sie sind im Sequenzprotokoll ("Sequence listing") zur vorliegenden Anmeldung zusammengestellt. Hierbei handelt es sich um folgende Nukleinsäuren (ungeradzahlige Nummern) und davon abgeleitete Aminosäuresequenzen von Enzymen oder Proteinen als Teilen von solchen Enzymen, die aus mehreren Untereinheiten bestehen (jeweils nachfolgende geradzahlige Nummern): wahrscheinliche Phosphat-Butyryl-Transferase (E.C. 2.3.1.19), definiert über SEQ ID NO. 31 und 32.

Diese werden mit der vorliegenden Anmeldung zur Verfügung gestellt.

Die gestellte Aufgabe wird somit durch die im Sequenzprotokoll angegebene, aus *B. licheniformis* DSM 13 erhältliche Nukleinsäure der SEQ ID NO. 31 einschließlich eines jeweils entsprechenden weiter unten definierten Homologiebereichs gelöst, über den eine Abgrenzung zu den im Stand der Technik beschriebenen Sequenzen erfolgt. Ebenso wird sie von dem hiervon abgeleiteten Genprodukt der SEQ ID NO. 32 gelöst, wiederum einschließlich eines entsprechenden weiter unten definierten Homologiebereichs. Die jeweiligen nächstähnlichen im Stand der Technik beschriebenen Nukleinsäure- und Aminosäuresequenzen sind unter Verweis auf die betreffenden Datenbankeintragungen in Beispiel 2 (Tabelle 1) zusammengestellt. Aufgrund dieser Angaben wurden die jeweils beanspruchten Homologiebereiche definiert. Vorzugsweise sind erfindungsgemäße Lösungen der gestellten Aufgabe jeweils solche Nukleinsäuren und Proteine, die tatsächlich aus Mikroorganismen stammen.

Weitere Lösungen stellen Fermentationsverfahren dar, in denen der Stoffwechselweg zur Synthese von Butanol und/oder Buttersäure (als Teil des Buttersäure-Metabolismus) funktionell inaktiviert ist, vorzugsweise über die oben genannten, auf diesen Wegen aktiven Enzyme/Proteine und besonders bevorzugt über die erfindungsgemäß zur Verfügung gestellten Nukleotidsequenzen. Letztere können in an sich bekannter und im Stand der Technik etablierter Weise genutzt werden, beispielsweise um Knock-out-Konstrukte zu erzeugen und über Vektoren in die Wirtszellen einzubringen, so daß eine Gendisruption erfolgt.

Weitere Lösungen stellen entsprechend modifizierte, vor allem für die technische Produktion relevante Mikroorganismen dar, alle Fermentationsverfahren, bei denen diese eingesetzt werden, und hierunter insbesondere solche, die der Produktion von Wertstoffen dienen.

Zudem stehen diese Genprodukte aufgrund der vorliegenden Erfindung für Reaktionsansätze oder Verfahren entsprechend ihrer jeweiligen biochemischen Eigenschaften zur Verfügung, worunter insbesondere die Synthese von Butanol und/oder Buttersäure zu verstehen ist.

Mit der vorliegenden Erfindung wird, zumindest was diesen wichtigen Stoffwechselweg angeht, eine ursächliche Geruchsbekämpfung ermöglicht. Denn durch Ausschalten des erkannten Stoffwechselwegs über die beteiligten Proteine mithilfe der für diese Proteine codierenden Nukleinsäuren kann weitgehend verhindert werden, daß die betreffenden Stoffe überhaupt erst entstehen. Damit kann zum einen die Zahl der nachfolgenden Reinigungs- und Aufarbeitungsschritte geringgehalten werden, was deshalb vorteilhaft ist, weil sie jeweils eine physikochemische Belastung für das erwünschte Produkt darstellen und die Ausbeute verringern; insgesamt wird also die Produktqualität verbessert. Zum anderen werden die Produktionsbedingungen an sich verbessert, und die Anlagen zur Filtration der Fermenterabluft können einfacher gehalten werden. Diese ursächliche Geruchsbekämpfung wirkt sich, weil sie auf genetischer Ebene ansetzt, auf die Eigenschaften des jeweiligen Mikroorganismus selbst aus, wodurch dessen Verträglichkeit für weitere Arbeiten an diesem Mikroorganismus erhöht wird.

Insbesondere die großtechnische Fermentation wird dadurch verbessert, was auch zu einer Kostenreduktion der Fermentationsprodukte führen sollte.

Zudem stehen damit die identifizierten Gene und Genprodukte für vielfältige Anwendungen zur Verfügung, zum Beispiel zur chemischen und/oder wenigstens zum Teil biokatalysierten Synthese der betreffenden Verbindungen.

Wie in den Beispielen zur vorliegenden Anmeldung beschrieben, konnten über eine Sequenzierung der genomischen DNA von *B. licheniformis* DSM 13, dem von der Deutschen Sammlung von Mikroorganismen und Zellkulturen GmbH, Mascheroder Weg 1 b, 38124 Braunschweig (http://www.dsmz.de) erhältlichen Referenzstamm für diese Spezies die genannten 25 neuen Gene identifiziert werden. Dabei handelt es sich um solche, die für Enzyme oder Enzymuntereinheiten codieren, welche an den hier vorgestellten Reaktionen zur Synthese von Geruchsstoffen beteiligt sind.

Die hierzu im Stand der Technik bekannten jeweils nächstähnlichen Gene und zugehörigen Proteine weisen die in Beispiel 2 (Tabelle 1) zur vorliegenden Anmeldung angegebenen Sequenzhomologien auf. Hierüber definiert sich der mit der vorliegenden Anmeldung jeweils abgedeckte Schutzbereich. Dementsprechend stellen alle folgenden Nukleinsäuren und Proteine prinzipiell gleichwertige Ausführungsformen der vorliegenden Erfindung dar:
- Nukleinsäure, codierend für ein an der Synthese von Butanol und/oder Buttersäure beteiligtes Genprodukt (wahrscheinliche Phosphat-Butyryl-Transferase; E.C. 2.3.1.19), mit einer Nukleotidsequenz, die zu der in SEQ ID NO. 31 angegebenen Nukleotidsequenz mindestens 51% Identität und zunehmend bevorzugt mindestens 55%, 60%, 65%, 70%, 75%, 80%, 85%, 90%, 92%, 94%, 96%, 97%, 98%, 99% und besonders bevorzugt 100% Identität aufweist;
- An der Synthese von Butanol und/oder Buttersäure beteiligtes Genprodukt (wahrscheinliche Phosphat-Butyryl-Transferase; E.C. 2.3.1.19), mit einer Aminosäuresequenz, die zu der in SEQ ID NO. 32 angegebenen Aminosäuresequenz mindestens 69% Identität und zunehmend bevorzugt mindestens 70%, 75%, 80%, 85%, 90%, 92%, 94%, 96%, 97%, 98%, 99% und besonders bevorzugt 100% Identität aufweist.

Im Zusammenhang mit der vorliegenden Anmeldung bedeutet ein Ausdruck der Form "mindestens X%" "X% bis 100%, einschließlich der Eckwerte X und 100 und aller ganzzahligen und nicht ganzzahligen Prozentwerte dazwischen".

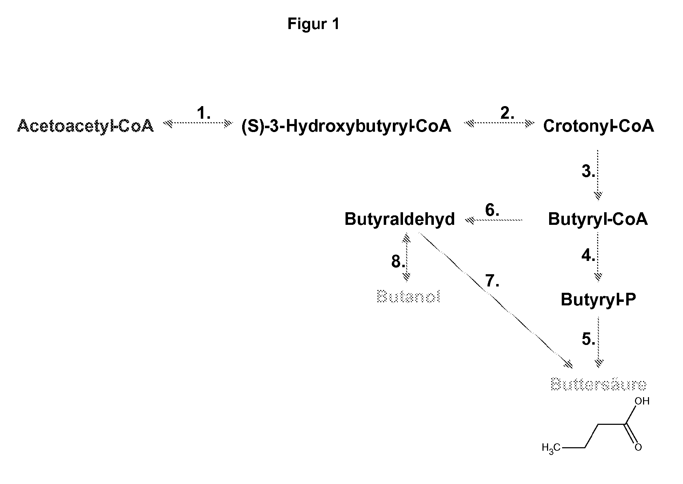

Die Bezeichnungen der jeweiligen Enzyme richten sich nach den konkreten, durch sie katalysierten Reaktionen, wie sie beispielsweise in der Figur 1 dargestellt sind. (Detailliertere Erläuterungen der Figuren und der betreffenden Stoffwechselwege folgen weiter unten.) So ist es auch möglich, daß ein einzelnes Enzym zwei Reaktionen zu katalysieren vermag, die chemisch nahezu identisch sind, anhand des jeweiligen Substrats hier aber verschiedenen Wegen zugeordnet sind. Damit kann auch eine unterschiedliche Enzymklassifikation (E.C.-Nummern) nach der IUBMB einhergehen. Die Enzymbezeichnung richtet sich erfindungsgemäß nach der jeweiligen konkreten Reaktion. Denn damit geht auch die konkrete im Zuge der vorliegenden Erfindung verwirklichte oder gegebenenfalls auszuschaltende Funktion einher.

Dieses Gene und Genprodukte können nun nach an sich bekannten Methoden, und ohne daß man die in Beispiel 1 geschilderte Sequenzierung nacharbeiten muß, gezielt anhand dieser Sequenzen künstlich synthetisiert werden.

Als weitere Alternative hierzu ist es möglich, die betreffenden Gene aus einem Bacillus-Stamm, insbesondere dem von der DSMZ erhältlichen Stamm *B. licheniformis* DSM 13, über PCR zu gewinnen, wobei die im Sequenzprotokoll angebenen jeweiligen Randsequenzen für die Synthese von Primern verwendet werden können. Bei Verwendung anderer Stämme werden die jeweils homologen Gene hierzu erhalten, wobei die PCR umso erfolgreicher sein sollte, je enger die ausgewählten Stämme mit *B. licheniformis* DSM 13 verwandt sind, weil damit eine zunehmende Sequenzübereinstimmung auch innerhalb der Primer-Bindungsregionen einhergehen dürfte.

Alternativ dazu können die im Sequenzprotokoll angegebenen Nukleinsäuren auch als DNA-Sonden eingesetzt werden, um die jeweiligen homologen Gene in Präparationen genomischer DNA anderer Spezies nachzuweisen. Das Vorgehen hierzu ist an sich bekannt; ebenso wie die Isolierung der auf diese Weise erhaltenen Gene, deren Klonierung, deren Expression und Gewinnung der zugehörigen Proteine. Insbesondere ist dabei an solche Arbeitsschritte gedacht, wie sie in Beispiel 1 für *B. licheniformis* selbst dargestellt sind.

Als Nachweis für die Existenz der betreffenden Proteine in einem interessierenden Stamm dient zunächst einmal ein chemischer Nachweis, ob die betreffenden Geruchsstoffe gebildet werden. Sodann können die hierfür vermuteten Enzymaktivitäten in geeigneten Nachweisreaktionen erfaßt werden. Dies geschieht beispielsweise so, daß die für die fragliche Reaktion relevante Ausgangsverbindung vorgelegt und mit einem Zellextrakt inkubiert wird. Bei Vorhandensein der betreffenden Enzymaktivität müßten sich die in dem betreffenden Stoffwechselweg nachfolgenden Produkte anhäufen, in dem Fall, daß alle nachfolgenden Enzyme vorhanden sind, bis hin zu dem Geruchsstoff.

Als Nachweis auf molekularbiologischer Ebene können anhand der im vorliegenden Sequenzprotokoll gezeigten Aminosäuresequenzen Proteine synthetisiert und hiergegen Antikörper gebildet werden. Diese sind dann beispielsweise in Western-Blots für den Nachweis des homologen Proteins in Zellextrakten der interessierenden Wirtszellen verwendbar.

Unter den hier genannten Nukleinsäuren, codierend für ein an der Synthese von Butanol und/oder Buttersäure beteiligtes und wie oben anhand SEQ ID NO. 31 definiertes erfindungsgemäßes Genprodukt, ist jeweils diejenige bevorzugt, die natürlicherweise in einem Mikroorganismus enthalten ist, vorzugsweise einem Bakterium, besonders bevorzugt einem grampositiven Bakterium, hierunter bevorzugt einem der Gattung *Bacillus,* hierunter besonders bevorzugt einem der Spezies *B. licheniformis* und hierunter ganz besonders bevorzugt *B*. *licheniformis* DSM13.

So ist es, wie soeben beschrieben, gegenüber der Neusynthese vergleichsweise einfach möglich, die betreffenden Nukleinsäuren aus natürlichen Spezies, insbesondere Mikroorganismen zu erhalten. Hierunter sind in Hinblick auf die gestellte Aufgabe zunehmend diejenigen bevorzugt, die sich fermentieren lassen und die in großtechnischen Fermentationen tatsächlich eingesetzt werden. Dazu zählen besonders Vertreter der Gattungen *Staphylococcus, Corynebakterium* und *Bacillus.* Hierunter sind beispielsweise *S*. *carnosus* und *C*. *glutamicum* zu nennen, sowie *B. subtilis, B. licheniformis, B. amyloliquefaciens, B. agaradherens, B. lentus, B. globigii* und *B. alkalophilus.* Am meisten ist *B*. *licheniformis* DSM13 bevorzugt, weil aus diesem exakt die im Sequenzprotokoll aufgelisteten Sequenzen erhalten werden konnten.

Diese Erläuterungen treffen in gleicher Weise auf die zugehörigen Proteine zu.

Somit ist unter den hier genannten, an der Synthese von Butanol und/oder Buttersäure beteiligten Genprodukten, definiert anhand SEQ ID NO. 32, jeweils solch eines bevorzugt, welches natürlicherweise von einem Mikroorganismus gebildet wird, vorzugsweise von einem Bakterium, besonders bevorzugt von einem grampositiven Bakterium, hierunter bevorzugt von einem der Gattung *Bacillus,* hierunter besonders bevorzugt von einem der Spezies *B. licheniformis* und hierunter ganz besonders bevorzugt von *B. licheniformis* DSM13.

Der in grampositiven Bakterien der Gattung *Bacillus* genutzte Stoffwechselweg zur Synthese von Butanol und/oder Buttersäure als Teil des Buttersäure-Metabolismus ist in Figur 1 dargestellt. Dieser Stoffwechselweg ergibt sich letztlich aus dem Fettsäurestoffwechsel.

An den in Figur 1 gezeigten Reaktionen sind folgende Enzyme beteiligt, wobei die zugehörige Nummer den jeweiligen in der Figur angegebenen Reaktionsschritt bezeichnet:
(1.) 3-Hydroxybutyryl-CoA-Dehydrogenase (E.C. 1.1.1.157),
(2.) 3-Hydroxybutyryl-CoA-Dehydratase (E.C. 4.2.1.55),
(3.) Butyryl-CoA-Dehydrogenase (E.C. 1.3.99.25),
(4.) Phosphat-Butyryl-Transferase (E.C. 2.3.1.19),
(5.) Butyrat-Kinase (E.C. 2.7.2.7),
(6.) Butyraldehyd-Dehydrogenase und
(8.) NADH-abhängige Butanol-Dehydrogenase A (E.C. 1.1.1.-).
Reaktion (7.) erfolgt in der Regel durch nichtenzymatische Oxidation durch Luftsauerstoff.

Lösungen der gestellten Aufgabe und damit eigenständige Ausführungsformen der vorliegenden Erfindung stellen somit Verfahren zur Fermentation eines Mikroorganismus dar, bei dem mindestens eines der Gene auf einem Stoffwechselweg zur Synthese von Butanol und/oder Buttersäure (als Teil des Buttersäure-Metabolismus) funktionell inaktiviert ist, wobei folgendes Enzym funktionell inaktiviert ist:
Phosphat-Butyryl-Transferase (E.C. 2.3.1.19).

Mit dieser Lösung sind die bereits erläuterten Vorteile verbunden.

Vorzugsweise handelt es sich dabei um jedes derartige Verfahren, bei dem der Mikroorganismus nur noch 50% der natürlicherweise unter denselben Bedingungen gebildeten Menge, vorzugsweise nur noch 10%, besonders bevorzugt kein Butanol beziehungsweise keine Buttersäure bildet.

Unter diesen Prozentwerten sind analog der oben für die Sequenzhomologie gemachten Aussage wiederum alle dazwischenliegenden ganzzahligen oder gebrochenen Prozentwerte in entsprechend bevorzugter Abstufung zu verstehen. Zur Bestimmung dieser Werte werden Zellen eines nichtbehandelten Stamms und eines behandelten Stamms unter ansonsten identischen Bedingungen fermentiert und geeigneterweise während der Fermentation die Bildungsrate an dem unerwünschten Geruchsstoff auf an sich bekannte Weise ermittelt. Da die Stämme ansonsten identisch sind, sind die Unterschiede hinsichtlich der Bildung dieses Stoffs auf die unterschiedlichen Genaktivitäten zurückzuführen. Dabei ist erfindungsgemäß jede Verringerung der Bildung des Geruchsstoffs erwünscht. Prozentual vergleichbare Werte erhält man, indem man aus beiden Fermentationen Proben (etwa aus der Abluft) nimmt und nach an sich bekannten analytischen Methoden den Gehalt des jeweiligen Stoffs bestimmt. Bevorzugt ist es, diesen Wert beim Übergang in die stationäre Wachstumsphase zu bestimmen, weil dieser Zeitpunkt meist eindeutig zu erkennen ist und zugleich in der Regel mit der höchsten Stoffwechselrate einhergeht.

Hiermit wird der im allgemeinen hohen Flexibilität von Mikroorganismen hinsichtlich ihres Stoffwechsels Rechnung getragen. So ist es denkbar, daß die Inaktivierung eines Gens durch eine Aktivitätsverstärkung eines anderen, *in vivo* möglicherweise nicht ganz so leistungsfähigen Gens und/oder Proteins zum Teil ausgeglichen wird. Zunehmend bevorzugt ist jedoch eine möglichst weitgehende Inaktivierung des genannten Wegs.

Bevorzugt ist ein solches erfindungsgemäßes Verfahren, wobei es sich bei dem funktionell inaktivierten Enzym um das in dem betreffenden Mikroorganismus natürlicherweise aktive Homologe zu einem der folgenden Proteine aus *B. licheniformis* DSM 13 handelt: Phosphat-Butyryl-Transferase (E.C. 2.3.1.19), definiert über SEQ ID NO. 32.

Bevorzugt ist ein solches erfindungsgemäßes Verfahren, wobei das Enzym auf genetischer Ebene funktionell inaktiviert wird, vorzugsweise durch Inaktivierung eines Gens, das der Nukleinsäure entspricht, die für eines der folgenden Proteine von *B. licheniformis* DSM 13 codiert: Phosphat-Butyryl-Transferase (E.C. 2.3.1.19), definiert über SEQ ID NO. 31.

Denn der gestellten Aufgabe entsprechend sollte vorzugsweise eine ursächliche, das heißt auf molekularbiologischer Ebene ansetzende Lösung gefunden werden. Wie dementsprechende Deletionen vorgenommen werden können, ist in Beispiel 3 erläutert; weitere Ausführungen hierzu werden weiter unten gegeben.

Bevorzugt ist somit ein solches erfindungsgemäßes Verfahren, wobei zur Inaktivierung auf genetischer Ebene eine der erfindungsgemäßen Nukleinsäuren innerhalb des oben bezeichneten Homologiebereichs zu
SEQ ID NO. 31
verwendet worden ist, vorzugsweise ein, besonders bevorzugt zwei Teile dieser Sequenz, die jeweils mindestens 70 zusammenhängende Positionen umfassen.

Eine weitere Ausführungsform der vorliegenden Erfindung stellt die Verwendung eines Gens, das der Nukleinsäure entspricht, die für eines der folgenden Proteine von *B. licheniformis* DSM 13 codiert, zur funktionellen Inaktivierung eines Stoffwechselwegs zur Synthese von Butanol und/oder Buttersäure und/oder Isobuttersäure (als Teil des Buttersäure-Metabolismus) auf genetischer Ebene in einem Mikroorganismus dar:
Phosphat-Butyryl-Transferase (E.C. 2.3.1.19), definiert über SEQ ID NO. 31.

Für derartige Verwendungen gilt prinzipiell das gleiche wie bisher bereits zu den entsprechenden Verfahren ausgeführt worden ist.

Bevorzugt ist dementsprechend eine solche erfindungsgemäße Verwendung von erfindungsgemäßen Nukleinsäuren innerhalb des oben bezeichneten Homologiebereichs zu
SEQ ID NO. 31
zur funktionellen Inaktivierung, vorzugsweise von einem, besonders bevorzugt von zwei Teilen dieser Sequenz, wobei diese Teile jeweils mindestens 70 zusammenhängende Positionen umfassen.

Weitere Ausführungsformen, die auf diesen Fermentationsverfahren und Verwendungen aufbauen, werden weiter unten ausgeführt.

In jeweils bevorzugten Ausführungsformen handelt es sich bei den oben beschriebenen erfindungsgemäßen Verwendungen der Gene und/oder Nukleinsäuren auf dem beschriebenen Stoffwechselweg um solche, wobei die funktionelle Inaktivierung während der Fermentation des Mikroorganismus erfolgt.

Denn der gestellten Aufgabe entsprechend sollte die Fermentation auf genetischer Ebene verbessert werden. Bei Fermentation von Mikroorganismen, die über diese Gene und/oder Nukleinsäuren entsprechend modifiziert worden sind, ist zu erwarten, daß die Menge der Geruchs-und/oder Giftstoffe geringer als bei nichtmodifizierten Stämmen ist. Dieser, sich im Laufe der Fermentation ergebende Vorteil ist erfindungsgemäß bevorzugt, weil er sich sowohl auf den Herstell-, das heißt Fermentationsprozeß als auch auf die nachfolgende Aufarbeitung vorteilhaft auswirkt.

Nach einer Alternative handelt es sich bei all diesen Verwendungen von Genen und/oder den beschriebenen erfindungsgemäßen Nukleinsäuren um solche, wobei jeweils eine für ein nichtaktives Protein codierende Nukleinsäure mit einer Punktmutation eingesetzt wird.

Derartige Nukleinsäuren können über an sich bekannte Verfahren zur Punktmutagenese erzeugt werden. Solche sind beispielsweise in einschlägigen Handbüchern wie dem von Fritsch, Sambrook und Maniatis, "Molecular cloning: a laboratory manual", Cold Spring Harbour Laboratory Press, New York, 1989, dargestellt. Zudem stehen hierfür inzwischen zahlreiche kommerzielle Baukästen zur Verfügung, etwa das QuickChange^{®}-Kit der Firma Stratagene, La Jolla, USA. Dessen Prinzip besteht darin, daß Oligonukleotide mit einzelnen Austauschen (Mismatch-Primer) synthetisiert und mit dem einzelsträngig vorgelegten Gen hybridisiert werden; anschließende DNA-Polymerisation ergibt dann entsprechende Punktmutanten. Hierfür können die jeweiligen Spezies-eigenen Sequenzen dieser Gene verwendet werden. Aufgrund der hohen Homologien ist es möglich und erfindungsgemäß besonders vorteilhaft, diese Reaktion anhand der im Sequenzprotokoll zur Verfügung gestellten Nukleotidsequenzen durchzuführen. Diese Sequenzen können auch dazu dienen, entsprechende Mismatch-Primer für verwandte Spezies zu entwerfen.

Nach einer Alternative handelt es sich bei all diesen Verwendungen von Genen und/oder den beschriebenen erfindungsgemäßen Nukleinsäuren um solche, wobei jeweils eine Nukleinsäure mit einer Deletions- oder Insertionsmutation eingesetzt wird, vorzugsweise umfassend die jeweils mindestens 70 bis 150 Nukleinsäurepositionen umfassenden Randsequenzen des für das Protein codierenden Bereichs.

Auch diese Verfahren sind dem Fachmann an sich vertraut. Somit ist es möglich, die Bildung eines oder mehrerer der beschriebenen Genprodukte durch die Wirtszelle dadurch zu verhindern, daß ein Teil des betreffenden Gens auf einem entsprechenden Transformationsvektor über Restriktionsendonukleasen herausgeschnitten und der Vektor anschließend in den interessierenden Wirt transformiert wird, wo über die - bis dahin noch mögliche - homologe Rekombination das aktive Gen gegen die inaktive Kopie ausgetauscht wird. In der Ausführungsform der Insertionsmutation kann lediglich das intakte Gen unterbrechend oder anstelle eines Genteils ein anderes Gen, beispielsweise ein Selektionsmarker eingefügt werden. Hierüber ist das Mutationsereignis in an sich bekannter Weise phänotypisch überprüfbar.

Um diese jeweils notwendigen Rekombinationsereignisse zwischen dem in die Zelle eingeführten defekten Gen und der beispielsweise auf dem Chromosom endogen vorhandenen intakten Genkopie zu ermöglichen, ist nach dem derzeitigen Wissensstand eine Übereinstimmung in jeweils mindestens 70 bis 150 zusammenhängenden Nukleinsäurepositionen, jeweils in den beiden Randsequenzen zu dem nichtübereinstimmenden Teil nötig, wobei es auf den dazwischenliegenden Teil nicht ankommt. Dementsprechend sind solche Ausführungsformen bevorzugt, die lediglich zwei flankierende Regionen mit mindestens diesen Größen umfassen.

Nach einer weiteren alternativen Ausführungsform dieser Verwendung werden Nukleinsäuren mit insgesamt zwei Nukleinsäureabschnitten eingesetzt, die jeweils mindestens 70 bis 150 Nukleinsäurepositionen umfassen und damit den für das Protein codierenden Bereich zumindest teilweise, vorzugsweise vollständig flankieren. Die flankierenden Bereich können dabei ausgehend von den bekannten Sequenzen über an sich bekannte Methoden, beispielsweise mithilfe nach außen gerichteter PCR-Primer und einer Präparation genomischer DNA als Matrize ermittelt werden (anchored PCR). Denn allein um den Austausch der beiden Genkopien über homologe Rekombination zu ermöglichen, braucht es sich dabei nicht zwangsläufig um proteincodierende Abschnitte zu handeln. Der vorliegenden Erfindung zufolge können die hierfür benötigten Primer anhand der im Sequenzprotokoll angegebenen Nukleotidsequenzen auch für andere Spezies grampositiver Bakterien und hierunter insbesondere für solche der Gattung *Bacillus* entworfen werden. Alternativ zu diesem experimentellen Ansatz können derartige, wenigstens zum Teil nichtcodierende Bereiche für viele dieser Gene aus verwandten Spezies, beispielsweise aus *B. subtilis* Datenbankeinträgen entnommen werden, beispielsweise der Datenbank Subtilist des Institute Pasteur, Paris, Frankreich (http://genolist.pasteur.fr/SubtiList/genome.cgi) oder den in Beispiel 2 angegebenen Datenbanken.

Die vorliegende Erfindung zielt insbesondere darauf ab, gentechnisch verbesserte Mikroorganismen für die biotechnologische Produktion zur Verfügung zu stellen. Somit stellt jeder Mikroorganismus eine Ausführungsform der vorliegenden Erfindung dar, bei dem mindestens eines der Gene funktionell inaktiviert ist, das der Nukleinsäure entspricht, die für eines der folgenden Proteine von *B. licheniformis* DSM 13 codiert:
- wahrscheinliche Phosphat-Butyryl-Transferase (E.C. 2.3.1.19), definiert über SEQ ID NO. 31.

Hierbei ist unter "entspricht" jeweils solch ein Gen des betrachteten Organismus gemeint, das für ein Genprodukt mit derselben biochemischen Aktivität codiert, wie sie oben im Zusammenhang mit den jeweiligen Stoffwechselwegen definiert ist. In der Regel ist das gleichzeitig dasjenige von allen *in vivo* translatierten Genen dieses Organismus, welches zu dem genannten Gen aus *B. licheniformis* die jeweils höchste Homologie aufweist (in der Regel mehr als 40% Identität, feststellbar über ein Alignment beider Sequenzen, wie in Beispiel 2 durchgeführt).

Weiterhin ist hierunter jeweils solch ein Mikroorganismus bevorzugt, bei dem es sich um ein Bakterium handelt.

Denn solche besitzen für die biotechnologische Produktion eine besondere Bedeutung. Zum anderen sind die betreffenden Wege an Mikroorganismen der Gattung *Bacillus* beschrieben worden.

Hierunter ist jeweils solch ein Mikroorganismus bevorzugt, wobei es sich um ein gramnegatives Bakterium handelt, insbesondere eines der Gattungen *Escherichia coli, Klebsiella, Pseudomonas* oder *Xanthomonas,* insbesondere um Stämme von *E*. *coli* K12, *E*. *coli* B oder *Klebsiella planticola,* und ganz besonders um Derivate der Stämme *Escherichia coli* BL21 (DE3), *E*. *coli* RV308, *E*. *coli* DH5α, E.*coli* JM109, *E*. *coli* XL-1 oder *Klebsiella planticola* (Rf).

Denn hierbei handelt es sich um wichtige Stämme für molekularbiologische Arbeiten an Genen, etwa zur Klonierung (siehe Beispiele) und außerdem um wichtige Produktionsstämme.

Alternativ hierzu wird jeweils solch ein Mikroorganismus bevorzugt, wobei es sich um ein grampositives Bakterium handelt, insbesondere eines der Gattungen *Bacillus, Staphylococcus* oder *Corynebacterium,* ganz besonders der Species *Bacillus lentus, B. licheniformis, B. amyloliquefaciens, B. subtilis, B. globigii* oder *B. alcalophilus, Staphylococcus carnosus* oder *Corynebacterium glutamicum,* und hierunter ganz besonders um *B. licheniformis* DSM 13.

Denn diese sind besonders wichtig für die biotechnologische Produktion von Wertstoffen und Proteinen, weil sie natürlicherweise in der Lage sind, diese ins umgebende Medium auszuschleusen. Zum anderen sind sie mit dem für die vorliegende Anmeldung eingesetzten *B. licheniformis* zunehmend verwandt, so daß die geschilderten, auf die jeweils offenbarten Sequenzen zurückgehenden Arbeitsschritte mit einem zunehmenden Maß an Verwandtschaft zu *B. licheniformis* DSM 13 umso erfolgreicher ablaufen dürften. So ist beispielsweise anzunehmen, daß ein im Sequenzprotokoll angegebenes Gen nach Punktmutation in einer verwandten Spezies unmittelbar für eine Deletionsmutation verwendbar ist, ohne daß das homologe Gen hierzu aus diesem Stamm selbst isoliert werden muß.

Die vorliegende Erfindung zielt insbesondere darauf ab, Fermentationsverfahren zu verbessern. Somit stellt jedes Verfahren zur Fermentation eines oben beschriebenen erfindungsgemäßen Mikroorganismus eine Ausführungsform der vorliegenden Erfindung dar.

Inbesondere handelt es sich bei diesen Verfahren und den Verfahren, welche oben jeweils im Zusammenhang mit einer Einflußnahme auf einen beschriebenen Stoffwechselweg beschrieben sind, um solche Verfahren, wobei ein Wertstoff hergestellt wird, insbesondere eine niedermolekulare Verbindung oder ein Protein.

Denn das sind die wesentlichen Einsatzgebiete der biotechnologischen Produktion durch Fermenation von Mikroorganismen.

Hierunter ist jeweils solch ein Verfahren bevorzugt, wobei es sich bei der niedermolekularen Verbindung um einen Naturstoff, einen Nahrungsmittelergänzungsstoff oder um eine pharmazeutisch relevante Verbindung handelt.

Denn das sind wichtige Produktgruppen der biotechnologischen Produktion durch Fermentation von Mikroorganismen.

Unter solchen biotechnologischen Verfahren zur Produktion von Proteinen durch Fermenation von Mikroorganismen ist jeweils solch ein Verfahren bevorzugt, wobei es sich bei dem Protein um ein Enzym handelt, insbesondere eines aus der Gruppe der α-Amylasen, Proteasen, Cellulasen, Lipasen, Oxidoreduktasen, Peroxidasen, Laccasen, Oxidasen und Hemicellulasen.

Denn hierbei handelt es sich um wichtige im großtechnischen Maßstab hergestellte Enzyme, beispielsweise für die Einarbeitung in Wasch- oder Reinigungsmittel.

Darüber hinaus stehen die erfindungsgemäß bereitgestellten Genprodukte für weitere Anwendungen zur Verfügung. Somit wird die vorliegende Erfindung auch durch jede Verwendung jedes erfindungsgemäßen Genprodukts in einem Reaktionsansatz oder Verfahren entsprechend seiner biochemischen Eigenschaften verwirklicht, das wie oben dargestellt unter Bezug auf SEQ ID NO. 32 definiert ist.

Hierunter fallen vorzugsweise Verwendungen zur Synthese von Butanol und/oder Buttersäure, gegebenenfalls in geeigneter Kombination mit weiteren Enzymen.

So handelt es sich bei den Produkten der dargestellten Stoffwechselwege um einfache organischchemische Verbindungen, an denen in der Chemie durchaus ein Bedarf besteht, beispielsweise um sie als Ausgangsstoffe für komplexere Synthesen einzusetzen. Deren Herstellung kann, insbesondere wenn es sich um stereochemische Reaktionen handelt, durch Einsatz entsprechender Enzyme erheblich vereinfacht werden, weil diese zumeist spezifisch ein Enantiomeres bilden. Man spricht von Biotransformation, wenn derartige Synthesewege zumindest in einem Reaktionsschritt von biologischen Katalysatoren übernommen werden. Hierzu eignen sich prinzipiell alle erfindungsgemäßen Genprodukte.

Die nachfolgenden Beispiele erläutern die vorliegende Erfindung weiter.

### Beispiele

Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laboratory Press, New York, 1989, oder vergleichbaren einschlägigen Werken angegeben sind. Enzyme und Baukästen (Kits) werden nach den Angaben der jeweiligen Hersteller eingesetzt.

### Beispiel 1

### Identifizierung der Nukleinsäuren gemäß SEQ ID NO. 1, 3, 5, 7, 9, 11, 13, 15, 17, 19, 21, 23, 25, 27, 29, 31, 33, 35, 37, 39, 41, 43, 45, 47 und 49 aus B. licheniformis DSM 13

Aus dem von der Deutschen Sammlung von Mikroorganismen und Zellkulturen GmbH, Mascheroder Weg 1 b, 38124 Braunschweig (http://www.dsmz.de) für jedermann erhältlichen Stamm *B. licheniformis* DSM 13 wurde nach Standardmethoden die genomische DNA präpariert, mechnisch fraktioniert und über Elektrophorese in einem 0,8%igen Agarosegel aufgetrennt. Für eine Schrotschußklonierung der kleineren Fragmente wurden die 2 bis 2,5 kb großen Fragmente aus dem Agarosegel eluiert, dephosphoryliert und als stumpf endende (blunt ended) Fragmente in die Smal-Restriktionsschnittstelle des Vektors pTZ19R-Cm ligiert. Dabei handelt es sich um ein Chloramphenicol-Resistenz verleihendes Derivat des von der Firma Fermentas (St. Leon-Rot) kommerziell erhältlichen Plasmids pTZ19R. Dadurch wurde eine Genbank der kleineren Fragmente erhalten. Als zweite Schrotschußklonierung wurden die durch eine partielle Restriktion mit dem Enzym Saulllal erhaltenen genomischen Fragmente in das SuperCos-1-Vektorsystem ("Cosmid Vector Kit") der Firma Stratagene, La Jolla, USA, ligiert, wodurch eine Genbank über die überwiegend größeren Fragmente erhalten wurde.

Aus den durch Transformation mit den betreffenden Genbanken erhältlichen Bakterien *E*. *coli* DH5α (D.Hannahan (1983): "Studies on transformation on Escherichia coli"; J. Mol. Microbiol., Band 166, Seiten 557 - 580) wurden die betreffenden rekombinanten Plasmide isoliert und sequenziert. hierbei kam die Farbstoffabbruchmethode (dye terminator chemistry) zum Einsatz, durchgeführt durch die automatischen Sequenziergeräte Mega-BACE 1000/4000 (Fa. Amersham Bioscence, Piscataway, USA) und ABI Prism 377 (Fa. Applied Biosystems, Foster City, USA).

Auf diese Weise wurden unter anderem die im Sequenzprotokoll der vorliegenden Anmeldung angegebenen Sequenzen SEQ ID NO. 1, 3, 5, 7, 9, 11, 13, 15, 17, 19, 21, 23, 25, 27, 29, 31, 33, 35, 37, 39, 41, 43, 45, 47 und 49 erhalten. Die hiervon abgeleiteten Aminosäuresequenzen sind - die zugehörigen unter der jeweils höheren Nummer - unter SEQ ID NO. 2, 4, 6, 8, 10, 12, 14, 16, 18, 20, 22, 24, 26, 28, 30, 32, 34, 36, 38, 40, 42, 44, 46, 48 und 50 angegeben.

### Beispiel 2

### Sequenzhomologien

Nach Ermittlung der DNA- und Aminosäuresequenzen gemäß Beispiel 1 wurden durch Recherche in den Datenbanken GenBank (National Center for Biotechnology Information NCBI, National Institutes of Health, Bethesda, MD, USA), EMBL-European Bioinformatics Institute (EBI) in Cambridge, Großbritannien (http://www.ebi.ac.uk), Swiss-Prot (Geneva Bioinformatics (GeneBio) S.A., Genf, Schweiz; http://www.genebio.com/sprot.html) und PIR (Protein Information Resource, National Biomedical Research Foundation, Georgetown University Medical Center, Washington, DC, USA; http://www.pir.georgetwown.edu) die jeweils nächstähnlichen, bisher bekannten Homologe ermittelt. Dabei wurde die Option nr (nonredundand) gewählt.

Die ermittelten DNA- beziehungsweise Aminosäuresequenzen wurden zur Bestimmung des Homologiegrads über Alignments einander gegenübergestellt; hierfür wurde das Computerprogramm Vector NTI^{®} Suite Version 7, verwendet, welches von der Firma Informax Inc., Bethesda, USA, erhältlich ist. Hierbei wurden die Standard-Parameter dieses Programms angewendet, das heißt für den Vergleich der DNA-Sequenzen: K-tuple size: 2; Number of best Diagonals: 4; Window size: 4; Gap penalty: 5; Gap opening penalty: 15 und Gap extension penalty: 6,66. Für den Vergleich der Aminosäure-Sequenzen galten folgende Standard-Parameter: K-tuple size: 1; Number of best Diagonals: 5; Window size: 5; Gap penalty: 3; Gap opening penalty: 10 und Gap extension penalty: 0,1. Die Ergebnisse dieser Sequenzvergleiche sind zusammen mit einer Angabe der jeweiligen Enzymnamen, das heißt Funktionen, E.C.-Nummern, und den zugehörigen Stoffwechselwegen in folgender Tabelle 1 zusammengestellt. Die Numerierung der aus dem Stand der Technik bekannten Enzyme ist die übereinstimmende Nomenklatur der oben genannten Datenbanken.

**Tabelle 1: Nächstähnliche Gene beziehungsweise Proteine zu den in Beispiel 1 ermittelten Genen und Proteinen.**

| Darin bedeuten: | | | | | |
|---|---|---|---|---|---|
| ID | die im Sequenzprotokoll zur vorliegenden Anmeldung angegebene SEQ ID NO.; | | | | |
| E.C.-Nr. | die Nummer gemäß der internationalen Enzymklassifikation (*Enzyme Nomenclature* der IUBMB). | | | | |

| **ID** | **Name des Enzyms (sofern möglich des Gens) und gegebenenfalls zusätzliche Informationen** | **E.C.-Nr.** | **Stoffwechsel-weg** | **Identität zum nächsten Verwandten auf DNA-ebene %** | **Identität zum nächsten Verwandten auf Proteinebene %** |
|---|---|---|---|---|---|
| 1, 2 | putative Verzweigt-ketten-Aminosäure-Aminotransferase | 2.6.1.42 | Valin- /Isoleucin-Katabolisums | 62,40% zu gb\|AE017003.1\|, *B*. cereus ATCC 14579, Abschnitt 6 v. 18 des kompletten Genoms | 69% zur Aminotransferase IV aus *B*. anthracis Ames (NP_655296.1) |
| 3, 4 | putative Verzweigt-ketten-Aminosäure-Aminotransferase | 2.6.1.42 | Valin- /Isoleucin-Katabolisums | 73,80% zu emb\|Z49992.1\|BSC ELABCD, *B.subtilis-* Gene celA, celB, celC, celD und ywaA | 79% zur Verzweigt-ketten-Aminosäure-Aminotransferase aus *B. subtilis* 168 (NP_391734.1) |
| 5, 6 | Lysin- und/oder Arginin-Decarboxylase (*speA*) | 4.1.1.18 bezieh-ungs-weise 4.1.1.19 | Cadaverin-und/oder Putrescin-Synthese (Lysin-und/oder Arginin-Katabolismus) | 74,00% zu emb\|X58433.1\|BSC ADDNA, *B.* subtillis, cad-Gen für die Lysin-Decarboxylase | 85% zur Lysin-Decarboxylase aus B. *subtilis* (NP_389346; A54546) |
| 7, 8 | NADH-abhängige Butanol-Dehydrogenase A (*yugJ*) | 1.1.1.- | Buttersäure-Metabolismus | 76,30% zu emb\|Z93934.1\|BSZ 93934, B.*subtilis,* genomisches DNA-Fragment von patB bis yugK | 89% zur NADH-abhängigen Butanol-Dehydrogenase aus *B. subtilis* 168 (NP_391015.1) |
| 9, 10 | Acyl-CoA-Dehydrogenase (so, das heißt allgemeiner im Sequenzprotokoll angegeben) / Butyryl-CoA-Dehydrogenase | 1.3.99.-/ Leucin-1.3.99.2 5 | Katabolismus, Valin-/Isoleucin-Katabolisums, Buttersäure-Metabolismus | 74,70% zu emb\|Z49782.1\|BSD NA320D, B.*subtilis,* chromosomale DNA (Region 320-321 Grad) | 82% zur kurzketten-spezifischen Acyl-CoA-Dehydro-genase, aus *B*. cereus ATCC 14579 (NP_835003.1) |
| 11, 12 | Acyl-CoA-Dehydrogenase (so, das heißt allgemeiner im Sequenzprotokoll angegeben) / Butyryl-CoA-Dehydrogenase Das erste Codon dürfte *in vivo* als Methionin translatiert werden. | 1.3.99.- | Leucin-Katabolismus | 59,30% zu emb\|Z49782.1\|BSD NA320D, *B. subtilis,* chromosomale DNA (Region 320-321 Grad) | 63% zur C-terminalen Domäne der Acyl-CoA Dehydrogenase, aus *B.* anthracis Ames (NP_653803.1) |
| 13, 14 | 3-Hydroxybutyryl-CoA-Dehydrogenase | 1.1.1.15 7 | Buttersäure-Metabolismus | 62,40% zu gb\|AE017015.1\|, *B*. cereus ATCC 14579, Abschnitt 18 von 18 des kompletten Genoms | 65% zur NAD-Bindungsdomäne der 3-Hydroxyacyl-CoA Dehydro-genase, aus *B*. anthracis Ames (NP_653804.1) |
| 15, 16 | putatives Enoyl-CoA-Hydratase-Protein | 4.2.1.17 | Leucin-Katabolismus, Valin-/Isoleucin-Katabolisums | 61,00% zu emb\|Y14078.1\|BSY 14078, *B. subtilis,* 8.7 Kb chromosomale DNA: stromabwärts der glyB-prsA-Region | 58% zu YhaR aus *B. subtilis* 168 (CAB12828.2) |
| 17, 18 | wahrscheinliches Enoyl-(3-Hydroxy-isobutyryl)-Coenzym A-Hydrolase-Protein | noch nicht ver-geben | Leucin-Katabolismus, Valin-/Isoleucin-Katabolisums | 61,90% zu gb\|AE017031.1\|, *B.* anthracis Ames, Abschnitt 8 von 18 des kompletten Genoms | 62% zur 3-Hydroxyisobutyryl-Coenzyme A-Hydrolase aus *B*. cereus ATCC 14579 (NP_832055.1; AAP09256) |
| 19, 20 | wahrscheinliche Enoyl-CoA-Hydratase | 4.2.1.17 | Leucin-Katabolismus, | 43,50% zu gb\|AC084761.2\|, | 48% zur 3-Hydroxbutyryl-CoA |
| | (*echA8*) Das erste Codon dürfte *in vivo* als Methionin translatiert werden. | | Valin-/Isoleucin-Katabolisums | Gallus gallus, Klon WAG-69H2, komplette Sequenz | Dehydratase aus *B. subtilis* 168 (NP_390732.1) |
| 21, 22 | Acyl-CoA Dehydrogenase | 1.3.99.- | Leucin-Katabolismus, Valin-/Isoleucin-Katabolisums | 49,90% zu gb\|AE015940.1\|, Clostridium tetani E88, Abschnitt 5 von 10 des kompletten Genoms | 61% zur Acyl-CoA Dehydrogenase aus *B*. cereus ATCC 14579 (NP_832051.1) |
| 23,24 | Acetyl-Coenzym A-Synthetase (so im Sequenzprotokoll angegeben) oder Propionat-CoA Ligase (*acsA*) | 6.2.1.1 | Propionat-Metabolismus | 63,00% zu db\|AP001511.1\|, *B.* halodurans, genomische DNA, Abschnitt 5/14 | 61 % zur Acetyl-CoA-Synthetase aus *B.* halodurans (NP_242003.1) |
| 25, 26 | 3-Hydroxybutyryl-CoA-Dehydratase (*yngF*) 27, 28 Acyl-CoA Dehydrogenase (so, das heißt allgemeiner, im Sequenzprotokoll angegeben) / Butyryl-CoA-Dehydrogenase (*yusJ*) | 4.2.1.55 1.3.99.-/ Leucin-1.3.99.2 5 | Buttersäure-Metabolismus Katabolismus, Valin-/Isoleucin-Katabolisums, Buttersäure-Metabolismus | 63,80% zu emb\|Y13917.1\|BSY 13917, *B*. *subtilis,* Gene ppsE, yngL, yngK, yotB, yngJ, yngI, yngH, yngG and *yngF* und partielle Gene ppsD and yngE 72,90% zu emb\|Y13917.1\|BSY 13917, *B*. *subtilis,* Gene ppsE, yngL, yngK, yotB, yngJ, yngI, yngH, yngG und *yngF* und partielle Gene ppsD und yngE | 65% zur Hydroxybutyryl-dehydratase aus *B. subtilis* (AAF32340.1) 82% zur Butyryl-CoA-Dehydrogenase aus *B. subtilis* 168 (NP_389708.1) |
| 29, 30 | 3-Hydroxy-Isobutyrat Dehydrogenase / hypothetische Oxidoreduktase (so, das heißt allgemeiner, im Sequenzprotokoll angegeben) (*ykwC*) | 1.1.1.31 beziehungsweise 1.1.-.- | Valin-Katabolismus | 72,80% zu emb\|AJ222587.1\|B S16829KB, *B. subtilis,* 29kB-DNA Fragment vom Gen *ykwC* zum Gen cse15 | 81% zur 3-Hydroxyisobutyrat-Dehydrogenase aus *B. subtilis* 168 (NP_389279.1) |
| 31, 32 | wahrscheinliche Phosphat-Butyryl-Transferase | 2.3.1.19 | Buttersäure-Metabolismus | 46,30% zu gb\|S81735.11\|8173 5, Leucine-Dehydrogenase | 65% zur Phosphat-Butyryltransferase aus *B. subtilis* 168 (NP_390289.1) |
| 33, 34 | wahrscheinliche Butyrat-Kinase | 2.7.2.7 | Buttersäure-Metabolismus | 72,50% zu emb\|Z99116.2\|BSU B0013, *B. subtilis,* komplettes Genom (Abschnitt 13 von 21): von 2409151 bis 2613687 | 80% zur Verzweigtketten-Fettsäure-Kinase aus *B. subtilis* 168 (NP_390287.1) |
| 35, 36 | Acetyl-Coenzym A-Synthetase (so im Sequenzprotokoll angegeben) oder Propionat-CoA Ligase (*acsA*) | 6.2.1.1 | Propionat-Metabolismus | 74,90% zu emb\|Z99119.2\|BSU B0016, *B. subtilis,* komplettes Genom (Abschnitt 16 von 21 von 3013458 bis 3213379 | 81% zur Acetyl-CoA-Synthetase aus *B. subtilis* 168 (NP_390846.1) und zur Acetat-CoA-Ligase aus *B. subtilis* (P39062, S39646) |
| 37, 38 | Acetat-CoA-Ligase (so im Sequenz-protokoll angegeben) oder Propionat-CoA-Ligase (*ytcI*) Das erste Codon dürfte *in vivo* als Methionin translatiert werden. | 6.2.1.1 | Propionat-Metabolismus | 70% zu emb\|Z99119.2\|BSU B0016, *B. subtilis,* komplettes Genom (Abschnitt 16 von 21): von 3013458 bis 3213379 | 73% zur Acetat-CoA-Ligase aus *B. subtilis* 168 (NP_390834.1, E69989) |
| 39, 40 | Lysin- und/oder Arginin-Decarboxylase (*speA*) | 4.1.1.18 bezieh-ungs-weise | Cadaverin-und/oder Putrescin-Synthese | 63,40% zu emb\|Z99104.2\|BSU B0001, *B. subtilis,* komplettes Genom | 62% zur Lysin-Decarboxylase aus *B. subtilis* 168 (NP_387908.1) und |
| | | 4.1.1.19 | (Lysin-und/oder Arginin-Katabolismus) | (Abschnitt 1 von 21): von 1 bis 213080 | *B*. perfrigens (NP_976355) |
| 41, 42 | wahrscheinliche Enoyl-CoA Hydratase (*ysiB*) | 4.2.1.17 | Leucin-Katabolismus, Valin-/Isoleucin-Katabolisums | 70,30% zu emb\|Z75208.1\|BSZ 75208, B.*subtilis,* genomische Sequenz, 89009bp | 73% zur 3-Hydroxbutyryl-CoA -Dehydratase aus *B. subtilis* 168 (NP_390732.1) |
| 43, 44 | Ähnliches zur 3-Hydroxyacyl-CoA-Dehydrogenase | 1.1.1.35 | Isoleucin-Katabolisums | 71,60% zu emb\|Z99120.2\|BSU B0017, *B. subtilis,* komplettes Genom (Abschnitt 17 von 21): von 3213330 bis 3414388 | 76% zur 3-Hydroxyacyl-CoA Dehydrogenase aus *B. subtilis* 168 (NP_391163.1) |
| 45,46 | 3-Methyl-2-Oxobutanoat-Dehydrogenase / 2-Oxoglutarat-Dehydrogenase E1-Komponente (so, das heißt allgemeiner im Sequenzprotokoll angegeben) | 1.2.4.2 | Leucin-Katabolismus, Valin-/Isoleucin-Katabolisums | 75,70% zu emb\|X54805.1\|BSO DHA, B. *subtilis,* odhA-Gen für die 2-Oxoglutarat-Dehydrogenase | 78% zur E1-Untereinheit der 2-Oxoglutarate-Dehydrogenase aus *B. subtilis* (CAB13829.2) |
| 47, 48 | wahrscheinliche Säure-CoA-Ligase (*yhfL*) | 6.2.1.- | Propionat-Metabolismus | 62,20% zu gb\|AE017001.1\|, *B*. cereus ATCC 14579, Abschnitt 4 von 18 des kompletten Genoms (NP_388908.1) | 72% zur Langketten-Fettsäure-CoA-Ligase aus *B. subtilis* 168 |
| 49, 50 | Agmatinase (*ywhG*) | 3.5.1.11 | Cadaverin-und/oder Putrescin-Synthese (Lysin-und/oder Arginin-Katabolismus) | 80,9% zu *B*. *subtilis,* Gen BSUB0020 (Genebank, komplettes Genom) | 95% zur Agmatinase (Agmatin-Ureohydrolase) aus *B. subtilis* 168 (P70999) |

Man erkennt, daß es sich bei den gefundenen Genen und den hiervon abgeleiteten Genprodukten um neue Gene beziehungsweise Proteine mit einem deutlichen Abstand zum bisher bekannten Stand der Technik handelt.

### Beispiel 3

### Funktionelle Inaktivierung eines oder mehrerer der Gene gemäß SEQ ID NO. 1, 3, 5, 7, 9, 11, 13, 15, 17, 19, 21, 23, 25, 27, 29, 31, 33, 35, 37, 39, 41, 43, 45, 47 und 49 in B. licheniformis

### Prinzip der Herstellung eines Deletionsvektors

Jedes dieser Gene kann beispielsweise mittels eines sogenannten Deletionsvektors funktionell inaktiviert werden. Dieses Vorgehen ist an sich beispielsweise von J. Vehmaanperä et al. (1991) in der Publikation "Genetic manipulation of Bacillus amyloliquefaciens"; J. Biotechnol., Band 19, Seiten 221 - 240 beschrieben.

Ein geeigneter Vektor hierfür ist pE194, der in der Publikation "Replication and incompatibility properties of plasmid pE194 in Bacillus subtilis" von T.J. Gryczan et al. (1982), J. Bacteriol., Band 152, Seiten 722 - 735 charakterisiert ist. Der Vorteil dieses Deletionsvektors besteht darin, daß er einen Temperatur-abhängigen Replikationsursprung besitzt. Bei 33°C kann pE194 in der transformierten Zelle replizieren, so daß bei dieser Temperatur zunächst auf eine erfolgreiche Transformation selektiert wird. Anschließend werden die Zellen, die den Vektor enthalten, bei 42°C inkubiert. Bei dieser Temperatur repliziert der Deletionsvektor nicht mehr und es wird ein Selektionsdruck auf die Integration des Plasmids über einen zuvor ausgewählten homologe Bereich in das Chromosom ausgeübt. Eine zweite homologe Rekombination über einen zweiten homologen Bereich führt dann zur Exzision des Vektors zusammen mit der intakten Genkopie aus dem Chromosom und damit zur Deletion des *in vivo* chromosomal lokalisierten Gens. Möglich wäre auch als zweite Rekombination die Umkehrreaktion zur Integration, das heißt ein Herausrekombinieren des Vektors aus dem Chromosom, so daß das chromosomale Gen intakt bliebe. Die Gen-Deletion muß daher nach an sich bekannten Methoden, etwa im Southern-Blot nach Restriktion der chromosomalen DNA mit geeigneten Enzymen oder mit Hilfe der PCR-Technik anhand der Größe des amplifizierten Bereichs nachgewiesen werden.

Erforderlich ist also die Auswahl zweier homologer Bereiche des zu deletierenden Gens, die jeweils mindestens je 70 Basenpaare umfassen sollten, beispielsweise der 5'- und der 3'-Bereich des ausgewählten Gens. Diese werden so in den Vektor kloniert, daß sie einen für ein nichtaktives Protein codierenden Teil flankieren oder unter Auslassung des dazwischenliegenden Bereichs direkt aufeinanderfolgen. Hierdurch wird der Deletionsvektor erhalten.

### Deletion der hier betrachteten Gene

Zur Konstruktion eines erfindungsgemäßen Deletionsvektors werden die 5'- und 3'-Bereiche des jeweils interessierenden dieser Gene mittels PCR amplifiziert. Zur Konstruktion geeigneter Primer stehen die im Sequenzprotokoll angegebenen Sequenzen SEQ ID NO. 1, 3, 5, 7, 9, 11, 13, 15, 17, 19, 21, 23, 25, 27, 29, 31, 33, 35, 37, 39, 41, 43, 45, 47 und 49 zur Verfügung, die aus B. *licheniformis* stammen, aufgrund zu erwartender Homologien aber auch für andere Spezies, insbesondere der Gattung *Bacillus* geeignet sein sollten.

Die beiden amplifizierten Bereiche werden geeigneterweise unmittelbar hintereinander auf einem für diese Arbeiten gebräuchlichen Vektor zwischenkloniert, zum Beispiel auf dem Vektor pUC18, der sich für Klonierungssschritte in E. *coli*. eignet.

Im nächsten Schritt erfolgt eine Umklonierung in den zur Deletion ausgewählten Vektor pE194 und dessen Transformation in *B. subtilis* DB104, etwa nach der Methode der ProtoplastenTransformation nach Chang & Cohen (1979; "High Frequency Transformation of Bacillus subtilis Protoplasts by Plasmid DNA"; Molec. Gen. Genet. (1979), Band 168, Seiten 111-115). Alle Arbeitsschritte müssen bei 33°C durchgeführt werden, um eine Replikation des Vektors zu gewährleisten.

In einem nächsten Schritt wird der zwischenklonierte Vektor ebenfalls mittels der Methode der Protoplastentransformation in den gewünschten Wirtsstamm, hier *B*. *licheniformis,* transformiert. Die solcherart erhaltenen und mit üblichen Methoden (Selektion über den Resistenzmarker des Plasmids; Kontrolle über Plasmidpräparation und PCR für das Insert) als positiv identifizierten Transformanten werden anschließend bei 42°C unter Selektionsdruck durch Zugabe von Erythromycin auf Anwesenheit des Plasmids kultiviert. Bei dieser Temperatur kann der Deletionsvektor nicht mehr replizieren und es überleben nur solche Zellen, bei denen der Vektor in das Chromosom integriert ist, wobei diese Integration mit höchster Wahrscheinlichkeit in homologen oder identischen Bereichen stattfindet. Durch Kultivierung bei 33°C ohne Erythromycin-Selektionsdruck kann dann nachfolgend die Excision des Deletionsvektors induziert werden, wobei das chromosomal codierte Gen vollständig aus dem Chromosom entfernt wird. Der Erfolg der Deletion wird anschließend über Southern-Blot nach Restriktion der chromosomalen DNA mit geeigneten Enzymen oder mit Hilfe der PCR-Technik überprüft.

Solche Transformanten, bei denen das betreffende Gen deletiert ist, zeichnen sich in der Regel zudem durch eine Einschränkung zur Bildung des aus dem zugehörigen Stoffwechselweg resultierenden Geruch- oder Giftstoffs aus. In den Fällen, in denen die Zelle über keinen Ersatzweg zur Synthese der betreffenden Verbindung verfügt, ist der betreffende Stoffwechselweg vollständig blockiert, so daß diese Verbindung überhaupt nicht mehr gebildet wird und der auf diese Weise modifizierte Stamm nicht mehr über die betreffende Geruchskomponente verfügt.

### Beschreibung der Figur

- Figur 1:: Stoffwechselweg zur Bildung von Butanol und/oder Buttersäure Erläuterungen: siehe Text.

## Patentansprüche

1. Nukleinsäure, codierend für ein an der Synthese von Butanol und/oder Buttersäure beteiligtes Genprodukt (wahrscheinliche Phosphat-Butyryl-Transferase; E.C. 2.3.1.19), mit einer Nukleotidsequenz, die zu der in SEQ ID NO. 31 angegebenen Nukleotidsequenz mindestens 51 % Identität und zunehmend bevorzugt mindestens 55%, 60%, 65%, 70%, 75%, 80%, 85%, 90%, 92%, 94%, 96%, 97%, 98%, 99% und besonders bevorzugt 100% Identität aufweist.

2. An der Synthese von Butanol und/oder Buttersäure beteiligtes Genprodukt (wahrscheinliche Phosphat-Butyryl-Transferase; E.C. 2.3.1.19), mit einer Aminosäuresequenz, die zu der in SEQ ID NO. 32 angegebenen Aminosäuresequenz mindestens 69% Identität und zunehmend bevorzugt mindestens 70%, 75%, 80%, 85%, 90%, 92%, 94%, 96%, 97%, 98%, 99% und besonders bevorzugt 100% Identität aufweist.

3. Nukleinsäure, codierend für ein an der Synthese von Butanol und/oder Buttersäure beteiligtes Genprodukt nach Anspruch 1, die natürlicherweise in einem Mikroorganismus enthalten ist, vorzugsweise einem Bakterium, besonders bevorzugt einem grampositiven Bakterium, hierunter bevorzugt einem der Gattung *Bacillus,* hierunter besonders bevorzugt einem der Spezies *B. licheniformis* und hierunter ganz besonders bevorzugt *B*. *licheniformis* DSM13.

4. An der Synthese von Butanol und/oder Buttersäure beteiligtes Genprodukt nach Anspruch 2, welches natürlicherweise von einem Mikroorganismus gebildet wird, vorzugsweise von einem Bakterium, besonders bevorzugt von einem grampositiven Bakterium, hierunter bevorzugt von einem der Gattung *Bacillus,* hierunter besonders bevorzugt von einem der Spezies *B. licheniformis* und hierunter ganz besonders bevorzugt von *B*. *licheniformis* DSM13.

5. Verfahren zur Fermentation eines Mikroorganismus, bei dem mindestens eines der Gene auf einem Stoffwechselweg zur Synthese von Butanol und/oder Buttersäure (als Teil des Buttersäure-Metabolismus) funktionell inaktiviert ist, wobei folgendes Enzym funktionell inaktiviert ist:
Phosphat-Butyryl-Transferase (E.C. 2.3.1.19),
insbesondere wobei das Enzym auf genetischer Ebene funktionell inaktiviert wird,
vorzugsweise durch Inaktivierung eines Gens, das der Nukleinsäure entspricht, die für folgendes Protein von *B. licheniformis* DSM 13 codiert:
Phosphat-Butyryl-Transferase (E.C. 2.3.1.19), definiert über SEQ ID NO. 31, insbesondere wobei zur Inaktivierung auf genetischer Ebene eine der Nukleinsäuren nach Anspruch 1 verwendet worden ist, vorzugsweise ein, besonders bevorzugt zwei Teile dieser Sequenz, die jeweils mindestens 70 zusammenhängende Positionen umfassen.

6. Verfahren nach Anspruch 5, bei dem der Mikroorganismus nur noch 50% der natürlicherweise unter denselben Bedingungen gebildeten Menge, vorzugsweise nur noch 10%, besonders bevorzugt kein Butanol beziehungsweise keine Buttersäure bildet.

7. Verwendung eines Gens, das der Nukleinsäure entspricht, die für folgendes Protein von *B. licheniformis* DSM 13 codiert, zur funktionellen Inaktivierung eines Stoffwechselwegs zur Synthese von Butanol oder Buttersäure und/oder Isobuttersäure (als Teil des Buttersäure-Metabolismus) auf genetischer Ebene in einem Mikroorganismus:
Phosphat-Butyryl-Transferase (E.C. 2.3.1.19), definiert über SEQ ID NO. 31.

8. Verwendung einer Nukleinsäure nach Anspruch 1 zur funktionellen Inaktivierung eines Stoffwechselwegs zur Synthese von Butanol oder Buttersäure und/oder Isobuttersäure (als Teil des Buttersäure-Metabolismus) auf genetischer Ebene in einem Mikroorganismus, vorzugsweise von einem, besonders bevorzugt von zwei Teilen dieser Sequenz, wobei diese Teile jeweils mindestens 70 zusammenhängende Positionen umfassen.

9. Verwendung nach einem der Ansprüche 7 oder 8, wobei die funktionelle Inaktivierung während der Fermentation des Mikroorganismus erfolgt.

10. Verwendung nach Anspruch 9, wobei jeweils eine für ein nichtaktives Protein codierende Nukleinsäure mit einer Punktmutation eingesetzt wird,
oder wobei jeweils eine Nukleinsäure mit einer Deletions- oder Insertionsmutation eingesetzt wird, vorzugsweise umfassend die jeweils mindestens 70 bis 150 Nukleinsäurepositionen umfassenden Randsequenzen des für das Protein codierenden Bereichs.

11. Mikroorganismus, bei dem das Gen funktionell inaktiviert ist, das der Nukleinsäure entspricht, die für folgendes Protein von *B. licheniformis* DSM 13 codiert:
wahrscheinliche Phosphat-Butyryl-Transferase (E.C. 2.3.1.19), definiert über SEQ ID NO. 31, insbesondere wobei es sich um ein Bakterium handelt, insbesondere eines, welches ausgewählt ist aus
(a) gramnegatives Bakterium, insbesondere eines der Gattungen Escherichia coli, Klebsiella, Pseudomonas oder Xanthomonas, oder
(b) grampositives Bakterium, insbesondere eines der Gattungen Bacillus, Staphylococcus oder Corynebacterium.

12. Verfahren zur Fermentation eines Mikroorganismus gemäß Anspruch 11.

13. Verfahren nach einem der Ansprüche 5, 6 oder 12, bei welchem ein Wertstoff hergestellt wird, insbesondere eine niedermolekulare Verbindung oder ein Protein,
insbesondere wobei es sich bei der niedermolekularen Verbindung um einen Naturstoff, einen Nahrungsmittelergänzungsstoff oder um eine pharmazeutisch relevante Verbindung handelt, oder wobei es sich bei dem Protein um ein Enzym handelt, insbesondere eines aus der Gruppe der α-Amylasen, Proteasen, Cellulasen, Lipasen, Oxidoreduktasen, Peroxidasen, Laccasen, Oxidasen und Hemicellulasen.

14. Verwendung eines Genprodukts nach Anspruch 2 in einem Reaktionsansatz oder Verfahren entsprechend seiner biochemischen Eigenschaften.

15. Verwendung nach Anspruch 14 zur Synthese von Butanol und/oder Buttersäure, gegebenenfalls in geeigneter Kombination mit weiteren Enzymen.
